# EUROPEAN PATENT SPECIFICATION

(11) **EP 1 304 380 B1**
(45) Date of publication and mention of the grant of the patent: **05.05.2010**
(21) Application number: 01909410.1
(22) Date of filing: 16.02.2001
(51) Int. Cl.: C12N 15/57, C07K 14/745

(54) **HUMAN SOURCE GENE LEADING SEQUENCE, GENE VECTOR AND GENE EXPRESSION STRATEGY**
HUMANE LEADERSEQUENZ, VEKTOR UND GENEXPRESSIONSSTRATEGIE
SEQUENCE GENIQUE DE TETE D'ORIGINE HUMAINE, VECTEUR GENETIQUE ET PROCEDE D'EXPRESSION DUDIT GENE

(30) Priority: 27.07.2000 WO PCT/CN00/00203
(43) Date of publication of application: 23.04.2003
(73) Proprietor: Xia, Jiahui, Changsha, Hunan Province 410078 (CN)
(72) Inventor: Xia, Jiahui, Changsha, Hunan Province 410078 (CN)
(74) Representative: Grünecker, Kinkeldey, Stockmair & Schwanhäusser Anwaltssozietät
(86) International application number: PCT/CN2001/000126
(87) International publication number: WO 2002/020803

(56) References cited:
- WO-A-97/40183
- US-A- 5 670 314
- US-A- 5 843 757
- DATABASE EMBL [Online] 1 May 2000 (2000-05-01), "Homo sapiens chromosome 21 clone RP11-589M2 map p11-q21.1, complete sequence." XP002326009 retrieved from EBI accession no. EM_PRO:AF254983 Database accession no. AF254983
- DATABASE EMBL [Online] 16 March 2000 (2000-03-16), "Human DNA sequence *** SEQUENCING IN PROGRESS *** from clone RP11-149I22" XP002326010 retrieved from EBI accession no. EM_PRO:AL161418 Database accession no. AL161418
- DATABASE EMBL [Online] 15 June 1998 (1998-06-15), "Homo sapiens PAC clone RP5-823F17 from 7q36, complete sequence." XP002326431 retrieved from EBI accession no. EM_PRO:AC004902 Database accession no. AC004902
- GREIG G M ET AL: "BETA SATELLITE DNA CHARACTERIZATION AND LOCALIZATION OF TWO SUBFAMILIES FROM THE DISTAL AND PROXIMAL SHORT ARMS OF THE HUMAN ACROCENTRIC CHROMOSOMES" GENOMICS, vol. 12, no. 3, 1992, pages 573-580, XP009046782 ISSN: 0888-7543
- HORIE K ET AL: "STRUCTURES OF REPLACEMENT VECTORS FOR EFFECIENT GENE TARGETING" BIOCHEMICAL AND BIOPHYSICAL RESEARCH COMMUNICATIONS, ACADEMIC PRESS, SAN DIEGO, CA, US, vol. 115, no. 3, 1994, pages 477-485, XP000864572 ISSN: 0006-291X
- HARRINGTON J J ET AL: "FORMATION OF DE NOVO CENTROMERES AND CONSTRUCTION OF FIRST-GENERATION HUMAN ARTIFICIAL MICROCHROMOSOMES" NATURE GENETICS, NEW YORK, NY, US, vol. 15, no. 4, April 1997 (1997-04), pages 345-355, XP000674543 ISSN: 1061-4036
- CHUAH MARINEE K L ET AL: "Gene therapy for hemophilia: Hopes and hurdles" CRITICAL REVIEWS IN ONCOLOGY / HEMATOLOGY, ELSEVIER SCIENCE IRELAND LTD., LIMERICK, IE, vol. 28, no. 3, September 1998 (1998-09), pages 153-171, XP002189373 ISSN: 1040-8428
- WAUGH J M ET AL: "Gene therapy to promote thromboresistance: Local overexpression of tissue plasminogen activator to prevent arterial thrombosis in an in vivo rabbit model" PROCEEDINGS OF THE NATIONAL ACADEMY OF SCIENCES OF THE UNITED STATES OF AMERICA, vol. 96, no. 3, 2 February 1999 (1999-02-02), pages 1065-1070, XP002326008 ISSN: 0027-8424
- GONZALEZ I L ET AL: "Human rDNA: Evolutionary Patterns within the Genes and Tandem Arrays Derived from Multiple Chromosomes" GENOMICS, ACADEMIC PRESS, SAN DIEGO, US, vol. 73, no. 3, 1 May 2001 (2001-05-01), pages 255-263, XP004432235 ISSN: 0888-7543
- DATABASE EMBL [Online] 6 March 2003 (2003-03-06), "Homo sapiens chromosome 16 clone RP11-837J3, WORKING DRAFT SEQUENCE, 2 unordered pieces." XP002326437 retrieved from EBI accession no. EM_PRO:AC140904 Database accession no. AC140904

## Description

### SUMMARY OF THE INVENTION

The invention deals with a gene leading sequence, by which a gene vector was constructed, and an expression strategy of a target gene introduced into the human source gene vector. Furthermore, the invention deals with the use of the gene leading sequence for the preparation of a vector for use in gene therapy.

### BACKGROUND OF THE INVENTION

Statistic data of Mendelian inheritance in man demonstrates, up to now, that 1660 single gene disorders have been identified and 989 diseases-related genes have been identified before June 30, 2000. Most of the recessive genetic disorders may be treated by introducing a normal gene into cells of a patient. With the research advancement, initiation of gene therapy regarding dominant genetic disorders and somatic cell-related tumors has begun. In 1995, the American scholar E. Marshall put forward in *Science* that the key point of gene therapy research was the development and discovery of a novel vector, but the problem of gene therapy vector remained unsolved so far. The main reason is that the researchers don't step out of the circle that the vector was constructed by viral components. Generally speaking, commonly-used viral vectors have many defects going as follows: 1) instability: the gene insertion efficiency is low; in part the vector is existing in the cell nucleus in form of an attached body that could not be stably inherited with cell division, so that the therapeutic gene cannot be expressed long and stable. 2) safety is not good: For example, mutation caused by random insertion may affect the normal gene function in integration sites, and even activate an oncogene, which may result in other diseases and tumors, respectively. Furthermore, viral vectors may generate wild-type recombinant virus with the ability of replication, being greatly harmful to the human body. In recent years, it was reported that an adenoviral vector caused the death of a patient during the manipulation of gene therapy. 3) Immunogenecity: the background protein produced by viral genes and protein contaminated during purification could induce an immunogenic-reaction and influence the gene expression.

In the middle of 1980's, a gene targeting vector was developed based on the principle of recombinant homology to achieve site-directed integration, which could avoid immunogenecity and random integration. But a gene targeting vector used for site-directed reparation in gene therapy and the replacement of a defect gene has to utilize specific fragments of the two sides of the gene as leading sequences. Therefore, its application is limited and the transfection efficiency is still low. In fact, it is usually proper for the gene knock-out of embryonic stem and fertilized egg cells, but not suitable for site-directed integration of mature somatic cells (Galli-Taliadoros LA, Sedgwick JW, Wood SA, et al.: J Immunol Meth 1995, 181:1-15; Hasty P, Rivera-Perez J, Chang C, et al.: Mol Cell Biol 1991, 11 (9):4509-4517).

Leon. E. Rosenbery, former president of the American Human Gene Society, Dean of medical school of Yale University, concluded that no case had shown a certain clinical effect among hundreds of gene therapy experiments (Rosenbery L E & Schechter A.N.: Science, 2000; 287:1751). So that to seek and develop a novel stable heredity gene vector with no harm to the human body remains a key question to be solved.

### DETAILED DESCRIPTION OF THE INVENTION

In 1981, the applicant found two families carrying a rarely reported bi-satellite microchromosome (BM), but the phenotype being normal. The microchromosomes steadily inherited in two families with 2 and 3 generations and showed no harm to the human body. Through document investigation, we found that there were seven similar families in both Europe and USA. Up to now, a total of 17 families have been reported, but no one thought of this chromosome to have components for human source gene vector construction. The inventor put forward in 1991 a proposal for the disruption of these chromosome components and the constitution of a human source gene vector. The project was initiated in 1994 and executed in 1995. The applicant firstly detected during investigation that these chromosomes originated from short arms of human D and G group chromosomes including chromosome 13, 14, 15, 21 and 22. The short arms of a D, G group chromosome contain a nucleolus organizing region and are rich in ribosome DNA. Preliminary biological research has revealed that polymorphism of different lengths (namely containing different concentrations of rDNA) were broadly founded in this region in the population, and the genes here could be transcribed very actively during cell division. Therefore, the applicant inferred that if they can isolate a specific fragment from a BM and use it as a leading sequence, a foreign gene can then be transferred site-directly into the nucleolus organizing region, and the expression of the gene should be effective, stable and unharmful. The resultant experiments have strongly proved that assumption.

It was of great signification to find a BM and isolate a specific DNA fragment showing homology to the nucleolus organizing region at the short arms of human D, G group chromosomes.

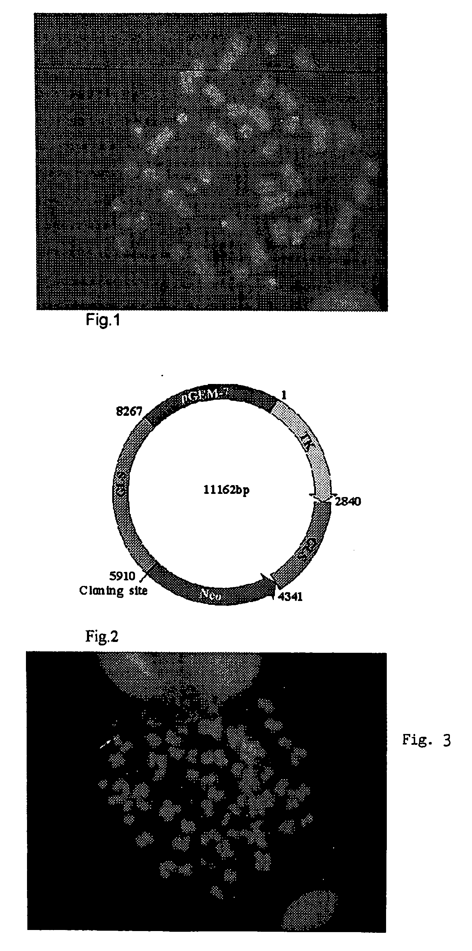

The applicant constructed a BM-specific pUC19 library by micro-dissection, PCR and microcloning techniques at first, and then isolated a single copy fragment by screening this library. The single copy fragment was proved to be from a BM and short arms of D, G group chromosomes by Fluorescent In Situ Hybridization (FISH) . The single copy fragment was further used as probe to screen a PAC genome library and gained a DNA fragment of 120kb (BMSF) (SEQ No.1), which was also confirmed to originate from short arms of human D, G group chromosomes (figure 1). Sequence analysis indicated that no physiological function related genes were found in this BMSF, suggesting the target site being safe.

Described herein is a DNA sequence without a pivot physiological function-related gene from the short arms of human D, G group chromosomes or a sequence sharing 50% or greater than 50% similarity to the short arms in human D, G group chromosomes as gene leading sequence. For example, in example 1, a sequence was selected from the SEQ No. 1 to construct a human source gene vector, this sequence coming from the short arms of human D, G group chromosomes.

The 120kb DNA fragment above can be used as gene leading sequence, and a smaller fragment with specificity selected from the 120kb DNA fragment can also be selected. In the applied case, we selected a 3.8kb fragment from nucleotide 75590 to 79448 in SEQ No. 1 as gene leading sequence (GLS). According to the requirements of gene vector construction, positive and negative screening genes should be added, too.

More concretely, to construct a human gene therapy vector, the 3.8kb fragment from nucleotide 75590 to 79448 shown in SEQ No. 1 was inserted into the pGEM-TK vector which contained a negative screening gene TK and a positive screening gene Neo being inserted into site 1500 of the GLS, which divided the GLS into two arms of 1.5kb and 2.3kb, thereby constructing the gene vector. The bacterial strain containing the gene vector was reserved in the China Typical Culture Collection Center on September 29, 2000 (Wuhan University, Wuhan 430072, China). The reservation number is CCTCCM200030. The administrator designated the reserve classification nomenclature which is Escherichia coli JM109/JH-4/pNS2. The figure 2 shows the vector, and the sequence is given in the SEQ No. 2.

Described herein is a specific target site coming from SEQ No. 1, which denotes a DNA leading sequence originated from the short arms of D, G group chromosome. No doubt, the gene vector constructed by the fragment above can transfer a gene of interest into specific target sites which are of no pivot physiological function-related genes, the targeting being safe. In particular, the present invention provides the following items:
1. A vector having the sequence of SEQ ID No. 2.
2. An in vitro method of gene expression including the following steps:
   (i) cloning a gene of interest into the vector of item 1;
   (ii) transferring the gene of interest into the target region of D, G, group chromosomes of human cells; and
   (iii) expressing the gene of interest.
3. Use of the sequence between nucleotide position 75590 and 79448 of SEQ ID No. 1 as gene leading sequence for the preparation of a vector for use in gene therapy.
4. A vector having a gene of interest cloned into the vector of item 1 for use in gene therapy.

All procedures including gene vector construction, recombination of a gene of interest onto the vector, transfer of a gene of interest into host cells and expression of a gene of interest can be conducted by conventional technologies.

As furthermore described herein, the leading sequence for a gene of interest can be selected from the DNA sequence of SEQ No.1. Moreover, the leading sequence of the gene of interest is at the positions from75590 to 79448 in SEQ No. 1.

The gene leading sequence above can be used to construct a gene vector containing positive and negative screening genes.

The examples 2 and 3 of the present invention explain the procedures above:
① The better specific 3.8kb DNA fragment selected from the 120kb BMSF is subcloned into the pGEM-TK plasmid vector, using TK as a negative screen gene, meanwhile inserting the positive screening gene Neo into the 3.8kb DNA fragment to construct the gene vector. ② The gene of interest is digested by enzyme, and is ligated into an exogenous gene cloning site on one side of the Neo gene. The polymerase chain reaction certified the existence of all parts and the orientation. ③ Selecting a single enzyme digestion site in the pGEM vector and linearizing gene vector, transferring the gene vector into target cells by means of electroporation or liposome transfection. ④ Screening transformed target cells using G418 and GCV to obtain site-directed integrating positive cells clones. ⑤ Detecting the expression of the gene of interest from positive cell clones so as to reach site-directed integration into the short arms of D, G group chromosomes and stable expression.

The positive cells expressing the gene of interest screened are embedded hypodermatically or injected intravenously into the body or the gene of interest encapsulated by liposome is directly injected into the body so that the gene can be expressed long and stably within the body to correct the clinical symptom caused by a defect gene.

The example of the present invention provides the DNA sequence of the gene vector as shown in SEQ No. 2, the leading sequence of which being from 75590 to 79448 of SEQ No. 1. TK is the negative screening gene, the positive screening gene Neo being inserted into site 1500 of the GLS, and the GLS being divided into two arms of 1.5kb and 2.3kb, the cloning site being at nucleotide 5910. The example of the present invention made public the in vitro expression in HT1080 cells of the genes for tissue-type plasminogen activator (TPA) used for treatment of blood occlusion disorders and the coagulant factor IX (FIX) used for treatment haemophilia B. The experiment showed the expression being both efficient and stable.

Therefore, the gene expression strategy provided by the present invention is of great promise in practical application. The gene expression strategy is not only used for manufacturing medicinal protein but also give an effective way for gene therapy.

The inventor made use of a chromosome being not harmful for the human being body as an unique material and created a completely novel strategy. The inventor firstly found that the short arm nucleolus tissue region of human D, G group chromosomes is the best target site for human gene therapy and expression (10 sites in short arms of nucleolus tissue region of chromosome 13, 14, 15, 21, 22) and constructed a novel gene vector capable of transferring a gene of interest site-directly into these target sites.

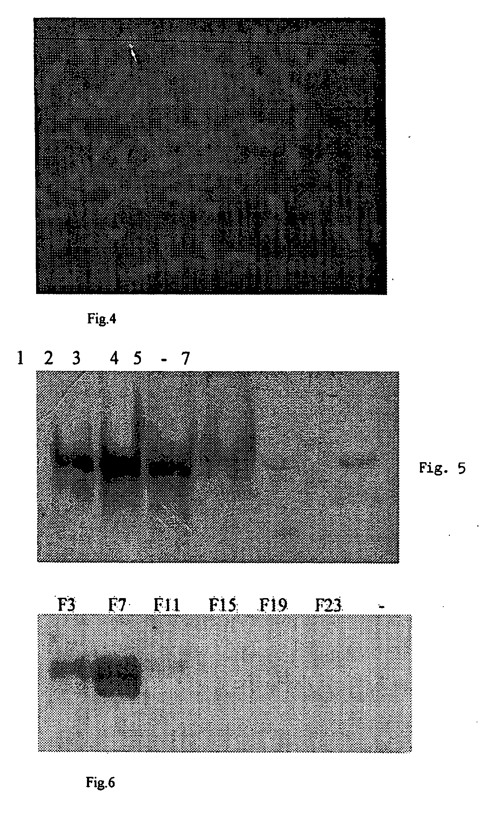

Compared with background technologies, the present invention has some striking advantages:
1. Good stability: using the DNA sequence between nucleotide position 75590 and 79448 of SEQ ID No. 1 as gene leading sequence to construct a vector, the human source gene vector can transfer site-directed a gene of interest into the short arms of D, G group chromosomes of a human somatic cell and allow the gene of interest to be stably inherited with the chromosomes;
2. Good safety: the target site containing no vital physiological function-related gene demonstrates that the target site is safe. Meanwhile FISH confirmed that the vector could insert site-directed a gene of interest into the safe target site in cells (figures 3, 4), excluding the insertion of a mutation at random integration and harm of recombinant wild-type virus. The expression of a gene of interest in target sites is safe. Although the present invention did not provide clinical demonstration of the gene expression strategies, the practical examples in example 4 found by the applicant and foreign researchers confirm its safety from another angle.
3. Efficient expression: First, the gene vector provided by the present invention comes from a leading sequence at the short arms of human D, G group chromosomes, so correspondingly there are 10 target sites in human cells, the insertion efficiency is at least 5-10 times higher than any other vectors; Second, the leading sequence comes from the short arms of D, G group chromosomes where gene transcription is active, so that the gene of interest delivered into the target sites can be expressed highly efficient.
4. No immunogenecity: the vector coming from human being, it has no immunogenecity to human being.

Figure 1 shows the mapping of the 120kb DNA fragment cloned in PAC by FISH
Figure 2 shows a structure map of the gene vector (the whole length of the gene vector is 11162bp); of which pGEM-7 (8267-11162) is: vector replication elements and prokaryotic screening system; TK(1-2840) is: eukaryotic cell negative screening gene which utilizes TK promoter and TK poly A signal; Neo (4342-5910) is: eukaryotic cell positive screening gene which utilizes sv40 promoter and sv40 poly A signal; GLS(2841-4341, 5911-8267) is: leading sequence; Cloning site (5910) is: insert site of the gene of interest;
Figure 3 is a FISH result showing mapping of exogenous tPA gene in positive clone cells, which demonstrate that the vector could target the tPA gene site-directed into the short arms of D, G chromosomes;
Figure 4 is a FISH result showing mapping of exogenous FIX gene in the positive clone cells, which demonstrate that the vector could target the FIX gene into the short arms of D, G group chromosomes;
Figure 5 shows the Western Blot of purified tPA; lanes 1-4 show the purified product of tPA; "-"denotes the negative control;
Figure 6 shows the Western Blot of FIX positive cells, F3-F23 are six different cell strains; "-"denotesthe negative control.

The examples provided in the present invention is one way to realize the present invention, and cannot be considered as being a limitation to the present invention.

### EXAMPLE ONE

The preparation of the gene leading sequence provided in the present invention:
1. Acquirement of the PAC clone containing gene leading sequence
1.1 Construct BM-specific pUC19 library through micro-dissection, PCR and microcloning technologies (Deng H-X, Yoshiura K, Dirks RW, et al.: Hum Genet 1992, 89:13.)
1.2 Acquirement and identification of BM-specific single copy DNA
   (1) The preparation of colony matrix membrane: Draw squares 14×14 on the two pieces of nylon membranes, mark A, B, place the two membranes on the two plates containing solid LB, respectively, pick at random white clones and transfer the clones into squares of two same coordinates, total 14x12 clones, single copy DNA of 100ng is added to the line 13 as positive control, no addition of DNA as negative control. The two plates are respectively placed in incubator at 37°C for 10-12hrs, membrane B is kept at 4°C,take membrane A out of the plate, processing with filter papers immersed in following solutions:10%SDS, 5min, 0.5N NaOH/1.5M NaCl, 3 min, 1.5M NaCl/0.5M Tris.HCl, 3min, 2×SSC/0.2M Tris.HCl, 10min, dry at vacuum at 80°C, 2hrs, store at 37°C for the use.

(2) The preparation of gDNA probes
Sample 50-70ng of gDNA and make up water to 11ml,boil at 100°C, 10min, denature, use the following as primer marker reaction system:

| | |
|---|---|
| 2mM dNTP(dATP) | 3µl |
| primer mixture | 2µl |
| klenow enzyme | 1µl |
| α-³²P-dATP | 3µl |

mix up on vortex, incubate for 30min at 37°C, add 8µl stop-mixture, filter through G-50 column to purify the probe, take one-tenth for liquid scintillation.
(3) Hybridization: colony dot matrix membrane is placed in 2 × SSC and immersed for 10min, remove carefully the debris on the surface of membrane, pre-hybridize at 65°C in 5ml hybridization liquid for 30min at least. According to the value of liquid scintillation, based on 1.2 × 10⁶cpm/ml hybridization liquid, sample probe liquid, boil at 100°C , 10min, denature, add 5ml fresh hybridization liquid to colony dot matrix membrane and allow for over 12hrs at 65°C ,and then wash the membrane under following conditions: 2×SSC/0.1%SDS, 10min at room temperature, 2 × SSC/0.1%SDS, 10min at 65°C , 0.1 × SSC/0.1% SDS, 10min at 65°C, autoradiography at -70°C, strong or weak hybridization signal is considered to be single copy elementarily.
(4) Sequencing, southern blotting detection: pick clones without hybridization signal on the corresponding position of membrane, expand, extract plasmid for DNA sequencing, compared with database of GenBank, the copy without similarity to other sequence is considered to be single copy. Finally, isolate inserted DNA by enzyme digestion. Label the insert by α-³²P-dATP by random primer method and then hybridize with the EcoR1 digested gDNA on the nylon membrane, the clone showing one or two bands is considered to be a single copy.

### 1.3 Acquirement and identification of BM and short arms of group D, G chromosomes specific PAC clone

### (1)Screen human PAC gDNA library to obtain positive clone

Using of α-³²P-dATP to label the single copy probe p8-7 of 260bp by random primer method → purify the probe by G-50 column(middle size of particles) → keep for use at 4°C → seven pieces of PAC membranes immersed in 2 × SSC for → 10min pre-hybridize for 3hrs at 55°C → denature probe for 10min at 100°C → add 50ml hybridization solution purchased commercially according to dosage of 4.6 × 10⁵cpm/ml,hybridize to PAC membrane for 1hr at 65°C → wash the membrane: 2×SSC,10min a time at ambient, wash with 2×SSC/0.1% SDS, 10min at 65°C, twice placed onto the x-ray film, autoradiography for 12 hours → develop X-ray film count positive clone number as instruction goes.

### (2) Pick at random positive clones number from five different plates, purchase PAC clones.

### 1.4 FISH of PAC DNA to metaphase cells confirms that the DNA was from group D, G chromosome, as shown in figure 1.

Experimental methods above referred to Molecular Cloning. Second Edition (Cold Spring Harbor Laboratory Press, 1989)
1. Isolation of gene leading sequence DNA
   Main materials: β-agarase (Bio-Labs) Not I Agarase
   ① Digest PAC 169 Plasmid by Not I enzyme;
   ② Isolate Inserted DNA of 120kb by PFGE;
      Pulse electrophoresis conditions: electrode buffer solution:0.5 × TBE, high strength Analytical Grade Agarase (Bio-Rad, Low Melting point Agarose LMP) 1%, Switch time:2 seconds → 15seconds → electrophoresis time: 18hrs, voltage: 6V/cm, angle:120°^{,} temperature: 14°C
   ③ After electrophoresis, stained with EB (0.2µg/ml), 30min, excise band of 120kb.
   ④ The gel cut is treated with β-agarase, precipitated in alcohol.

### EXAMPLE TWO

The preparation of gene vector provided by the present invention
1. Construction of gene vector and transfer of gene of interest
1.1 Construction of vector
   1.1 PAC DNA is digested by Nsi I and Stu I(blunt enzyme), 3.8kb DNA fragment is recovered by general agarose gel electrophoresis, purification with electricity elution;
   1.1.2 Digest pGEM-TK vector DNA by Hind III, make it blunt by Klenow enzyme, to generate a blunt end;
   1.1.3 The linearized pGEM-TK /Hind III is further digested by Nsi I;
   1.1.4 The digested PAC DNA of 3.8kb and pGEM-TK were ligated at 16°C for 17hrs;
   1.1.5 Ligated product was transformed into JM109 competent bacteria, incubate in plate containing ampicillin for 18hrs at 37°C;
   1.1.6 Pick monoclone at random, determine positive clones by Nsi I and Nhe I digestion after plasmid extraction, the recombinant plasmid was named pGEM-TK-3.8kb .
   1.1.7 Obtain Neo gene by digesting pCDN-GPR plasmid with Xba I and Nhe I
   1.1.8 Ligate Xba I and Nhe I digested Neo gene with Nhe I digested pGEM-TK-3.8kb to construct pNS2 gene vector;
2.1 Transfer of TPA and FIX genes
   2.1.1 Clone TPA and FIX (CDS) into pcDNA3.1(-), respectively;
   2.1.2 Design the primers TPCF and TPCR to amplify TPA and FIX gene and expression elements (CMV promoter and BGH poly A signal), introduce enzyme Avr II restrictive sites into the two ends of primers, the sequences of primers go as following : 
TpcF: ATgCATCCTAggggAggTCgCTgAgTAgTg
AvrII
 
TpcR: TgCATgCCTAggTACCCCCTAgAgCCCAg
AvrII
   2.1.3 Digest amplified TPA/FIX gene and expression components (CMV promoter and BGH poly A signal) by Avr II, and ligate it into pNS2 vector digested by NheI enzyme.

The procedures above, please see to "J. Sambrook et al. Molecular Cloning. Second Edition. Cold Spring Harbor Laboratory Press.1989"
3. Extraction of gene vector DNA
3.1 Materials
3.1.1 QIAGEN Plasmid Maxi Kit
3.1.2 Culture Media: liquid LB

| | |
|---|---|
| Trypton | 5g |
| Yeast extract | 2.5g |
| NaCl | 2.5g |
| Add ddH₂O to | 500ml |

Autoclaved
3.1.3 Ampicillin:100mg/ml(100 ×)
3.2 Procedures
1) Pick and inoculate positive clones into 3ml LB(Amp⁺),incubate 1hr at 37°C
2) Put 100µl of primary culture above in 100ml LB (Amp⁺), incubate 16hrs at 37°C, 250rpm
3) Harvest bacteria by centrifugation at 6000g for 15min at 4°C
4) Add 100ml buffer P to resuspend the bacterial pellet
5) Add 10ml buffer P2, gently invert flask six times to mix up completely, stand for 5min at ambient
7) Add 10ml pre-cooled buffer P3, gently invert flask six times, place on ice for 20min
8) Centrifuge at 20000g for 30min at 4°C
1) Transfer swiftly supernatant to 40ml centrifugation tube, centrifuge at 20.000g for 15min at 4°C
9) 10ml buffer QBT equilibrates QIGEN tip 500
10) Transfer the supernatant to QIGEN tip 500,filter through the column
11) Wash the column with 2 × 30ml buffer QC
12) Elute the column with 15ml buffer QF, collect the elution liquid
13) Add isopropanol (0.7-times volume) 10.7ml to elution solution, thoroughly mix up
14) Centrifuge at 15000g for 30min at 4°C
15) Remove the supernatant, add 5ml, 70% alcohol to DNA precipitate, centrifuge at 15000g, 4°C for 10min
16) Remove 70% alcohol, dry the DNA in the air for 10min,add a certain amount of TE resolve DNA precipitate

### EXAMPLE THREE

Introduce gene vector carrying the TPA or FIX gene into host cells and express them in vitro
1. Materials
   1.1 cell: HT1080
      culture medium: high-sugar DMEM+10%FBS(HT1080)EMEM+10%FBS
   1.2 Electroporation apparatus: Bio-Rad company
2. Methods:
   1) Cells are inoculated in 75 cm² canted-neck flask, culture and grown up to 70%-80% confluence
   2) Harvest the cells and wash twice with HeBs buffer solution, count the cell number
   3) Centrifugation at 1500rpm, 4°C for 10min
   4) Resuspend with proper volume of HeBS, dilute the cell density to10⁶-10⁷/ml
   5) Take 0.4ml electroporation cuvette, add 0.8ml cell suspension, 10µl vector DNA
   6) Electroporate the cells at 260v, 550uFf, lasting 11-13 ms
   7) The electroporated cells above are transferred into 75cm² canted-neck flask, add 14ml culture medium containing ampicillin/streptomycin, incubated in 5%CO₂, at 37°C, 24-48hrs.
   8) Add G418 to culture medium to a final concentration of 300µg/ml, screen, replace culture medium every 2-3 days and renew G418, HT1080 without gene transfer was used as control
   9) The control cells died after 7-10days,count the survival clone cell number within transformed cells, and use maintenance volume of G418 of 150µg/ml
   10) Continue to screen transformed cells with GCV of 500ng/ml
   11) Most of cell clones died after 7-10days, add maintenance volume of GCV 250ug/ml, when the left survival cells grow up to 70%-80% confluence, detect the expression activity of transferred genes
3. Results
   TPA gene and FIX gene are introduced into HT1080cells, respectively, by electroporation using human source gene vector, positive clones were obtained after positive and negative screening. Site-directed integration of two genes were confirmed by FISH (Figures 3, 4). The results of the activity determination are detailed in the following tables 1 and 2.
   In negative control of HT1080 cells, TPA activity is 0 µg/10⁶ cells/24hrs, after the transfer TPA activity is 408 µg/10⁶ cells/24hrs, expression efficiency is 407 at day 95 after the transfer, the expression is very stable (Table 1). FIX activity is increased from less than 0.5ug/ml up to 2.5µg/ml, the expression content remain 2.6µg/ml, see Table 2. The expressing products of two genes have been testified by Western Blotting (Figures 5, 6), the expressed protein of TPA has been purified.

**Table 1 Activity detection of positive cells transformed by TPA (µg/10⁶ cells/24hrs)**

| Days after transformed | T1 | days after transformed | T15 |
|---|---|---|---|
| 33 | 408 | 54 | 88 |
| 37 | 396 | 58 | 204 |
| 60 | 411 | 95 | 114 |
| 68 | 430 | | |
| 74 | 430 | | |
| 88 | 441 | | |
| 90 | 440.9 | | |
| 95 | 407 | | |

| | | | |
|---|---|---|---|
| (T1, T 15 are positive cell strains of tPA) | | | |

**Table 2 Activity detection of FIX gene (µg/10⁶cells/24hrs)**

| Days after transformed | clone F23 |
|---|---|
| 60 | 2.5 |
| 72 | 2.4 |
| 100 | 2.9 |
| 109 | 2.6 |

### EXAMPLE FOUR

Safety case among human being
Prof. Xia Jiahui has been engaged in research on human and medical genetics since 1973. He found and identified 732 abnormal karyotypes first reported in the world, which claimed by 470 clinical cytogenetists who working with 189 labs around China. Among these 732 karyotypes, 41 of which involve in the short arms of group D, G chromosomes. No matter which chromosome is from chromosome 1-22, the fragment translocated into short arms of group D, G chromosomes or the lengths of fragment from the same chromosome are different, the number of gene contained is from one to thousands, but the phenotype of carrier is normal, which explains that the genes translocated into short arms of D, G group chromosomes can express normally. So it is should be safe to use short arms of D, G group chromosomes as targeting sites for gene therapy.
1. Karyotype: 46, XX, t(1;12;22;15;11; 8) (1qter→1p11::8p23→8pter; 12pter→12q11::1p11→1pter; 22qter→22p11::12q11→12qter; 15pter→22p11→22pter; 11pter→11q21::15q15→15qter; 8qter→8p23::11q21→11qter)
   Phenotype: female, 28 years old, normal phenotype carrier;
   Material provider: Wu subing, Cytogenetics laboratory, Department of gynecology and obstetrics, first affiliated hospital of Zhongshan Medical University, Guangzhou.
2. Karyotype: 46, XY, t(1;13) (1pter→1q32::13p11→13pter; 13qter→13p11:: 1q32→1qter). Phenotype: female, 24 years old, normal phenotype carrier;
   Material provider: Xiao Chen, Department of biology, Harbin Medical University, Harbin 150086.
3. Karyotype: 46, XX, t(2;15) (2pter→cen→15qter ;2qter→cen→15pter) Phenotype: female, 26 years old, normal phenotype carrier;
   Material provider:Guo Yuping, et al., Cytogenetics Department, Jiangxi provincial gynecology and obstetrics hospital, Nanchang 330006, Jiangxi province.
4.Karyotype: 46, XY,t(2;21) (2pter→cen→21pter; 2qter→cen→21qter ) Phenotype: male, 32 years old, normal phenotype carrier;
   Material provider: Kang Guoqing, et al. Department of genetics, the second affiliated hospital of Shangxi Medical College, Taiyuan 030001.
5. Karyotype: 46, XY,t(3;21) (2qter→cen→22pter; 3qter→cen→22qter ) Phenotype: male, 26 years old, normal phenotype carrier;
   Material provider: Gao Yun., Department of toxicology, Bingzhou municipal Medical College, Bingzhou 256603, Shangdong province.
6. Karyotype: 46, XY,t(3;22) (3pter→cen→22pter; 3qter→cen→22qter ) Phenotype: male, 29 years old, normal phenotype carrier;
   Material provider: Shi Huajin., Department of genetics, Jingzhou Women and Baby hospital, Jingzhou 121000, Liaoning province.
7. Karyotype: 46, XX, t(4;15)(4qter→4p13::15p13→15pter;15qter→15p13::4p13→4pqter ) Phenotype:female,28 years old, normal phenotype carrier;
   Material provider: Zhou Ling,et al., Laboratory of genetics, the Wuhan Children hospital, Wuhan 430016, Hupei province.
8. Karyotype: 46, XY,t(4;21)(4qter→4p15::21p11→21pter;21qter→21p11::4p15→4pqter ) Phenotype: female, 25 years old, normal phenotype carrier;
   Material provider: Xu Jinfang, et al. Laboratory of genetics, the sixth people's hospital of Shanghai, Shanghai200000.
9. Karyotype: 46, XY, t(4;14)(4qter→4q31::14p11→14pter;14qter→14p11::4q31→4qter ) Phenotype: male, normal phenotype carrier
   Material provider: Zhou Mingjun, et al., Xuchang Municipal Central Hospital, Xuchang 161000,Henan province.
10. Karyotype: 46, XY, t(4;14)(4qter→4q35::14p11→14pter;14qter→14p11::4q35→ 4qter ) Phenotype: male, 27 years old, normal phenotype carrier;
   Material provider: Zhang Xiuquan, et al., Hushan Municipal Women and Nursling Hospital, Hushan 528000, Guangdong province.
11. Karyotype: 46, XX, t(6; 22)(5qter→5q13::22p11→22pter; 22qter→ 22p11:: 5q13→5qter ) Phenotype: female, 32 years old, normal phenotype carrier;
   Material provider: Zhao Jianping, Anyang Municipal Women and Nursling Hospital, Anyang455000, Henan province.
12. Karyotype: 46, XY, t(6;22) (6pter→cen6→22qter; 6qter→cen22→22pter ) Phenotype: male, 25 years old,normal phenotype carrier;
   Material provider: Zhu Xinxia,et al., Laboratory of cytogenetics, Department of Gynecology and Obstetrics, Number 88 Hospital, Taian 271000, Shangdong province.
13. Karyotype: 46, XY, t(6; 22)(6qter→6p21::22p11.2→22pter;
   22qter→22p11.2::6q21→6pter)
   Phenotype: male, 33 years old, normal phenotype carrier;
   Material provider: Yang Qinglan, Department of Gynecology and Obstetrics, affiliated hospital of Bingzhou Medical College, Bingzhou 256603, Shangdong province.
14. Karyotype: 45, XX, t(7;21) (7qter→7p22::21p12→21qter) Phenotype: female, 23 years old, normal phenotype carrier;
   Material provider: Sun Qingji, et al., Laboratory of genetics, the Wuhan Children hospital, Wuhan 430016, Hubei province.
15. Karyotype: 46, XY/ 46XX, t(7;14)(7pter→7q11::14p11→14pter;
   14qter→14p11::7q11→7qter)
   Phenotype: male, 28 years old, normal phenotype carrier;
   Material provider: Li Luyun, Xia Jiahui, et al., State Key Laboratory of Medical genetics (Hunan Medical University), Changsha 410078, Hunan province.
16. Karyotype: 46, XY, t(8;14)(8pter→8p21::14p12→14pter;14pter→ 14p12::8p13→8pter ) Phenotype: male, 27 years old, normal phenotype carrier;
   Material provider: Shi Huajin et al., Department of genetics, Jingzhou Women and Baby hospital, Jingzhou 121000, Liaoning province.
17. Karyotype: 46, XY, t(9;14)(9pter→cen→14pter; 9qter→cen→14qter) Phenotype: male, 28 years old, normal phenotype carrier;
   Material provider: Cheng Qiuyun et al., Department of reproduction medicine, first affiliated hospital of Hengyang medical college, Hengyang 421001, Hunan province.
18. Karyotype: 46, XY, t(9;22) (9pter→9p13::22p12→22pter;
   22qter→22p12::9p13→9pter )mat
   phenotype: female, 31 years old, her mother, a young sister of her, a young brother of her and her son have the same phenotype as her, that is normal phenotype carrier;
   Material provider: Li Luyun, Xia Jiahui, et al., State Key Laboratory of Medical genetics (Hunan Medical University), Changsha 410078, Hunan province.
19. Karyotype:46, XX, t(9;14) (9pter→9q12::14p12→14pter; 14qter→14p12:: 9q12→9qter). Phenotype: female, 32 years old, normal phenotype carrier;
   Material provider: Sun Yanyang et al, Department of biology, Harbin Medical University, Harbin 150086.
20. Karyotype:46, XX, t(9;15) (9pter→9q21::15p12→15pter;
   15qter→15p12::9q21→9qter)mat.
   Phenotype: female, 36 years old, normal phenotype carrier;
   Material provider: Zhu Guizhen et al., Laboratory of cytogenetics, Department of Gynecology and Obstetrics, Number 88 Hospital, Taian 271000, Shangdong province
21. Karyotype:46,XX, t(10;13) (10pter→10q24::13p11→13pter;
   13qter→13p11::10q24→10qter) Phenotype: female, 28 years old, normal phenotype carrier;
   Material provider: Yan Dunqing., Department of Gynecology and Obstetrics, affiliated hospital of Qingdao Medical College, Qingdao266003, Shangdong province.
22. Karyotype: 46, XX, t(10;13) (10pter→10q24::13p12→13pter;
   13qter→13p12::10q24→10qter)
   Phenotype: female, 29 years old, normal phenotype carrier;
   Material provider: Zhang Yinru et al., Department of neurology, First affiliated hospital of Zhongshan Medical University, Guangzhou510080, Guangdong province
23. Karyotype: 46, XX, t(11;14) (11pter→cen→14pter::11qter→cen→14qter)
   Material provider: Wang Zhiyong, Department of genetics, Zhacheng County people's hospital, Zhacheng County 476200, Henan province.
24. Karyotype: 46, XX, t(11;21) (11pter→11p11::21p11→21pter; 21qter→21p11::11p11→11pter)
   Phenotype: female, 26years old, normal phenotype carrier;
   Material provider: Zheng Jun et al., Department of genetics,Shanxi provincial women and nursling hospital, Xian710003 Shanxi province.
25. Karyotype: 46, XX, t(11;15) (11pter→11q13::15p12→15pter; 15qter→15p12::11q13→11qter)
   Phenotype: male, 23years old, normal phenotype carrier;
   Material provider: Yang Ruifang et al., Medical center of Obstetrics, affiliated hospital of Shandong Medical University, Jinan250012, Shandong province.
26. Karyotype:46, XX, t(12;14) (12pter→cen→14pter::12qter→cen→14qter)
   Phenotype: female, 28years old, normal phenotype carrier;
   Material provider: Han Weitian et al., Department of eugenics, Liaoning provincial institue of family planing, Shenyang 110031, Liaoning province.
27. Karyotype:46, XX, t(13;16) (13qter→13p11::16p11.2→16pter;
   16qter→16p11.2::13p11→13pter
   Phenotype: female, 27years old, normal phenotype carrier;
   Material provider: An Songlan., Department of genetics, Dalian municipal gynecology and obstetrics, Dalian 110078, Liaoning province.
28. Karyotype: 46, XY / 46, XX, t(13;13) (13qter→13p12::13p12→13qter)
   phenotype: male, 39 years old, normal phenotype carrier
   material provider: Li Luyun, Xia Jiahui, et al. State Key Laboratory of Medical genetics (Hunan Medical University), Changsha 410078, Hunan province.
29. Karyotype:46, XY, t(14;18) (14pter→cen→18pter;14qter→cen→18qter)
   Phenotype: male, 30years old, normal phenotype carrier;
   Material provider: Wang Sugui et al., Beijing Institute of family planing technology guidance, Beijing 100006.
30. Karyotype:46, XX, t(14;15) (14pter→14q13::15p13→15pter; 15qter→15p13::14q13→14qter)
   Phenotype: female, 28years old, normal phenotype carrier;
   Material provider: Li Luyun, Xia Jiahui, et al., State Key Laboratory of Medical genetics(Hunan Medical University), Changsha 410078, Hunan province.
31. Karyotype:46, XX, t(15qter→cen→22qter)
   Phenotype: female, 27years old, normal phenotype carrier;
   Material provider: Li Luyun, Xia Jiahui, et al., State Key Laboratory of Medical genetics (Hunan Medical University), Changsha 410078, Hunan province.
32. Karyotype:46, XY, t(15;18) (15pter→cen→18pter;15qter→cen→18qter)
   Phenotype: male, 30years old, normal phenotype carrier;
   Material provider: Ren Guoqing et al., Beijing Institute of family planing technology guidance, Beijing 100006.
33. Karyotype:46, XX, t(15;20) (15pter→cen→2pter; 15qter→cen→2qter)
   Phenotype: female, 26 years old, normal phenotype carrier;
   Material provider: Wang Xin et al., Laboratory of genetics, department of obstetrics, the second affiliated hospital, Hunan Medical University, Changsha 410011, Hunan province.
34. Karyotype:46, XX, t(15;22) (15pter→15q11::22p13→22pter; 22qter→22p13::15q11→15qter)
   Phenotype: female, 27 years old, normal phenotype carrier;
   Material provider: Hu Shengdi, Department of genetics, Hainan provincial people's hospital, Haikou570011, Hainan province.
35. Karyotype:46, XX, t(15;22) (15pter→15q22::22p11→22pter;
   22qter→22p11::15q22→qter)
   Phenotype: female, 29 years old, normal phenotype carrier;
   Material provider: Li Murou, Department of genetics, Xinjiang Medical College, Urumchi 830054.
36. Karyotype:46, XY, t(16;21) (16pter→16q11::21p11→21pter; 21qter→22p11::16q12→16qter)
   Phenotype: male, 29 years old, normal phenotype carrier;
   Material provider: Zhang Huifang,et al, Institute of family planing technology of Guangdong,Guangzhou510080,Guangdong province.
37. Karyotype:46, XX, t(18;21) (18pter→cen→21pter; 18qter→cen→21qter) Phenotype:female, normal phenotype carrier;
   Material provider: Shi Huajin et al., Laboratory of genetics, Jingzhou women and nursling hospital, Jingzhou 121000, Liaoning province.
38. Karyotype:46, XX, t(18;21)(18pter→18q11::21p12→21pter; 21qter→22p12::18q11→18qter)
   Phenotype: female, 26 years old, normal phenotype carrier;
   Material provider: Li Xiulin et al, Laboratory of genetics, department of pediatrics, first affiliated hospital of Chinese medical university, Shenyang110011, Liaoning province.
39. Karyotype:45, X, dic(Y;13)(Ypter→Yp1200:: 13p11→cen→13qter)
   Phenotype: male, 4 years old, normal phenotype carrier;
   Material provider: Xia Jiahui, et al. State Key Laboratory of Medical genetics(Hunan Medical University),Changsha 410078,Hunan province.
40. Karyotype:46, XY, t(Y;15)(15qter→15p12:: Yq12→Ypter) pat.
   Phenotype: male, 4 years old, normal phenotype carrier;
   Material provider: Xia Jiahui et al. State Key Laboratory of Medical genetics (Hunan Medical University), Changsha 410078, Hunan province.

Abnormal chromosomes carriers above showed no any abnormal syndrome, which explains that not only nucleolus tissue can receipt foreign genes but also allow foreign genes to express normally.

### Sequence listing

<110> Xia Jiahui
   <120> Gene leading sequence, gene vector, gene targeting site and gene expression strategy
<160> 2
<210> 1
   <211> ∼120kb
   <212> DNA
   <213> Homo sapiens
<400> PAC clone sequence 
1 TATTTGTATG TATCCTGGAA TACACAGAAT AAGATCTTGC ATGTAAGAAA
51 CATTTTAATA GTTTTTAATG TGAATCAACA AACAGGTAAA TGGGCTTTTT
101 TATAATAGAA TGTTACAGGT AACATAATAT GCATGACATA TCTAATAATT
151 AAATCTATTA AATTTCTTGA GTGTTGAATT TATCTTTTCT TCTAATGTTG
201 AGAATGTATA AGTTGAGGTG ACATGTTGAG AAAATATGTC ATATAGAAGA
251 AAATAAAATT TAGAAAAGAT GAAAAGGAAA TAGGAAAGGT GGATACAGCC
301 TGTTTTCTAA TATCTTCCAA CTGGAAGCTT AGATTAGGAT TTTAGATTAA
351 ATTTTCTTAA ATTTTTAAAG ACCCAATCAT TTTCTATTAA ATACCTGTTT
401 TTCAGGACAT ACATTACTCT TTCAAATTCT GAATAAAAAT TCTTTCTCCA
451 GCTGTTAAGT TGCTGGAATT TTTTTTCCCT CTCTCTGTAG TAAGAATGTT
501 TCAACTTTCA GATCAATCAT CATGGTTAAC TCTTGGGATG TGTTATTTTA
551 TAGTAAACAA ATGAAACTCT TTCTTACATA AGTATTTACA CAAAACAAAT
601 TGTCGAGTAA ATGCTAACCA GTATTATTAA GCATATATTA TGAACACAAG
651 GTTTTCTTTT AAGCTCATAT TTGATTGACT GGTCATGTCT CTTTTTTTGT
701 TTGTTTCTCC CTCCTTCCCC TTTGTTTAAA AATGATTTAC CTCAGTCTAA
751 CTTTCTCCTT GCAGCCCACA TGTCCTTAGT GTCTATTCTT TCATTTCACT
801 TCTGTTTCTA TTGTCAGCAT ACTTAGTTAC AGCTTTCTAT TTAGTAGCTA
851 TGTGTGGCCT TCATTCATGA ATCATTGCTC CGTAAGGTGG ATGGTTGCTT
901 TGTTCTGTCT TTTTTGGAAG GAGCTTAAGT TATACTATAA TTTGTTTTTA
951 GCTTTTGACA CACTGAATAG AAGCAACTTG TACTGTTTCT GATGCCAACC
1001 CATTTAAATA TAGTACTACT AAAAACTACA TTCTCACATT TTTTCCCACA
1051 AAATGCAATT TGTACAAATA TACATCATGC ATAGCTATTT TGAAAAATAA
1101 TGTGATTAAT TTACTTTTTT ATTATTAGTT TTATTCGGTA ATACTCTAAA
1151 CTACATAGGG TACATTTTTG TTCTAGTTTG TTGTGGTTAA TAAAAGAAAC
1201 TAATGGATAT CATAGTATTT GTATAATAAT CAACTGGGGT TGAAAGGAGT
1251 AACATTTTGA AAATATCTGT CTCACCAATC ATAACCATCA TGCCATGTGA
1301 ATCTGTAGAT AAGTAAATGG CAGACTTGAG GTTTGAATCT ACATATGAGT
1351 GACTTCAACG TCCATACTTT TCCTGTTAGA TCATGTTGTA ATGGTGGGTA
1401 TTATCATTAT TTTAACTTGC CCCATGTTAT TCTTAAACCT ATTGTGTTTT
1451 TCTTCTAGAC TATTTCAAAA CTATACATCC CAGAGAGAAG GATTATTTCT
 
1501 CTATGATCGA TAGAAGATGG TAAGCTATTT GAAGCCTGCC TATTGCAGTA
1551 TTTACTGATT CTTCATTTGT TTTTGTTTTT TTTTTTCTTT TTTAAACACA
1601 GTCTTACTAG TCTGCAGCCC AGGCTGGAGT GCAGTGGTGC GATCTTGGGT
1651 CACTGCAGCT TCCACCTCTT GGGTTCAAGT GATTCTCCTG CCTCAGCCTC
1701 CCAACTAGCT GAGATTACAG GTGCGCACCA CCACACCTGG CTAATTTTTG
1751 TATTTTTAGT AGAGATGGAG TTTCACAATG TTGTCCAGGC TGGTCTCAAA
1801 CTTTTGACCT CAAGTGATTC CCCCGCCTAG GCCTCCCAAA CTGCTAGGAT
1851 TACAGGCGTG ACCCACCACA CCAGGCCTTG TGGATTATTA TTATTATTTT
1901 TATAAGGATA GAGTCTTGCT ATTTTGCCCA GGCTGGTCTC AAACTCCTGG
1951 GCTTCTCAAG TTATACTTCT GCCTCAGCCT TCTGAGTAGC TGGAATTATA
2001 GGAACAAGAC ACTGTGTTCT TTTATGTTTT TAGTACTCTG TAGTTTTCTA
2051 TTGTTGATTT AAAATGCATT TTACTTTTTC TTTCATAGTG CTTCCTCCTG
2101 TTGAAGAGTG TTTTGACAGG TATGATTTCA CAGATTTTTT AAAGTGATAT
2151 GTTAACTAAG TGAATAGAGA GAATAGAAAC TAGTATCTGT TTAGTGTTCT
2201 ACTCTGTGCT AGACACCATA TTACATGCTT AACATTTATC ATGTCATGTC
2251 ATCTTCACAC GCTTTACAAA CTATTTGTGC TATTATTGCT TTTTTACTAA
2301 TAATAGGCAA CTCTGCTTTA AAGAGGTTGA AATATTGGCT CATGATTCCA
2351 CAGTTAACAG GTAGCCAACC CAAGATTAGA CCATCATCCT GCCTGGCTCT
2401 CAAATCACTT CATTTGCCCC TAAGCATAGA TGGATAGAGG CCTATGCAGC
2451 AATGGGATCA CGGTACTGAT TTACATCGGA TCAATTCAGA AAGTCACATT
2501 TTGTTATATA TTAACTGTCT TTAGAGTGTT ATTTAGAAGC CTGGCTACTC
2551 CAAAAGTTTG TCCAAATATT TTGAAATGGC AGTGGTAACC ATATTACTTT
2601 TTTTTTTAAT CATCAAAGTT TTAAAGGCAG TTATCAGTTA TCTGTGGCCA
2651 CAGGACCCTA AGTTTTTCAT AAGCAAGACC AGGCCAGTCC TAGAAAAATA
2701 TTATCTTACT GTGCTTGGAG AATATCTAAA TATAGTCCAT GTTATGTTAA
2751 CTATATTTAG CATATATTAA AAGGATATTT CTAATTCATT TCTCTACTTA
2801 CTACCTACCC AGTTAGGTTT TCTCTTTCAG TAAGTACCCT GCCTGGTTCT
2851 CTGAAGCTTT TTCTTATTTT CTGGATTCTT TTTTTTCCTT CTGTGACATT
2901 TTAATAATAT TTTCTTGATT TCTTTTTACT TTCTCCTCTG CTTTTCTTAG
2951 GGTTTATGAT CTTCTATTAT AGTTTTCATT TAAGGCAGCT AAATATTCCC
3001 CATTTTCCTA TAGACAAAAT CAGAGGTGCA TAGAATTTCA GAATATTAAG
3051 AAATCCTAGA GACTAAACAA AATATCTTCT AGTACTTGAA TCCTTGCTTA
3101 ACATCCTGAT CAAGTGGTTG TTCAGGTGGA GAACCTGAAA CTCAAAGAGA
3151 GAAAATTATT TGGATACAGT AAATCAGAGA AATGAGAATT GCACTCAGGT
3201 TTCTTAGTTC AAAACCCAGT CTTCTTTTAC ATCATTCTGT CATTGAGTGA
3251 TTTTAGTTTT AGAAAGAGGG AGTGGCTCTG GTGAACACAG TGGAAAAGGA
3301 TGAGAATGGA ATGAGCTGTT GAACCCAATG AAAGTGGATA AGAATGGAAT
3351 TTGCAGGGGA CAGACAAATT TGAAGATGTA GATTGGAGAT TGTCATTGAT
3401 CCCTGCAGAA GAGGGATCCA GAAGGGGGAG CCCATAGGAA GGTATTTAGA
3451 TAGTGATGAA TGAAATGAAG CTGTAAGTAC TCAGGAGGGA GACTTTTCCA
3501 GCAGTCTCTC TCTTGAGTAT CTGAGTGTTT ATGAAGGTTC TTAAGCTTGC
3551 TGGTTTTTGT GGACCTGAAT AAGGCAGGAT CTATATAGTG ACAATAATTG
3601 GATTTTATAA TTTTTAATGT TTTAATCTTC TGTGAGGAAT ATTCCCAGAG
3651 TACATTTGTA CTTTGACTTT TTTACACTCA GCTTTCAAAC ACTTGCAGTG
 
3701 TTTCAGGGGG CTCCCTGTAG AGTTCTAGAG TAAAGAGAAT CAATGGGCAA
3751 TCTAAGTAGC TTACATGCTG AAGATCCAAG ACTCCCATTT TCCAGTGACA
3801 CAGATTAGTC TTTGAATCAG AAATAGATAA TGCAGAAGAC ACAGTGCCTT
3851 TTCTACCTTG TTTTAGGTTA TCGGGTTTAC TGCAGTTGAG TAACAAAAGT
3901 TGTTTCAGAT ATCAATTGGA TTTTCAGTTT AGTCTTTAGG GTAGATAATT
3951 TATAAAGACA TATTATTGTC TGGCCGTGCC ATTGTAATGC CTGTCACTAT
4001 CTGTTACGGG TTTAAGGGTG AGTCTGCATT GGATATTTCA TAGGTTGGGA
4051 GAGGTGGAGG CAGAAATAGG TAACTGAAAT GTTTTCTAAA ACGGAAGCCA
4101 TATCTTAATT ATACCAAGAA ATATTATTTA ATATAAGGAT AACTGACCTT
4151 CCTCAGACTT TGTTTTACCA TTTTTTTGTG GAGGGGACAT GCATGTATAG
4201 ACTGATGGTT TTTGTTTTCT TTTAAGAAAC GAACGTGCTC ATTTTTGTCA
4251 TATCTTTTTG CTCTGTAGGT GTCGCTACCT ACTGGATAGT GTTGCACAGC
4301 CTGTGACAAA GGATAAGTTT GCTTTGGAAT CTGAAGTAGA GTACTGTCTC
4351 GTGAAATTAA TTTTCTCATT CTGAATCTCA TTTTTTGTAT TATTTTCTTC
4401 TAAAACTTAG CGATTGTCTA CCTATCATTG TTTTATGTTA GTATTAAAAC
4451 TTTTATTAGA GATAACCATT TTAAAGAAAT AGGAGGGCTT AATTTTAATT
4501 TTTTTTTTCA CTTTGCAAAT AAGAAATAGT TGATAAATAC GTTGAGAGGT
4551 GTGATCTGAA AAAAAAAAAA TACATAGTGA CAGAAAAATG TTGTTTGGGA
4601 ACGCTTTCCC ACAAGAGAGG AGATACAAAT TTTGATCTAG GCTTATCTCA
4651 GTAAGTGTTT TTACTTTGAG TTGTATATCA TATGAGTATG ACTAATAATA
4701 CCTCTGTTTA ACTGAATGTT TGTTACATCA GATGACTTAT TATGGGAATC
4751 ATGAAATCAC CCTGTTACAC ACAGCATATA GTTTCTTTTT ATTTCTTGTA
4801 CACCTATTTT AACATCATAC CATATTTTTG CAGAGAGCTA TTTCTTTATT
4851 TTTATTCTTG GTTCTATATA ATTAGACTAA AATAATATTA GAAATTGTGG
4901 AAATTTAACT AGACATGGTA TCATGTGCCT GTAGTCCCAC CTATTCAAGA
4951 GATCAAGCCA GGAGAATTTC TTGATCCCAG GAGTTTAAGA CCAAGCTTGG
5001 CAATATAGCA AGAGCTTATC TCTAATTTTA AAAAGTTGTG GAATATTAGA
5051 AATTTAAATT CTGTTCTCAC ACCTGTATTA GAGAGGGTTT ACCCTGTGTT
5101 TTCCAAGTCT TTTTTATTAA GAGTATACTG TAAACTCTTT ATCTAATCGA
5151 AGAATATGCA TTTTGTTTAA AATAACAACC TGTTTGATAA GCAGAGACTC
5201 TTCATATTTC TGTGGTCAGA CTTTAACTTC ACAAGATTTT TGCATTGTAA
5251 TTAAAAAAAA ATCCGGTCGG GGTCTTTGTA TCTCATTCTA AATTTCAAAT
5301 TTCAGAGGCT TTTGTGCTTA GTTATTGAAA AAATAATTGT AAAGCTCCTG
5351 CATTCGTATG AGCCACTTGA AGCTAGAAAA ACTATTTGTA TGTATCTTGA
5401 GTACCCAGAA TAAGGTCTTG CATGTAAGAA ACATTTTATT AGTTTTTAAT
5451 GTGAATCAGC AAACAGGGAA ATGGGCTTTT TTTAATGGAA TGTTACAGGT
5501 AACATAATAT GCATAAGATA TCTAATAATT AAATCTATTA AATGTCTTGA
5551 ATGTTCTGAA TTTATCTTTT CTTCTAATGT TGAGAATCTA TAAGTTGAGG
5601 TGAGTTATGC TGAGAAAATA TGTCTGAATT TATCTTTTCT TCTAATGTTG
5651 AGAATCTATA AGTTGAGGTG AGTTATGCTG AGAAAATATG TCATAGAATA
5701 AAATGAAAAT TGGAAGAGAT GGAAAGGAAA TAGGAAAGGT GGATACAGCG
5751 TGTTTTCTAA TATCTTCCAA CTGGAAGCTT AGATTGGGAA TTTAGATTAA
5801 ATTTTCAAAG CCCCAACCAT GTTCTATTAA ATACATATTT TTCAGGACAT
5851 ACATTACTCT TTTGAATTCT GAATAAAAAT TCTTTCTCCA GGTGTTAAGT
 
5901 TCCTGGAATT CTATTGTTTT TTTTTTTCCT CTCTCTGTCA CTCTCTCTCT
5951 CTCTCTCTCT CTTTGTAGTA AGAATGTTTC AGCTTTCAGG TGAATGATCA
6001 TGGTTAACTC TTGGGATGTG TTATTTTATA GTAAACAATC TAAACTTTTT
6051 ATTACATAAG TATTTACACA AAACAAATTG TCGAGTAAAT GCTAACCAGC
6101 ATTATTAGGC ATATATTATG AACACAAGCT TTTCTTTTAA GCTCATATTT
6151 GATTGACTGG TCATGTCTCT TTTTTTGTTT GTTTCTCCCT CCTTCCCCTT
6201 TGTTTAAAAA TGATTTACCT CAGTCTAACT TTTTCCTTGC AGACCACATG
6251 TCTTTAGTGT CTATTCTTTC ATTTCACCTC TGTTTCTGTT GTCAGGATAC
6301 TTAGTTACAC CTTTCTATTC AGTAGCTATG TGTGGCTTTC ATTCGTGAAT
6351 CATTGCTCCA TAAAGTGGAT GGTTGCTTTG TTCTGTCTTT TTTGGAAGGA
6401 GCTGAAGTTA TACAATAATT TGTTTTTAGC TTTTGACACA CAGAATAGAA
6451 GCAACTTATA CTGATTCTGA TGCTAGCCCA TTTAAATATA TTACTACTAA
6501 AAACTACATT CTCACATTTT TTTCCCACAG GAAGTAATTC ACACAAATAT
6551 ATATCATGCA TAAATATTTT GAAAACTAAA GTGATTAATT GACTTTTGAT
6601 TAGTTTTGTT CAGTAATACT CTAAATTACG TATGAGTAAA TTTTTGTCCC
6651 AGTTTGTTGT GGTTAATAAA AGAAACTAAT GGATATCATA GTATTTGTAT
6701 AATAATCAAC CGGGGTTGAA AGGAGTAACA TTTTGAGAAT ATCTGTATCA
6751 CCAATCATAA TCATCATCTC ATGTGAATCT GTAGATAAGT AAATGGCAGA
6801 CTTGAGGTTT GAATCTACAT ATGAGTGACT TCAAAGTCTA TCCTTTTTGT
6851 GTTAGATCAT GTAGTAATGG TGGCAATTAT CATTATTTTA ACTTGCTGAA
6901 TGTTGTTCTT AAACCTATTG TGTCTTTCTT CTAGACTATT TCAGAACCAT
6951 ACTTTACGAA CAGAAGGACT ATTTCTCAAC AATCTGCAGA AAAGTGTAAG
7001 CTATTTGAAA CCTGACTATT GTATTATCTA CTGATTCTTT TTTTTTTTTC
7051 TTTCTTATTT TTTTGTAGAC AGAGTGTTAC TCACTGTTGC CCTGGCTGGA
7101 GTGCAGTGGC ATGATCTTGG CTCACTGCAA CTTCCACCTC CTGGGTTCAA
7151 GTGATTCTCC TGCCTCAGCC TCCTGAGTAG CTGGGATTAC AGGTGTGCAC
7201 CACCACACCT GGCTATTTTT GTATTTTTGG TAGAGATGGA ATTTCACCAT
7251 GTTGGCCAGG CTGGTCTTGA ACACCTGACC TCTTGTATGA TCTGCCTGCA
7301 TGGGCCCACA AAAGTGCTGG GATTAAAGTT GTGAGCCCCC ACACCCGGTT
7351 CTTATTTATT GATTCTTAAT TACACGCATT TCATCTACTC TTGACTTTTT
7401 TTTTACTGTA GTAGATGCTG CATGTGGCAT TGAAAAAACA AAATATGGAA
7451 CCTTTTTTTG AAGACCAAAA TGTTGATAAG GTAAATGAAG ATGTGGTTAA
7501 AAGCCAACAT AGAATAATCA GAGTCGAGTC CTGTTCACCA ACTCACTCTT
7551 ATCTGTTAAT GATCTCTAGT TTTACAATGG TAAATTGTTT TATTTGGAAA
7601 ATATTTTTCC CATGCTTTAT TCACTTGCCA TCTCCCTGTC TTTATAACGA
7651 TGACAAGGAT CCTATAAGGG AATGGAAGTT CTCCAGGTAA TAATAGAAAA
7701 GAAGTGTAAC AACAAGGGAA ATATATGCAT GGGACCAGGA TTCCTAAAAG
7751 GTCATGGGAG TAAATGATTG TTAGGTTTGC CATTAGGGAA GGAAAGAAGT
7801 AGAAAGCAAC CTGAAAGAAC AGCTCCACAA ATACAAAGGT GAAGAGGGGA
7851 AAGAAGTAAG AATACAGAGT TGAATAAGAG TGTCAAGATG ACAAAGATTA
7901 GTATAAAACA CCTCAAAAAC GGTGAATTTA AATGACAGTA GAATGTCTCC
7951 ATATCATATT ATAGAATTAT TTTAAATATA GATTAGGAAA AGAAAGCCAA
8001 GTAATTAAAA TAATGCTCAG ATTCTTCTGA GTGACTTACG GGTGATTTTA
8051 ATAAAGGATT GGAAGAAAAT TTATGGGTGA CTATATTTAA CATTCTGTTT
 
8101 AACATTTAAA AATAGCCAGG AGAGAATAAC TTGAATATTT CTAGTTTAAA
8151 TAAAACATAC ATATTTAAGG TAATGTGTAT CTCTGTTACC CTCATTAGAT
8201 TATATAGATG GAACAAATCA TCACACGTAC ACTGAAAATA TATGCATCTA
8251 TTTATTAATT TAAAAATTCT AAATGGAAAG AAAATTTAAT CCTAACTTTT
8301 TAAAATTTCA GTAAAGTGAT GTCTCATATT TTATCTAGTG AATATTGTGT
8351 TCATAGATCA TGCAAAGTAA AATGTGTTTT AGGCTATATC AGATTTTGAA
8401 TGAATCATTA ATTTTTGACT CCTGTTAAAA GTTTTTTAAA GTAATATTTG
8451 GTATATTCCC AAGTTGCTTA ACTAATTTAT TTCACTTTTA CATCATCTTG
8501 GCAGCTGTTT TAGCCTTTTT TTGATAATAG AGAAAATAAA TAAGTAATTA
8551 AATAATAGAA AATAAAAAAT AATAAAAATT AATAAAAAAA TTAATAAAAA
8601 TTAAAATTTC TGGTTAATTT TTTTTTTTTG AGAGGGAGTA TCGCTCTGTC
8651 GCCCAGGCTG GAGGGTGGTG GGTGATCTCG GCTCACTGTA AGGTCCACCT
8701 CCCGGGTTCA TGCCATTCTC CTGCCTCAAC CTCATGAGTA GCTGGGACTA
8751 TAGGTGTCCG CCATCACGCC CGGCTAATTT TTAAATATTT TTAGCGGAGA
8801 CGGGGTTTCA CTGTGTTAGC CAGGATGGTC TCGATCTCCT AACCTCATGA
8851 TCTGCCTGCC TCGGCCTCCC AAAGTGCTGG GATTACAGGC GTGAGCCACC
8901 ACACCTAGCC ATTTCTGGTT AATATTAAAG GAGGTTTCTA ATTTATGTTC
8951 ATTGAATGAG AAAAAATATC TTTTTAACCT GGAACCCTCT TAAAGAAATA
9001 AATTATTACT TATTTCTGGA GCTAGTCTGA CTGACCTTGT GCTGATAACC
9051 TCTGTACTTA AGAACATACA TGTGATGAAT GCAGTCAACA CTCATGATTG
9101 TTTTCTAAAC AAAGTACCTA TTTTGATAGA ATCGTAAGGA AAGAAATATT
9151 GCCAACAGTA CACAGAATTA TCTCTGGAAT ATTACTTTTT ACTTTTAAAA
9201 CTATCTCCTG CAGTTGGGAC GTTTTTTATT ATTCTCTGGA AGCATTATTA
9251 ACTTTAGTAT TTATAGTTAT ACCTTGCACA TAAATTTATT CAGCTCTAAA
9301 ACTCAGGAAT CCTTTTGACT TGATGTTTAA AATAAATTTC TTCTCTCTTC
9351 ATGTGAGTAT TGAGTCATGA GTGACAGGCA TAGTGTATTT GAAGACAAGT
9401 AAGTCTTCAG CTTGCATAGT CATATGATTA TTTTACTTGA TCCTTTCTCT
9451 GATTTTTAAC TATTATCTTT ATGGCATAAG TAGGCAATTA GAGCTATTAG
9501 TATATCATTT CAGGAGAATC AGAAAACCAT ATGAACTTTA AAATAGATTT
9551 TGCATTTCTC TCTTATATGT TAGAAGTACT TAAAATGCCT TATAATTCTA
9601 TAATAGAATA AACATTTGTG CAAGTTAAAA ACTTTTAAGA TGATTTTTTG
9651 AAAATGAGCT TTCTTAGGAC ACCTTGACTT ACTGGTTTAG ATAACTGTTT
9701 TCATTAAGTT CAAAAATGCA GATGACCATA ATATTCCAGA AATAAATATC
9751 TGCATACATT TAGAAAATAT ATTTTATATC TTTTAATCCA GTATAGAAAT
9801 ATATAATTGA AATTTTGAAT CACATGTTTT GTTTTCTTTT TATTTTCAAA
9851 ACTTCAGGGT TCTACATAGG TTTAAATTAT TGAATCCTAC CAGTTTAGTA
9901 TACTTTACAA ATGTTGACCT TCTCAACAAC ATATGGTTTT TTTGAGTAAG
9951 GTCATCTATT TCTACTCCAA ATGTATTTAC CCAGATCAGT CTTCTAAGTC
10001 CCCTTTGTGC CTCAAATTTA ATAGGTCTCA AACTGCCTTC CAGATTCTTT
10051 CCTGACTCTT TGTAATCCAT GCTGTCATCT CACTTCCCAA TTTTAGTAAA
10101 TAACACCAAG GAGGTAGTAA TGAAACATTG AAGCAGAAAA ACTGCACTCC
10151 AGGTTTCCCT CACATAGCCA ATCCAGTGAC TCACCAATTT TTGTATTTTC
10201 TACCTTTAAC CACTTCTCAT ATCTTATTAC TGTAATACAC TGTCGGCCCT
10251 ATACTTTTGG AATGTTCGTT TTTGTTTCTT TCTTCTCCCC ATGTTTATCT
 
10301 ATCTATATAT GTCTTTCAGG GTGACCTTTC TTAAACATGT TTACTTTGAT
10351 CTAAGTCATC TGTTTATGGC TGGACCCTCA TTACATGAAA CAAACATTTA
10401 CATTCTAGCA TAACACTTAT TTTGTGGTTT GGCCTGTTTC TCCCATTTTA
10451 TCTCTCCACT CCACTTTCTC TGTCTGTGTC CAAGTTTTAC CACGTTATTG
10501 TGGTATTTCT TATTGGCTAT GCTGTTTCAT GCTTTATTTT TTTGCACATG
10551 CTGCCCTCAC TAGAAACACT TCACACTCTT ACTCTTCCCT TGTCTATTTT
10601 AAAAATAGAA CCCTAAATTT GCAGTTTCTC AGAAGCTTCC CAAGTAGAAT
10651 TGTTATTGCC TTTGTGCTAT CACTGTATTT TATTGACTTG AATTGTAGAA
10701 ATAATGAATT GTGTCTTTCT GCTTTTGTTT GTTTTTATAA ATTTCTTTTT
10751 TCACTTGATA AATAAATAAA ATTGGTATTT TATTCATATT ATCAGATTTT
10801 CCAGTATAGA TCTTATGAAT TTATAGACAC GGAAAATGTT TCTTGAATTA
10851 CTGACTGATT AAGTAGTAAA TATGACATTT TCTGAAGATT TCTTTTTTTT
10901 TTTTTTAATA GGAAAGAAAA GCACTACCAG CAACTGGACA AAAAGCAAAT
10951 GGTATTGGTA TTATAAAAAG TGCTCCATGA GAGCAATCAA ATAATGATAA
11001 TGTTGTATTT TGTGTACAAT AAAAGATGGT GAGGTAGTGA ATATAGCTGA
11051 ATAATTTTCT ATGCTTTAAT AATATAATTT TTGAAAATAA ATATAACTAA
11101 TTTAAATATA ATTTAAAATA AATTTAAAAT TAAATTAATT GTTAAATTAC
11151 ATTAAATTAT AAGCCTAATT TTAATTAAAT TATAGTTATA ATTTATTAAT
11201 TTTAATAGTA AATATAATTT AATTTAAACA TACTTTTTCT TAAAACTTTG
11251 GTGAACACTT AAACTTGTAG ATCAAAATAT AATATTCATT GTTGAGAAAT
11301 GGACATTAGT ATATTTACAA AAAAATGTGA GGTGGGATTG TGTAAATTAA
11351 GAAAGCAGCT GGCTAGGTAT TTGGAGGTTT CTGATGAGGA AACTTGAGGG
11401 AACTCACTTT ATGTAGACTC AGTATATTCC CACTCAAAGA GAAGATTAAA
11451 TGATTGCTGC TTTGGAGCTT ACTGGAGGCA GAGGGTAGAA GAACGACAGG
11501 AAACCACAGA AACTCATTTC TTCTCTCTAT AGGGGTTACA CATCAATGAT
11551 ATGTGCTTCA TTCATGTTTG TTAGTGAACT GGGATGCACT TGGATATCAA
11601 AACATTGGCA GTTTTCTTTA AAACAATGCT GTTTTTGATG AAATAGCCAT
11651 TGTATAAAAG TACCTCAGAG GCTGCTATGC TATAGCATAT CAAACTGACT
11701 AGAAAAAAAA CAAACGAGAA ATTTATTTCT TTAGTACTAA AAGTGTAACT
11751 GTCAATAATC ATGGCAAATA TTTTGATATG TAAAAGTTGA TTAATCCAGG
11801 CGCAGTGACT CACGGCTGTA ATCCCAGCAC ATTGGGAGGC TGAGGCGGGA
11851 GGATCACGAG GTCAGGAGAT GGAGACCATC CTGGCTAACA TGGTGAAACC
11901 CCATCTCTAC TAAAAATACA AAAAATTAGC CAGGCGTGGT GGCATGTGCC
11951 TGTAGTCCCA GCTACTAGGG AGCCTGAGGC AGGAGAATCA CTTGAACCCG
12001 GGCGGCGGAG GTTGCTGTGA GCCGAGATTG TGCCACTGCA CTCCAGCCTA
12051 GGTAACAGAG CAAGACTCCA TGTCAAAAAA GAAAGTTGAT TAGTATGAAA
12101 AAAAAGTCAA TGGTGATTCA GAGATTTTTG GTTACATTTT GTAAATGAAA
12151 ATCTGAGTAC TCATTAGTTA TTTGATGTGT AACATATACT TTTTTTGCAT
12201 AAGGGAATGA AAAGATGGCA AGAGAACTAA AGTTGAGAAT CCAGAAGTTG
12251 AAAATATCAG AAGTCTTCAT GACTGTGGAT AACATGAGTA TTTTTAGAAA
12301 CGATTTTTCT CCAAGTAGAT ATCTAAACTA ATGATTGAGA GCATTTCCTG
12351 CCAGCAGAAG CGAATGATAC ATTTTCTTTT CTTTTCTTTT TTTTTGAGAC
12401 CGAGTCTCGC TCTGTCACCC AGGCTTGAAT GCAGTTGCGG GATCTCAGCT
12451 CACTGCCAAC TCCGCCTCCC GGGGTTCACG CCATTCTCCT GCCTCAGCCT
 
12501 CCTGAGTAGC TGGGACTACA GGCGCCTGCC ACCAAGCCCA GCTACTTTTA
12551 TGCATTTTTA GTAGAGATGG GGTTTCACGG TGTTAGCCAG GATGGTATCG
12601 ATCTGCTGAC TTCGTGATCT GCCCGCCTCG GCCTCCCAAA GTGCTGGGAT
12651 TACAGGCGTG AGCCACCGCG CCCGGCCGCG AATAATACAT TTTCATGCTC
12701 TCTCATTGCA ACTTCATAGT TTCTAACATT TATTCTTCTG GGGTCCGCTT
12751 TGGTTCTCTC ATTTGACGTC ACTTTTTTTG GCTTCATCCA GCAGATTCGC
12801 ATTCGTAGAA ATACTTTTCT ATGTTACTTG TAGTCATGTG AAACCTAATC
12851 TCACCCTTGC AATCTGGACT GCATGTTTTA TAGAATCTAT ATTGTGATTT
12901 TTCATGTGTA TATTCCTGTC ATGTTTGTGT GTTAACCAAA ACAAGAGCAA
12951 AGCAACCCAA AGCCTAATGG GTAACAATTT CAAGGGCAAG GACGAAGATT
13001 TCAGCTGGAT ACAAACACTG CATGATTGAT GGTAGGCTAT GTCATATTTC
13051 CCTGTAGTCA ATTTTGTGTT CCTTTTGTTT TTTAGTGTCT CCTGAGCAAC
13101 CGCCTTTATT CACGGTAAGC ATATTTTCTT TAATTATTAA CAAAATGCTC
13151 TGTGATATAT ACATGATATA TTAATCATTA TGTTGTCAAA CCCATTCAGC
13201 ATACGGTGAA AGACAGAGAT CACATTTCAA CTAGATTCTT AGGAAGTATG
13251 GATTCACCAA CTTCCAGTGA AGGTAAATTT GCCACTACAA ATTTCATTCT
13301 AGAAAATGTA GATGAAAATA TTGAAAATGC TCATAGTTTT TTGATTCCCA
13351 CTTTTTATCC AAATGAGATG GAAGGATTTG ATGTAAATAT GCTGATGTTC
13401 TTGTTAATAT CTTTGTTTAT AAAATGATAT TTAAGGCATA AGGCGGACAT
13451 TTTACACCAT GAGCTCTAGC CCAAATGCTT TTCCTTTAAA GTTGTCATCA
13501 TCTGAAGATC CCAGTTTTGA ATTCCTGTGC ATGTTAGGGA TTTGAGGAGG
13551 TGTATTTTGA CACTAAGTAT TTTCAGTGCT TCAAAATTGA TTGGAATACT
13601 CTTCTCTCTT CTTCATCTAG AGAAAAGCTA TACCTGCTGA CGTTACAATT
13651 GTTTTAGAAC TTCAACTCCT TTATGTCAGT GTTGTTACAT TGAGAATCTT
13701 TACTTAATCA CATCTTCTGA TTACCAGATT GAGTTTCTGC ATGTGTATGT
13751 GTGTGTGCAT CTCAGATTAA GAATGAATAA AATGATTAAT CATTCTTTTG
13801 TAACTCTTTG GTAGATACAG TGTTTTAAAA CAATGATTCT GAGCTGTTTT
13851 GGCCTTAGAA TATTTTCTTC TACTACAATT TATTGCCTAC AGGTAGCCAA
13901 CAGCCTGAAT TAACGTTTTT TACTTTTAAG TCACTGCAAT GCACATTAAA
13951 AATACTTTAC AAGATACTTG CACTTTCATA GGTAATATGT AGAATCTGTT
14001 TCCAAGTATC AAGTCTTCTA TACGATTTCA CACAGCGTAC ATAATAGCTG
14051 CAACTCGGCT TTTCTAATCA GTGGTATATA TTTTGGATCT ATCCAGGTTG
14101 ACACAATTTG TTCATCTTTG ATTTGTTGTA TGGTCTTGTG GTATGCCATT
14151 TTATGACTGC ACCACAATTA ATTAAAGTCT CTTCATGCTG ATACAAAATG
14201 AACATAAATA TGATGACATG CCAACATAGA TTTGCTTATG TGGTTGTCTT
14251 TATTGATTTG TACTATATAA GAAATTAAAT AGAAGTATTT CAGATACCCT
14301 GAGTATAGCT GTATACACTG CCATAGCTGT ATTGACTACA TTCATTACCC
14351 CTTAGAGCCA TGTTTTTCCA TCATATTATG ACTCTGAAAA GCCCTAGTGT
14401 ATGCTTTTCA GAGAATGACA TTGGAAAGAA GAAACGGCCA AAGTATCATT
14451 TGGTACCTTG GCTTCCTTAC ATAGATGTTG AACTCCAAGA ATGATCAAAT
14501 CACAGTTTAG AACCCCTTTG TTGGCCCTAT AAAGGGTTTC TGGATATCAA
14551 ATGATAAATA GTCTCCAGAT GAAGAAATGC TCTATAACGC CCCACCGCTG
14601 TGTTTTCCTA TATTTTGTCC TTTTCTGAAA ACTTTAAATA CACGAATTTT
14651 TGGTAGAAAT GAAAATCTTT CTGTTTTATA TATTTGTTTC TGTCCATGGC
 
14701 ACTCTGATAT CTTTGAATCT AGTAAGGATA TAGCCTTGTC TTATTTATAC
14751 CAGCAAGCAG TGTTGTCACT TAATGCTCTC ATTTTTCACG TAAATGACTG
14801 ACATTTTCCC AAGTCTTCGC AAGTGATTTT TGAAGACGTT GCTGCATTAA
14851 GCAGAGACTA TGTCATTGTA ATTGCAGAAG TTTTTAAATT AAATATGTTT
14901 AATAAAATTT TAGAGCCTGT TTCTGTGGAA CACGTAACAC ATAATGGTGG
14951 TGTAATGTGT ATTTAACCAT AAATTAGCTT CACAAAAATT TGTGTACTTA
15001 AAAGTGTTTA TATCCAAAGA AATTCTTATT TACTGCTCAG TAATCTCTTG
15051 TGTAGAAGAA AATATGTAAC ATAGTTTGCT GAGTCTTTGA TAATAACCCC
15101 CAGTGTAAAA TAAAGCTGTA AAGATGAAAT GAGAGTTGAT ACTCAATGAA
15151 ACTGATGTGA GTGAATACAA ACTATGAAAC TGTGTCTAGG GGAGAGAGGA
15201 GGACATGGGG CTGCTATTGT GAAGAAGGAA TTTGTACAAG TTAGTCCATT
15251 TTCTGTAACT TTTATATTCT AATAAAGGAA AACCATATCT TCATATTTAT
15301 AAAAATGGGA TTTTACTGGT TTGATCACAG GGGTTGTGAG AATAATGAAG
15351 AACAAAATGT GGAAGGCAAA GAATGAAGAC CAGATAGTGA GATTAGATTT
15401 ATCAACAAAA AAGAGTGCAA GTGGGGTGGG ATGGTGTAAA TAAAGACAGC
15451 AGCTGGCTAG GTATTTGGGG GTTTCTGATG AGGAAACCTG AGAAAACTCA
15501 CTTTATGTGG ACCTGGTATA TTCGCAGTCG AACAGAATAT TCGATTATTG
15551 TTGCTTCAGA GCTTAATGGA AGCAGAGGTG GAAGAATGAG TAAACCACAG
15601 GGACTCAATT CTTCTCTCTG TAGGGGTAAC ACATCGACAC TAGGATAGGT
15651 GCTTCATGTT AGCAAAATGC GATGCAGTGC ATGTCAAACT GTGGAAGCAA
15701 AACAATCAAG AAATAAATTT CTTAAGTATT ATTTATTTAT TTATCGTTAT
15751 TATACTTTAA GTTTTAGGGT ACATGTGCAC AACGTGCAGG TTTGTTAAAA
15801 TGTAACTGTC AATGATCATG GCAAATATTT TGATATATAA AAGGTGATTA
15851 ACATGAAAAA AGCCTCTGAT ATTTCAAATA TTTTGGTTAG ATTTGTTTTA
15901 TGGGAATCTA ATTACATATT AGGTATTTGG AGTGTAATGT ATACATTTCT
15951 TGCATAAGTG AATGAAGAGA TGGCAGGAGG GCTAAAGTTG AGAATCCAGG
16001 AAATGGAAAA ACACCAGAAG TCTGCATGAC TGTGGACAAC ATGAGTATTT
16051 TTGTTAACTA TGCTTCTGTA TGTAGATATC TGAACTAATG AACTGAGAAT
16101 ACATCCTGCA AGCAGAAGGG AATGATACAT TTTAATGAAA CTGATAGTTT
16151 TTAAGTTAGA GGACAAAATG AAGAACACGA AAACTATTGT AGTATTGTAG
16201 TATAAATGGT TCTTGGGTGA TTTCTTCAGG GTACACCATA GCGTTCTACC
16251 ATGGGCTTTG ACATTTATCC TCCTGGGGTC CTTTCATTTG ACATCACATT
16301 TTTTGGCTTC AGTTAAGAGA ATCACATTGG TAGAAGTACT TCCTTGTGTT
16351 AGTTGTAGTC ATGTGAAACC TAATCTCTCT CTTGAAATCT GGACTACATG
16401 TTTTATGAAA ACTTAATTAT GTGTCCCTTT TGCTTTGTAG AATCTTCTGA
16451 GCGACCTCCT TTATCCACAG TAAACAAATA TATTTTCGTT TTTAATTAAC
16501 AAAATGCTTT GTGATATATA TATGATATAT TAATCAATTA ATCATTATGT
16551 TGTAAAACCC ATTCAGCTTA CTCTGAAAGA GGCAGATCCC AGTTCAAAAG
16601 TAGCCATGAG AAGGAAGGAT TCACCACCTC CAGGAAAAGG TAAATTTGCC
16651 AATACAAATT TCATCTGGAA AGAAGGACAT GAATATATTG GAAATGTTCA
16701 TGGTCTTCTG ATTCTTATTT CTTTTACCCC AATAAGATAG AAGGATTTGA
16751 TCTAAATGAT GCTGATGCTG TTGTTAGTGT CTATGATTAT AAAATGATAT
16801 TTAGAAGAAC AAGGAAAAGA AATGTTTAAA ATGTGAGCTC TAGCTCAGAT
16851 GCTTTTCCTT TTAGGTTGTG ATAAATATCC AATGTGGAAT TTGTATGCAT
 
16901 GTTAGGGGTT CAAGGAGGTG AATTTTGAAA CTATAAATAT TTTTCAGTGT
16951 TTCAATTTCT GGTTGCAATA CTGTCCTTTA CCTAGAGAAA AGCTATACCA
17001 GCTGACATTA CAATTGTGTT AGAACTTCAA CTTCTTTATG TCGGTGTTGC
17051 CACACTGAGA ACCTTCAAAT CCTCACCCAG TCACATGCTC TGATTACCAG
17101 CTTTTGAATT TCTGCATGTG TATGTGCGTG TGTGCTTTTG TTTGTGAGTT
17151 TGGGTGTGTG TGATACCTCT GATTCTGGAA AATGAATAAA GTCATTAATC
17201 ATTTTTTGGT GACTCTTTGG TAGATACAGG GTTTTAAAGC AATGATTCTG
17251 AGCTGTTTTA GCCTTAGAAT CTTTTCTACT ACTACAATTC ATTGCCTACA
17301 GGTAACCAAC AACCTGAATT AATGTTGGTT CGTTTGTTTT GTATTATACC
17351 ATAAGTTCAG GGATACATGT GAAGAACATG CAGGTTTTTT ACATAGGCAT
17401 ACATGTGCCA TGGTGGTTTA CTGTACCCAT CAACCCATCA GCTACATTAG
17451 GTATTTCTAC TAATACTATC CCTCCCCTAG GACCCCAACC CCCTGACAGG
17501 CCCCGGTGTG TGATGTTCGC CTCCCTGTGT CCATGTGTTC TCATTGTTCA
17551 ACTCCCACTT ACGAGTGAGA AAATGTGGTG TTTGGTTTTC TTTTCCTGTG
17601 TTAGTTTGCT GAGAATGATG GTTTCAGGCT TCATCCATGT CTTTGCAAAG
17651 GACGTGAACT CATCCTTTTT TATGGCTGCA TAGTATTCCA TGATGTGTAT
17701 ATGCCACATT TTCTTTATCC AGCCTATCAC TGATGGGCAT GCGGGTTGGG
17751 TCCAAGTCTT TGCTATTGTG AATAGTGCTG CAATAGACAT ACATGTGCAT
17801 GTGTCTTTAG AGTAGAATTA TTTTTAATCT TTGGGGTATG TATTCAGTAA
17851 TGGGATTGCT GGGTCAAATA GTATTTCTTG TTCTAGATCC TTGAAGAATC
17901 ACCACACTGT CTTCCACAGT GGTTGAAATA ATTGACACTC CCACCAACAA
17951 TATAAAAGTG TTCCTATTTC TCCACATCCT CTCCAGCATC TGTTGTTTCC
18001 TGACTTTTTA ATGATCACCG TTCTAACTGG CGTGAGATGG TATCTCATTG
18051 TGGTTTTGAT TTGCATTTCT CTAATGTTCA GTGATGATGA GCCTTTTTTT
18101 CATATGTTTA CTGGCTGAAT AGATGTCTTC CTTTGAGCAT ATCCTTCACC
18151 CACTTTTTGA TGAGGTTGTC TGTTCTTTTC TCATAAATTT GTTTAAGTTC
18201 CTTTTAGATT CTGGATATTA GCCTTTTGTC AGATGGAGAG ATTGCAAACA
18251 TTTTCTCCTG TTCTGTGGGT TGCCTGTTCA CTCTGATAAT GGTATTGGAA
18301 GTTCTGGCCA GGGCAATCAG ACAAGAGGAA AAAATAAAGG GTATTCAAAT
18351 AGGAAAAAAG GAAGTCAAAT TGTCTCTGCA GATGATATGA TTGTATATTT
18401 AGGAAACCAA AACGTCTCAG CCCCAAATCT CCTTCAGCTG ATAAGCAACA
18451 TCAGCAAAGT CTCAGGATAC AAAATCAGTG TGCAAAAATC GCAATCATTC
18501 CTATACAACG ATAAAAGACA AACAGCCAAA TCATGAGTGA ATGTCCATTC
18551 ACAGTTGCTA CAAAGAGAAT AAAATACCTA GGAATACAAC TCACAAGGGA
18601 TGGGAAAGAC CTCTTCAAGA ACTACAAACC ACTGCTCAAG GATATAAGAG
18651 AGGACACAAA CAAATGGAAA AACATTCCAT GCTCATGGAT AGGAAGAAGC
18701 AATATCATGA AAATGTCTAT ACTGCCCAAA GTAATTTATA GGTTCAGTGC
18751 TATAGACTAC CATTGACTTT CTTCACAGAA TTAGAAAAAA ACTACTGGAA
18801 ATTTCATATG GAACCAAAAA AGAGCTCATA TAGCCGAGAC AATTGTAACC
18851 AAAAAGAACA GAGCTGGAGG CATCACGCTG TCTGACTTCA AACTATACTA
18901 CAAGGCTATA GTAATGAAAA CAACATGGTA CAGGTACCAA AACAGGGATA
18951 TAGACCAATG GAACAGAACA GAGGCCTCAG AAGTAACACC ATACATCTAC
19001 AACCATATGA TCTTTGACAG ACCTGACAAA AAATAATCAA TGGGGAAAAG
19051 ATTACCTATT TAATAAATGG TGTTGGGAAA ACTGGCTAGC CTTATGCAGG
 
19101 AAACTGAAAC TGGACCACTT CCTTACACCT TATACAAAAA TTAACTCAAG
19151 ATAAATTAAA GACTTAAATG TTTAAGTAAG ACCTAAAACC ATAATAACCC
19201 TAGAAGACAA CCTAGGCAAT ACCATTCAGG ACATTGGCAT GGGCAAAGAC
19251 TTCATGACTA AAACACCAAA AGCAGTGGCA ACAAAAGCCA AAATTGACAA
19301 ACGGGATCTA ATTCAACTAA GGAACTTGTG CAGCTTTATT TGGAAGTGTG
19351 CAATGAGGTA CGTCTGAGTT TCAAAAATGA AGAAAGTAAG TAGTCATGCT
19401 TTCCTGACTC TTTGGTAGAC AGCCTTTTAA GACGGTGATT CTGAGCTGTT
19451 ACTGTTTTGG GTTTTCTATA ATACTAAAGC TTACTGCCAA AATGTAACCA
19501 AGAGCTTGAA TTAAGTAAAA AGAAATCACC CAAATGCACA TTAAAAACCT
19551 CTTACAACAT ATGTGCACAT TCATAGATAA CATGTAGAAC TTGATTTTGT
19601 GTATTAAAAA CTTGTAGAAA AGTTCAGGCA GTGCACTTAT TGAATGCAAC
19651 TTGGTCTTTG TAAAATCAGT GATATATATT TCAGATCTAT CCACCTTGAC
19701 CCAGTGAGGT ATTTCTTGAT TTATTGTATG ATCTCATGAT ATGCCATGTG
19751 ATGACTACAG CATATTATGC TCTCTTCATG CTGATACCAT ATGGACTTAA
19801 ATATGATGAC ATACCAGCAT GGATATGCTT ACGTGGTTGG TTTTATTGAT
19851 TTGTACTATA TTAGAAATGA AACAGAAGTA TTGGAAATCC TAGCAAGCAT
19901 AGCTGTATCT CTCCCATGGC TGTGTTAATT GCAACTGTTT CCCCCTTAAA
19951 GCATGTCTGT TTGACATGTC CTAACTCTGA GAAAATCCAG TGTGTGCTTT
20001 TCAGAGAATA GCAGTAAGGA GTGGAAATGG CCAATGGTCA AAGTGTTACT
20051 TGTCCTCTTG GCTCCCCTTC ATGAATGTTA AACTCTAAAC TACTCAGGTC
20101 ACAATTTAGA ACCCCTTTGT TGATCCCTAT AGAGTGTTCC CAGATATCAA
20151 ATGACAAATA GGTCTTTGAT GAAGAAACAC CCTGTAAAGC CATATTGCTC
20201 TGGTTTTTGT GTGTGAGTGT GTGTGTGTGT ATGTGTGTGT ATTTTCTTCT
20251 CTTTTGAAAA CTGTAAATAG AGGAATTTTC ATTACAAATG AAAATGTTTC
20301 TGTTCCATAT TTATTTCCTG TCTAATGTAC TTTGCTCTTC CTGGATCTAG
20351 TAAGGATCTC AGCTTGTCTT TTTTATACCT GCAAAAAATT ACATCAAGCT
20401 TCATTTTTCA TGTCAATTAC TGACATATTT TCAAGCCTTC ACAAGTTATT
20451 TCTGAAGATT TGGTGCATCA AGGAGAGACT GTCATTGTAG TTAAAGAAGT
20501 TTTTAAATAG GTTATATTCA ATAAAATTTC AGAGCTTGTT TCTCTGGAAA
20551 GCATAGACAT AGTGGTGTTA TGGGTAGTTA AACATAAAAT AGCTCCACAA
20601 AGTGTTGTGT ACATAAAAGT GTTCATATCC TGGAAAATTC TACTTTATTG
20651 CTCAATACTG TCTGCTGGAG AGGAAAATAG GTAAGATAGG CTGCTGAGCC
20701 TATGATAATA ACTCATAATA TGAGGTGAAA GCATAGAGAC AAAATGAGAG
20751 ATGATAGATA CTCAAATGGA TGTGAGTGAA GAACAGCTGT GAACGTGTCT
20801 ATGGGAGATA GGAGGCCATG GGGCTGCTTT TGTGAAGAAG GAATTTGTAC
20851 ACGTTAGTCA AGTGTCTTGA CACATTTAAC ATTTTAATAA AGCTAAACCT
20901 TATCTTCAGA TGTGTCAGAA TGGGATTGTA CAGATGTCAC ATTACAGTGG
20951 TGGTGAAAAT AATGAAGAAA CGAATGTAAG GGTCAAAGAA TCAAGTCCAC
21001 CAATATGGAT ATTAGATTTA TGAATGAAAA AGAGTGTATG TCAAATTGGG
21051 CAGGTGTAAA CAAAGAAAGC AGCTAGCTAG GTAATTTGGA GGTTTCTGAT
21101 GAGGAGATTT GTGGGAAGTC ACTTAATGGA AAGCAGAGGT TAGAAGGATG
21151 AGGGTGACCC ACAGGATCTC ATTTCTTCTC CCTAGAAGTT TTGCACATCA
21201 ATGATACATG CTTCATTCAC ATCAGTTTTT CTTTTTTGGA AAGCTGTGTT
21251 TGCTGGTGTA GTTATTTTGT AAAAGAACCT GAGAGACCCC TATGGTATAT
 
21301 CATGTGAAAC TAGATTTAGA AAAAAGGAAT CAAATAATGC ATTTTTAGAG
21351 TACTAAACAG ATAACTGCCA ATAATGGTGA CAATCATGAC ATATGTATAT
21401 ATATGTATTA TGTCGTATTG GTTGGTTATT TATAAGAAAA GAAGTCTCTA
21451 GTGATTTAGA AACTTTAGTT TATTTTCATA GGAATCTGAT TACACATTAT
21501 TTCACTGATG TGTATGTTTT TGCAAAAGTG GACGAAGAGA CGGTGAGAAG
21551 GCCGAACTGC TGAATCCAGG AAATGTAAAA ACATCAGGAG TCTTCATGAG
21601 CATAAACAAA ATGATTTTTT AAATTATAAC TCTTAGATTA AGTGAACTCA
21651 CTTCAGATGC ATTCAGAATA TTTGCATAAA GGATGATTTG ATTTTTGGCT
21701 GTTCCAGGAA CTACTGGAAG CAGGAAACAG TGATAGAATT GGGATAAACC
21751 ACAGTGACTC ATTATTCCTC TTTGTTACTA TTGGGCACCA GAGGTATATG
21801 TTTTGTTGAT ATTGGTTATT CAAATGAGAT AAACGTGAAT ATGCATACAT
21851 TGGCTTTGTT TTTCAAGGAG GTATTGGATA AAATAGCAAC TTAATAAAAA
21901 TTCTCTAGAG AATAACATGA TAATTTAACC AGACTATTTT AGAAGTGAAA
21951 ATAATGTTGA ATTCATTACT TGACTCCCAA ATGGTTATTT TCAAGGAATA
22001 TTGGCGTGAT TTCCAGATGT AAAAGCTTAT TCATATCTAA TGCTTGTAGC
22051 AATTTTATTT TGTATAAGTA TGTCAAATTT GGTAATTTAT TATACTTTTT
22101 GATGAAGTTT ATACATTATA CCTTGTTGCC ATGAGTGGAT GAAGATTTCG
22151 GAAGGCTAAA CTAGAAGATG CAAGAGATGT AGGCACATTA TTACACCATA
22201 TGGGTATGAG AAATAATGAA CATTACATAC TATAATTCAC CAAACATATA
22251 TCCAAGCTGA TTAAATTAGG ACACTTCCAC TGAAGTGATG TGAAGTGTAC
22301 ATTCAACTAA AGTGTCATTA TAATTGTGTA CCTTCTCACT GATTGGTCAA
22351 GTTAAAGAAC ATGATGAATG TTTGCAGTAA AATGTTCCAA ATCATTCTGA
22401 TATCTTGCAT GAAAGACACG CGGGTGCATG TATCACCTGC TTTGACGTTG
22451 ATTCCCAGGT GTATGAGTTG AACTCTGATT TTAGATCACA TTTGTCCTCA
22501 ACACTCAGCA TATCCACATT GATATTGACA TGGCTTTATT TTAGTTCTAG
22551 ACATATGGAG AAAGTTATAT CACATTTGAA ATTGTCAGTA TATATTTCTT
22601 GAAGCCTGTA TTACTATTTT CTTCAGTGTA TTTCCTTCAT GTTTAGTCCC
22651 AAGAAACAAA GTATAAAATA TCAAAGCCTA CAGTAATACA GGCAGGAGTA
22701 TAAAACTTGA TGCTAACATG CTATCCATGC ATTAATGTAT GGATAACATT
22751 ATCATATTTA CATATGATTG ATTATGTAAC CCTTTTGCTT TTCAGTGTCT
22801 TCTCAGAAAC AACCAGCTGA GAAGGTAATT AAAGTCTCAT TTATCTGTTG
22851 AATTATTAAC TGTATAGTCT ATGAAACCTA CTTTACGTAT TATTTTGTTT
22901 TCAAATCCCA TTCAGGCTAC AAGTAATGAG AAATATTCTG TTTCAAATAC
22951 AGCCACAGAA ATAAAGGGGG GACCAATATC TGGGACAGGT AATTTTGCAA
23001 AACACATCTA ATGTCACGTT CAATCAAGAT AGAAGAGAAC GTCCCTTCTC
23051 CAAATAAATC AGTGGAGAGT TCATCTAAGC TGCACGTTCT GATTCAGTAT
23101 GCCTGAGATT GTTCATTTGT AGTGAGTTCT CAGGTGACCC TGATGCTGCT
23151 GGTCCTTGGC CATGATCTGA GTAGTAAGAT TATAGACTTC CCTACATTGA
23201 AATTGGGAAG AAGAACCATT GGAGAGCAGT TCAACACATA CCAGGCTAAG
23251 GGAGCAGCAT AATTTTGCTT TAATTCTACA GCATGTTTCC ATCAAGAGGG
23301 GAAAGAGAAC GAGATGAAGT AATAGATATT ATAGGCATCA TATCGTATTG
23351 TTATAAACAG AGGGACAAGT GATCTGAATA ACTCCACAAA CACTGTAGAA
23401 TGAGAACTAA GAAGACCACT GATGGAGCAA TTATTTTCCT CAAGGAAGAG
23451 GGATTGTGAG GCAGGAAGGA GGAAAAAGAA GAAAGTATTT ATGTAATTTT
 
23501 GGGGTTTCTG CTGAGGAAAC CTGAGTGAAC TCATTTCAGA TGCATTTGGA
23551 ATATTTGCAT TAAAGAAGAT TTGATTTTGG CTGCTCCAAG AAATACTGGA
23601 AGCAGGAAAC AATGCTAGAA TCGGGATAAA CCACAGTGAC TCATTACTCC
23651 TCTTTGTTAC TATTAGGCAT CAGAGATACA TGTTTTGTTG ACTTTAATTA
23701 TAAAAATGAG ATAAACTTGC ATATGAATAC ATTGGCTTCC TTGTTCAAGG
23751 AGCTACCTCT TGGATAAAAT AGCTATTTCA CAAAACTTCT TTAGAGAATA
23801 ACATGATAAT CCCAACAAGG CTATTTTAGA AACAAAAATG ATGTTGAATT
23851 CTAATTAACT CCTAAAGTGG TCATTTTCAA TGAATACTGG GGTGATTTCT
23901 GAATGTAAAA CTTATCAATA TCTAATGCTT GTAGCAGTTT TACTTTGTAG
23951 AAGTATGTTA ACATCGGTAA TTGATGATAT TTTTATTGAG GCTAATATAT
24001 TATCCTTTGG TGCCATGAGT GGATGAAGAA ACTTTCAGAA GGCTAAACTA
24051 GTGGATACAA GAAACTTAGG CAAATTATTA CACCACATGG GTGTGAGAAA
24101 TAATGAATAT TATCTACTAG GTTTCAGGAA ACATATGTCC AAGGTGATCA
24151 ACTTAGGACA CTTCCACTGA AGAGACATGA AATGTAAATT TAACTGAATT
24201 GTCATTGTAA TTGTGTATGT TCTACTTATT GGGCAAGTTA AAGGGCACGA
24251 TGAACGTTTG TAGTATAATG GTGTAAATCC TTCTAATTTC TTGCAAGAAA
24301 GACATACTGG GTCATGTACC ACCTGCTTTG ACATTGATTC TCAGGTGTGT
24351 GAGTTACTCC TCTGATTTGT CCTCATCACT CAGCATATCC ACATTGATAT
24401 TGACACGGTT TTATTTTAGT TTTAGACATA TCACGAATCA TACCATGTTT
24451 GAAATTGTAA GGGTATATTT TGTGAAGCCT GTATTCCTTT TTTTTCAGTG
24501 TATTTCTGTC TTGTTCCAGT CTCCAGACAA AAAGTAGGAA ACATCAAAGC
24551 CTACACTAGT GCAGGCAGGA AGATACAGCT TGATAATAAC ACTGCATGAT
24601 ATATGGATAA ATTTATCATA TGTACATATG AGTGATTATG TATCCCTTTT
24651 GCTTTTCAGT GTCTTCTCAG AAACAACCAG CTGAGAAGGT AAACTCTCCT
24701 TTATATTTTT TATTAGTAAC TGTATGGTCT TTGAAACATT CTTTATATAT
24751 TGATAATTTG CTCCAAATTA CTTTCAGGCT ACAAGTGACA AGACAGATTC
24801 TGTTTTGAAT ATAGCTACAG AAATAAAGGA TGCTTTAAAG TTCATATGGA
24851 ACCAAAAAAG AGCCTGCATC ACCAAGTCAA TCCTAAGCCA AAAGAACAAA
24901 GCTGCAGGCA TCACACTACC TGACTTCAAA CTATACTACA AGGCTACAGT
24951 AACCAAAACA GCATGGTACT GGTACCAAAA CAGAGATATA GATCAATGGA
25001 ACAGAACAGA GCCCTCAGAA ATAACACCGC ATATCTACAA CTATCTGATC
25051 TTTGACAAAC CTGAGAAAAA CAAGCAATGG GGAAAGGATT CCCTATTTAA
25101 TAAATGGTGC TGGGAAAACT GGCTAGCCAT ATGGAGAAAG CTGAAACTGG
25151 ATCCTTTCCT TACACCTTAT ACAAAAATCA ATTCAAGATG GATTAAAGAC
25201 TTAAACATTA GACCTAAAAG CATAAAAACC CTAGAAGAAA ACCTAGGCAA
25251 TACCATTCAG GACATAGGCA TGGGCAAGGA CTTCATGTCT AAAACACAAA
25301 AAGCAATGGC AACAAAAGAC AAAATTGACA AATGGGATCT AATTAAACTA
25351 AAGAGCTTCT GCACAGCAAG AGAAACTACC ATCAGAGTGA ACAGGCAACC
25401 TACAAAATGG GAGAAAATTT TCGCAACCTA CTCATTGACA AAGGGCTAAT
25451 ATCCAGAATC TACAATGAAC TGAAACAAAT TTACAAGAAA AAAACAAACA
25501 ACCCCATCAA AAAGTGGGCA AAGGACATGA ACAGACACTT CTCAAAAGAA
25551 GACATTTATG CAGCCAAAAA ACACATGAAA AAATGCTCAT CATCACTGGC
25601 CATCAGAGAA ATGCAAATCA AAACCACAGA GATACCATCT CACCCCAGTT
25651 AGAATGGCAA TCATTAAAAA GTCAGGAAAC AACAGGTGCT GGAGAGGATG
 
25701 TGGAGAAATA GGAACATTTT TACACTGTTG TTGGGACTGT AAACTAGTTC
25751 AACCATTGTG GAAGTCAGTG TGGCGATTCC TCAGGGATCT AGAACTAGAA
25801 ATACCATTTG ACCCAGCAAT CCCATTACTG GGTATATACC CAAAGGACTA
25851 TAAATCATGC TGCTATAAAG ACACATGCAC GTGTATGTTT ATTGCGGCAC
25901 TATTCACAAT AGCAAAGACT TGGAACCAAC CCAAATGTCC AACAATGATA
25951 GACTGGATTA AGAAAATGTG GCACATATAC ACCATGGAAT ACTATGCAGC
26001 CATAAAAAAT GATGAGTTCA TGTCCTTTGT AGGGACATGG ATGAAATTGG
26051 AAATCATCAT TCTCAGTAAA CTATCGCAAG AACAAAAAAC CAAACACCGC
26101 ATATTCTCAC TCATAGGTGG GAATTGAACA ATGATATCAC ATGGACACAG
26151 GAAGGGGAAC ATCACACTCT GGGGACTGTG GTGGGGTGGG AGGAGGGGGG
26201 AGGGATAGCT TTGGGAGATA TACCTATTGC TAGATGATGA GTTAGTGTGC
26251 AGCGCACCAG CATGGCACAT GTATACATAT GTAACTAACC TGCACAATGT
26301 GCACATGTAC CCTAAAACTT TAATAATAAA AGAAAGAAAA AGAAAAAAAA
26351 TTAAATATGG TACTGAAGCA AATAAACCAC TAATAGCTGA GAAAAATAAA
26401 TAAAAATAAA TTGTTGATGT AAAAAAAAAA GGATGCACTA CAATGTGGGA
26451 CAGGTAATTT TGCAAAACAC ATTTAATGTC ATTTTCAATC ATTTAGAAAA
26501 GAACTTCTCT TCCCCGAATA AATCAGCGGG GCGGGGCTCG TCGAAGCTGC
26551 ACTTTCTGAT TCAGCAGACC TGAGATTCTT CATTTGTAAT AAATTCTCGG
26601 GTGATGCTGA TGCTGCTGAT CTGGAACATG ATCTTCGCAG TAATATTATA
26651 CACTTCCCCA CATTGAAATT GGGAAGAAGA AACATTGGAG AGCAGATCAA
26701 GACATAAGGG GATCAGGGGA CAGCATAATT TTGCTTTAAT TCTACAGCAT
26751 GTTTTCACCA AGGGTGGAAG GAGAATGAGT TGAAGTATAG ATTTTACAGA
26801 CGTCACATCG TATTGCTAAA AACAGACGGA AAAGTTATTG TAATAACCAG
26851 TAAAATTGTG GAACGAGAAC CAATGAGACC ACTGATGTAG CAATTATTTT
26901 ACTCTAGCAA GAGGGATTGT GAGGCAGGAA GGAGGGAAAA GAAGAAGTTA
26951 TTTATGTAAT TTTGGGGTTT CTGCTGAGGA AACCTGAGTC AACTCACTTC
27001 AGATGCATTT AGCATGTTTA CACAAAAAGG ATTTTATTTT GGCAGCTCCA
27051 GGAACTACTG GATGAAGCAA AGAAAGCTAG AATTGGGATA AACCACATTG
27101 ACTAATTACT TCTCTTCGTT ACTATTAGGC ATAAGACATA TCTTTTGTTG
27151 ATTTTTGTTA TAAAAATTAG ATAAACTTGA ATATCAATAC ATTGGCTTCA
27201 TTCATCCAAG TGCTATCTCA TGGCTAAAAT AGCTATTTAA TGAATATGAT
27251 TTAGAGGATA GCATAATCCT CCTAATGAGA CAAAAACAAC TATAATGTTG
27301 AGTTCATCAA CTGACTCTTA AAATGGTTCA CAGGCGTGAA CCACCGTGCC
27351 TGGCCTAAAA ATATAAGGTT TTATTCAGAT GTTTCTACTT TTACATTTTG
27401 ATACTCTGAA GTTTCCAATT TGGAATTTCA ATAGTTTTTA GAGATTTAAA
27451 GAGATCAATT TTGACACTGT AAAATATTTG TTTTGCTTTA AAAGTCAATT
27501 CAAATTATGG CCACTTTGGT CTCATATATT GTGAGGCTGG TTTGTTATTA
27551 GGGGCATAAA GACTTAGGAT TGTTATGTTT TGTTGATTAA CTGAACCTTT
27601 TACTATTGTG ACATGACATT TGTTATTTAT TGATAGAATT TTTTTTTTTT
27651 TTGCTATGAA ATCTACTTTG TTATTAATAC AGCCACTCCT GCTCTGCTGC
27701 CTTTGGCAAC TGTTAGCATG TATCTTTTTC CATCCTTACA ACCAATTTGT
27751 GTCTTTACAT TTAAAGTGCA TTTCTTATAG GCAGCCTGCA GTTGGGTCTT
27801 GCTTTCTTGC TGAGCCTGAC AGTCTGTATT ATAGTTGAGG TTCTTAGACA
27851 AGTGTCGTTT ATAATGTGAC TATTGATACA GTTATGTTTG TCTAGTAGCT
 
27901 GTTTGATTGC TATTAGTCCC AACTGTTCTT TGTTCCCCTT CTTTTCCTGC
27951 TTTCTTTTAG ATTAGTCAAC TGTTTTTTAC GATTCAATTT TATATATATT
28001 TTTGGGTTTA TTAGCGATAA CTGTTTTGCT ATCTTAGTGT TTAGGATTTT
28051 TAGTATATAA TTTTAACTTA TCACAGTTCA CCTTCAGTAA TATTATACAT
28101 CATAGATGGT ATAAAAATTA CAATGATACA TTTTCATTTT TTTCTGTCCC
28151 AGACACTTCC TACCTGAATC TGTGAGATTA TGGTTTGTAT TAAGCATTAG
28201 AATATTTTGG GCCATTTTTT CTTTAATTTT TTTTCTGTCT CTTCTTTTTT
28251 ATTTGGGGAG TTATCTGTGA GCTGCTTGAA AGTTTCTTGC AGTTTATTGT
28301 GTCAAATTTA GGAATTTTTC CTTTGTTATC TCTAATTTGT GTTTATTTCC
28351 ATCCACTATA GTTTTTACTT CTGACAATGT AATTTGCATC TCTGCAAGTA
28401 TGGTTTGTTT TTTTTTTTTT AAAAAAAAAA ACCTTCCCTG CTTCCACTTA
28451 TTGTCTTGAG TTTTATTTGG AGTACAGAGA TTTACTTAAA ATCTTTCCAT
28501 GCTTGCTTTT AGATTTGGGG TAATTCCTCA CTCCTGAGAC AAGACCTTTC
28551 TGAATATTCA TTGTCCTGTG AAGTATGTGG TATTTTGGCC TGGCTCATGG
28601 GAATGGACGC TCTTCCAGTC ATTGTGTGAA CCCCAGACAT AGTTTCTGCT
28651 AATTTTCTTT AACTTTTTTT AAATTTTATT TTCCATTGTT CTTAGGAAGT
28701 TTCCTAGCAC ACAAGCACTT TTCATTACTC CACTAAATAC ACAAGAGGGA
28751 ATCTCTGCAC ACCTCCAGGA TTTGTTTCCT GTACTGCTTT TTTCTCTCCA
28801 GTTTTCTGTC CTGTGATCTC TATCTGCTTT GGTTTTCCTA GCCTCTCAGC
28851 TTCATCACCC CAGCTCTATG TCAGATTTTC TCCCTTTTTC ATGGCCTTGA
28901 ACCTCTGTTA AAACTCTGTC AAAAGAGCTG GGACATTTGT AATGCCTGCT
28951 ATATTTGTTT TCTGTCTTTC AGTGATCACC TACCATCTTC ATTGCCTGAA
29001 GTCCACTGTG TTGAAAATCA TTGTTTAATG TATTTTGTCT GTTTTGTTTT
29051 TTATTCATTT AGATAAGATG AAAAATCAGT ATCTGTTACT CCATCAGAGC
29101 TAGTAACAGA TTGCAGCAGA GCTTCAGCAC AGCACATGCT GCAAACCGGC
29151 CAGAGTGATT TTCTTTCCTC TTTGCTTAAC TGCTTCTTTC TCATCCTTCA
29201 TTTCTCAGTG TAGCATCTCT TCCTCACGGA AATCTTCCCT GGATGAAGTA
29251 TAGATCAAAT TATCTTATGC AATCATATAA CTATGGTTTG TGTTTTTTTT
29301 ACAGGACCCA TCTGAGTTTA TAACTGTCAT CTTTTTATCT TGGTTCTTCG
29351 CTATACTTTA AGCTAAAATA ATAGCAGAAG TGTTGTCTTA TCTTCAGAGC
29401 TTATTATAAT ATCTTCCACC TTGTAGGCAC TCAATATGTA CATACTTGTT
29451 CAGTGTCTGA ATGAATACAT GTTAAAAATG CACGGTCCTT TATATCCAGA
29501 AATCTACTCA TGTATTTTAT CTCTACTTTG CTTTTTCCCA GTTACAGATT
29551 GTGTTCACCT ATTGAAAATT AAAAATACAT TTTGTTTGTG TAAAAGATGA
29601 ATAGAACTTA AAGAAAATCT CTGTGAGCAA CTTGGAGTAA AAATTCAAAA
29651 AAATGAAAAA TAAGGCTAGT GTACTACAAA AGAGACTATC TGAAAAAGAA
29701 GAAATAAAAT CACAGTTAGA GCATGAAACA ATTGAATTGG AAAAAGAACT
29751 CTGTAGTTTG AGGTATGACC TAGTTTTAAA TAAATGTTTG AACTATTTGT
29801 TTTATATTAA AGACATATAA GGAGAGGTTT TGTAATTATG AACTTTCCTT
29851 CTAGGATTTA ACAAGGAAGA AAGCTTCTTA ATCTTATAGA GTGTGAAATT
29901 ATTGGATATA ATATCACTAG TTCTTAATTG TGAATACTTC TCTAATAATT
29951 AAATGCATAT TCTTTTAAAT CATAGTTTTA ATGGCTGTAT AGAATTTTAT
30001 GCTTTGGAAA TCTCATTATT TAATTGATGA ATTGAATTTT AGGTTTTAAA
30051 AAGTTTTTGT AATAATGCTA CAAGGAACAT CTTTTATATA CACATAGTTT
 
30101 TCTACATTTC TAATTATTTA CCTTGGATAA ATTCTTCTGG TTAAAGTATA
30151 GACACTTTTT TAGATCTGTT TTACATCTTT GATCAATATT TCTAAATTGT
30201 TCTTAAGAAT GTTTATATTA ATTTATAATT CAACCAACAG AGTATAAAAC
30251 AGATATTTTT CTTTACACAG AATAATTATT TTAAAAACTA TCAGCCAGGT
30301 GCAGTGGCTC ATGCCTGTAA TCCCAGCACT TTTGTAGGCC GAGGTGGGCA
30351 GATCACGAGG TCAGGAGTTT GAGACCAGTC TGCCCAACAT AGTGAAATGC
30401 TATCTCTACT AAAAATACAA AAAATTAGCC AGGTGTTGTA GTTGAACCTG
30451 TAATCCCAGC TACTCAGGAG GCTGAGGCAG GAGAATCACA TAAACCTGGG
30501 AGGCAGAGGT TGCAGTGAGC CAAAATTGCA CCATTGCACT GCAGCCCAGT
30551 GATAGTATGA GACTCCCTCT CAAAAAAAAA AAAAAAAGGA ATCCAGTTTT
30601 ATTGTTAATA TGCAGGGAAT AAAAAGTAAA ATAGAAAGTG ATTACTGATT
30651 AGCTTATTCA ACATCTCTGT CTCTGCTATG TAGATAAAAT TAATTCAGTT
30701 TTATGCTGAA GACATGTATT ATTCTTTATT GTTTAATTAA ATATTAGATC
30751 TTGTGTTGTT CCAAAACATA TTTTAAAATT GGTTATAAAA TACATACTAA
30801 TCAATAGGAT AGACTGGAAG TGTTACCCAA AAAACAATCT TAAAAAGTGT
30851 AGGGTAACAT ATTACATTCT TAACCTAGTC TTGGATTACA ATAATCTGTA
30901 GATATGTTTC ATAAAGACAT CAAAAATGCT ATCTTACAAT ATAAATTATG
30951 TTTAGGGATA CTTGCAATGA ATGTTTCATG AAGATGAAAA TGTATTTCTA
31001 GTGAATGTAT CAACTTGTTT ATAAAAAGTA ACTTTATGTT AATTAACTAA
31051 ATGATTCATC CTAACTGAGG AGTAATTACT GTGTGAAGAA AAGATAATTT
31101 TTATCTTGTA ACTTTACTGA ATAATTTTCA ACATCCTTTT TCATACTATT
31151 TGCTGGAGTT ACTAGTAACA GAAACTTATG CAGGTTGTTC TTTTATCAAT
31201 ACATTTCAAC TTACACATGT CCTTTGGATG AGATTGAGGT GAGAAACTAA
31251 AAACATGAGG ACTAGAAAGA AAAATAATAT TTAAGAACAT AGAAATTTCT
31301 TTAGGATAAT AAACCAACAT ACGAATGTTT TCTAATATCA ACAAAGAGAG
31351 TCAAACTCTG TAAGATATTT GAAGAGATTT ATTCTGAGCC AAATATGAGT
31401 GACTGTGGCC CCTGACACAG CCCTCAGGAG GTCCTGAGAA CATGTTCCCA
31451 AGGTGGTCGG GGCGCAGCTT GGTTTTATTC ATTTTAGGGA GGCATGAGAC
31501 ATCAATCAAA TACATTTAAG AAATACATTG GTTTGGTTCA GAAAGGCAGG
31551 CCAACTCAAA GCTGGGGCTT CCAGGCTACA GGTAAATTTA AACATTTTAT
31601 CGTTGACAAT TGGTTGAGTT TATTTGAAGA GCTGGGATTA ATGGAAAGAA
31651 ATGTTCAGAT TAAGATAAAT GATTGTGGAG ACCAAGTTTT ATTGTGCAGA
31701 GAAATCTCTC AGCAGACTTC AGAGAGAGCA GTTGTAAAAT GTTTCTTATC
31751 GGACCCAAAA GGGTGCCTGG CTGTTAGCTG ATTATCCCCT GGATCTGCAT
31801 AGAAAGGAAG GAAAACAAAG GGGAAAGGGG GTACTCTATG GAATGTGGAT
31851 TTTTCCCACA AGAGACTTTG CAGGGCAATT TCAAGGCATG GCAAGGAAAT
31901 ATATTTTGGA TTAAATATTT TCTTCCTTGT CTCATAATGT TATGCCAGAG
31951 TCAGATTGAA AAGCAAGTCA CAATATACAG GGTCAAATAA AACCCATCTG
32001 ATGAGAATCC ATGGTTTGTA GAGCATGACT CCCTGGACCC CTTAGGTAGG
32051 AATTTGGGCA AGATAAAAAA TTAGAGCTTA GTTCTCACTG ATAAGATAAA
32101 TTCTAAGATA AGTATATTTA CAGATTTGCC ATGCAGCAAG GAAAAAAAGA
32151 AAAGAAGAAA TGTTGAAGAG TTGCACCAAA AAGTTAGGGA AAAGTTAAGA
32201 ATAACAAAAG AGCAATGGAA GATAGAAGCT GATACGACAA AACAAATTAA
32251 ACCGGCTCTT GAATCAGTGG AGGTGGAATT AAAGACAGGA GGAAATAATT
 
32301 CAAATCAGGT AAATTAATGT TTGGTAAAAC TTCATATTTC TACTCATTAA
32351 TATTACTTAC AATATCTCTT TCATTTAATG TATATTATTT AGACCTAAAC
32401 AGTCCCCAAA TTTTATTTCA TCTTAAAAAT GAATCATGGT GGGCCGGGCG
32451 CGGTGGCTCA CGCCTGTAAT CCCAGCACTT TGGGAGGCCG AGGCAGGCGG
32501 ATCACAAGGT CAGGAGATCG AGACCATCCT GGCTAACACG GTGAAACCCT
32551 GTCTCTACTA AAAATGCAAA AAAAAGTAGC CAGGCATGGT GGTGGGTGCC
32601 CGTAGTCTCA AGCTACTAGG GAGGCTGAGG CAGGAGAATG GCGTGAACCC
32651 AGGAGGTGGA GGCTTGCAGT GAGCCGAGAT CCCGCCACTG CACTCCAGCC
32701 TGGGCGACAG AGTGAGACTC TGTCTCAAAA AAATAAAAAA TAAAAAATAA
32751 AAAATAAAAA AAAGAATCAT GGCATTTATA GCTACAATTA TTTATAATAA
32801 ATCTTGAAAT ATTTTATTTT AGTTCAAAGG CCTTATGGAA AGCAATGCTA
32851 TTCTATAATA TATACTTAAT GATATTGTAA GTATTTTGTT CCTTGTGACA
32901 TAGTTCAGCA TATTTTCCCC TATTTCATGT TAATTACGTT TCAAATGTTA
32951 TGGAAAAGAA ATAAAAGTTA TCCCAATAGT AAATAATCTC ATGATTCTCT
33001 AAAAAGAGCT TTATAAATTT AATTTTCTTG CCTTTTGGTG TCTTGAAATA
33051 GAAGATTATT TTTGTATGTA TGTATCTACC TCACAGAAGT TATTGATTTG
33101 GTGGAAGAGC ACTAGGAGTA GAGTCAGAAA AGCTGGGAAA AATTCCTGCC
33151 AGCTTCCCTT AAATTTTTAA CCTTTTGCTA TAGAATTATA ACTAAATGAG
33201 TTCATTGATT TGTGTGTGTA AAAGTGCTTA GTACAATGCC TAGACTTATC
33251 ATTTATCAAT AAATATCATT CTTAAAACTG ACAATAAAAA TATTAGAAAA
33301 GTAGAATATC TAGACAATAT TTTAGAAAAA GGGAGCTTAA AGAATTTGGA
33351 AAATGTCATT CATCTGTCCA AATGTCTGCC AAGCTACGGC TCTCACTACA
33401 GGGAGAGGTA TAGTTTAGAT GTTAGAGTGT AAACCCAATT TTTTAATGTG
33451 GTCATAGTTA TTAATTCTTT ATGCCTTGCA AATTGTTGTA ATTCAGTAAA
33501 AGCCTTTGTT CATCCTGAAA ATTTAAAAAA ATTATCTAGT GGTTTTTTTT
33551 TTGCTTTCAT GGATTCACTG TCTTCAAATA AACTTTTGAA CTTTGGGGAA
33601 TTTATGCCAT ATGAAGTTTG AGGTTTTGAC TCAACTTCTT TTTTCCCATT
33651 TAGATATACA GTTATGGCAA CCTCTCATTG TATAAATGTA CGGGTTATTA
33701 TTTAATTTCA GAAACAATCA CAATATGTTA TCCTATTGGA TACTAGTTAC
33751 AAGTTTTCTT TGTTTTATTT AGATTTCTGA AACTGATGAA AAAGAAGACC
33801 TGCTGCATGA AAACCGCTTG ATGCAAGATG AAATTGCCTG GCTCAGGCTG
33851 GAAAAAGACA CAATAAGAAA CCAAAACCTG GAAAATAAAT ACTTAAAAGA
33901 CTTTGAAATT GTGAAAAGAA AGCGTAAAGA CCTTCAAAAG GCTCTAAAAC
33951 GGAATGGGGA AACAATTAGC AACAATGATA GCCTGTTATA GTGGACAGCT
34001 TGCGTGCTCT GACAGATGAA AACACAACGC TGCATTCCAG ACTGGAGAAG
34051 CAAAGAGAGA GCAGGCAAAG ACTGGAAACA GAAATGCAAT CATGTGGTTG
34101 TAGACTGAAT GCTGCCTCTA TGTGATCATG ATCAAAGTCA TTCATCACGA
34151 AAGAGATCAA GAGCTTGCTT TCCAGGGCAC AGTAGATAAA TGGTGTCCAT
34201 TAACAGGAAA ATTTGAATTT TTGATCCTGA TTCTTTTCTC TGCAACTTTT
34251 CCAAAGCTGA GAGTAAGTCC AAGAGTCCTC GAAACTTGAG CTCCATTACA
34301 CAAGAGAGGC TCTGAAAGAA AAGGCTTTGG TTTTTGAACA CGTGCAAAGT
34351 GAGCTAAAGC AAAAACAGAG TCGAGTGAAG GACACTGAAA AAATGTACAA
34401 AAGTGGATAC AGTACAATGG AAAAATGCAT AGAAAAGCAG GAAAGATTTT
34451 GTCAACTAAA AAAACAAAAT ATGTTGCTTC AACAGCAATT GGATGATGCT
 
34501 CGCAACAAAG CTGACAATCA AGAAAAAGCA ATACTTAATA TTCAAGCCAG
34551 ATGTGATGCT AGAGTAGAAA GCCTTCAAGC TGAGTGCAGA AAGCACCGTC
34601 TTTTACTAGA AGACAGTAAA AGGTTGGTCA ATGGATTGAA TCATTTGAAA
34651 GAAAAAGAAC GTCAATATGA AAAAGAGAAA GCAGAAAGAG AAGTAAGTAT
34701 CAAGAAAAAT AAGTATTTTT CAAACTTCCT GAAGCACAAT TTAAAGTAAT
34751 ATTTGGTTAC AGCTGAATGG TGGATCTAGT TGAATATAAA AAAGGATACA
34801 TATGATAAAT ATATCTGCTT AGAAACATTC CTTGTTTCCA ACAAGTCAAA
34851 GTTAGATCTG AGAGATGTTT TCCTCTGATT AAAGTCAATG TGTCACTTAT
34901 AAAATTTTAA GTTATAAAAT GTCAATATAG ACTAATAGTA ATAATATAGG
34951 TCATACTACT GAAATAATAA TTTTAATGTA TTTATGTTGC AACATTTTAA
35001 GACCATGATA AATCAGATAT ATGGAAATGC TCATACCTAA AATGGTATTT
35051 TGAAGTTGAT TCAATAAAGT GCGGTCCTTT GACAGTTAAT TTCAGATTTC
35101 CTAGATGAAC TGAAGTGTAT TCCCTATTTC ATAATTACTT TTCTTCAGTA
35151 GCTTTAAATA TGTCTTAGTT GGTAAAATTT TGTTTTTCTT CATGTCAGTT
35201 TGACTTAAAT CTCTAACTAT TTCAATCTCA AGTTATGTAT AGAAATGATC
35251 ATTCTATTCT TCAAAGGCAT CTAATTTTAC TTATAATATG GGGAAAATGC
35301 AGTAAATTTT AGCCCAAATA ATATTTGATT TAATCTTCCC ACTGGCATTT
35351 ATAAATTACT TTCATTTTTA AATAAAAAAT TTGCTCATAA TTTTTATTTC
35401 AAGGATCAAT TACTATCATT TGGATATAAC TTTGTTCAGG ACAAAAAGAG
35451 GCATAGCTAT CTGTGATTTA TTAGTTTGAC ACTGGATCCC CATTTTCAGA
35501 CTAAGGAGGA TTTCAGACTA ACGAGGAGTG GCAGGACTCA GAGTAGGAAT
35551 GGAGTGAGTA GGGAAGAGAG ATATAGCAGC TGAGTCAGGT GTGGGAGGTG
35601 GAGGGCAGGT TAATTAGAGC ATCTAAGGCC ACTGTAATTT TACTTTTCTT
35651 CTGAGATAGA CATCTATTGG AAGGATTTAA GCAGATGATT TAATGTGAGG
35701 AACTCTGAGG TTGATTTGAG TTTCTAATAA AAAGAAAGAG GGAAATCATT
35751 CCACAATGTA TAATTTACTA CCATCGGTCT CACCCACATA CTCATTTCTT
35801 TTTGAGACTT CAGGTTTTTA AGCATTGCAG ATTCATCAGG GGAGGAATGA
35851 CTAGTGGGCT GAATATGTTG TGTGAATAAC AATACCAGTT TGGCAGGAAG
35901 ATAACACCTT CTGTATCCTT ANCTGGATTC AGTAATACAC AGGAATGTGT
35951 ACACATGAGG AAAAGAAGGT GAATCGATCT GTGTGGTGAT ATTTTTCAAA
36001 GTGTATGCTT TAGAGTTAAA TATTATTAAT GGTTTAATAA TAAGGTGATT
36051 TGTAAAATCA GTAACAAAAA TAACATCAAG TAGCTGTGAG ACAGCTTCAA
36101 TGAAAACGAG ATGATGTCTT AAACAAACAA TCAGCAACAA AAGCTTTGCT
36151 GGATGCTTCA TCGCGTCATT GCATCTATTT AGAAAATGAG ATGCAGGATT
36201 CAAGGAAGAA ATTAGACCAG ATGAGAAGTC AAGTATGTAT GAAACTTTGC
36251 ATGCCAACAA CTGTTAATCT GTAGCTAGTT AACTAATATA AAGTGTTTTG
36301 GGGTACTAAT TTTGGTGGAT GGCTTTCTTT TGTATTTTTA TTATAATTAA
36351 TTTTATTAAA ATTTTATAGT GGATGGCTTT CTTTTGTATT TTCCTTATTA
36401 TTATTTTTAT TAAAGTTTTA TTATAATGAA CCTATATCTT AATCTCTTTT
36451 ATTCTGCCAT TTTTTATACA TATTTTTTTC TTAAATATTT AACCTTAGGA
36501 AAGTTGAGAA TTATGCATCA TTTCTCACAG AAGTTGAGAG AGTTTTTTTT
36551 TTTTTTTTTT TACCTGTTAA ACAGTCTATT TTTAATGATT TCTCTGTTGG
36601 CATGGTGAGG CAAGCCAGAT TAATTCAGAG GACAATGTCT AATGGAATGT
36651 TTCAGAAAAT TATCTTATTT TTAGTCTCTA CTTTTCTGAA CGTATAAAGA
 
36701 ACTTGTGTGT ACTTATTTCA TAGATTTCAG GTTAACTTGT TCAGAAAGGC
36751 CATTTTACTG AATAAATTTT TATTATGAAG AAAATCCTTA CTCTCTTTCT
36801 ATTGGGCTCA GAGAACACAT TTTGTCTCTA TATGAATAGG ACAGTTAGCA
36851 TTTGCCAACA TGTATCTATT TTCTCTTATT TATAGAAAAA GCTAAACTAA
36901 AAAGGGGGTT ATAGAAGGTC AGCAAAGGAT GAGTTGAGAT GTTCAGGGTT
36951 GGTTAAGTGG GCATTTAGAC AACAAGGTTT CTCCTTTGGC ATGTTTAATG
37001 GACATCTTTG CAGTTTAAGA TGACGCTTTT AAATTACTTC TCTCCTAATG
37051 ATGACTTGAG TCCTGCTATT CAATGGGAGA GTCAATAAGA TCCTGTAGGA
37101 TCTTATTTGG AACTGACTTT GTCGATTTTA ATTTTGTTCC TGCTTGTTTT
37151 TAAATTTTCT TGTTGTTTCC CTAGAAAGGA AAGATGTCGC TTAGTTTTAA
37201 ATATTTAAAA ATGTGCAAGT TGCTTTGCTA TAATAAAACT AAATGCATAC
37251 ATACAAAAAA TAAAATTATA GTTGATGTGG TAGTGTTTGG AATTCAAAAT
37301 ATAAATGCTT AGCGTGAGGT AATCCTTTAT CTTTCCACAT TTTACCAGTT
37351 TGCAAGTTTG AGTATTTAAC TGATAAAATG TAATTCAAAA GCAAGAAGAA
37401 TGTGTTTTAG TCCTAGAGCA GGGGTCTGTG AACTTTTCTG TAAAGGGCCA
37451 GATATTATTT AAGGCTTTGT GAGCCATAAT ATCACTGTTG CAATAACTCA
37501 GCCCTGCAGT TATAGCACAA AAGTAGCCAT GAACAATATG CAGATTGAAG
37551 GGGTGTGGCA GTGTCCCACT AAAATTAATT ACAAAAAACA GGTAATGGGA
37601 TGATTTATCC TAGATTCTGA CCTTTTTTAA ATAAAAAAGA TTGTGATAGT
37651 CTAAAATATT TATATATATT TTGTTGATTC ATTCATCTAC TGATGGACAT
37701 TTAGGTCATT TCCAAATGTA ATTTTTTTTT AATTCTNTGT TTTAGTTTCA
37751 AGAAATACAG GATCAACTTA CAGCTACTAT AAGAAGTACT AAGGAGGTGG
37801 AAGGTGACGC ACAAAAGTAA AATTTGAAGC AGCACACAAA ATAACTTGAG
37851 TATTTATAAA GCAAAAGAGC ACTGTAGTAT GAAAATTGTA TCAGTTATGA
37901 TAATAAGTAT GTCTTTGTGA AGCCAAAAAA GTTTCATCTG TAAGCTATAT
37951 TGAAATACAT CATTTTTCTA CATTATTCCT AAATTTTGCA TATTATCAAA
38001 ACACAGGTAG AAAAATGACA GTAGCCCAGT CATCTACTTT TGCAATTGCT
38051 AAGAATTTGT GTAATTATAC CTTCAGAAGT TTGTTTAGAA TTTACATGAT
38101 TTAAAAACAA TTATGTGTGA GAGTAATTAT TTTAAAATGC ACATTTTAGG
38151 CTTGAAGTAG AAAATGCCAT GATGAGAAAA ATGATTGyAA AAAACAGGAT
38201 GACCAAATTG AGTGGCTTGA GAAAATCCTG CAGCGTTCAA GTTTGGTAAG
38251 CTGATCTCTT AATTTCTGTC ATACTGAAAA GGAATTTTAT TTTTCCAGTA
38301 GGATGGGTTA AATATCCCTT GTCCAAAATG CTTGGGACCA AAAGTAGATT
38351 TTTTCTCAGA TTTTGGAATA TTTGTATATA GCTAATGAAA TAGCTTGGGG
38401 ATGGTACCTG AGTCTAAACA TGAAATTCAT TTATGTTTCA TATATACCTT
38451 ATGCACATAG CCTGAAGGTA ATTCTCTACA ATGTTTTACA GTAATTTTTT
38501 GCAGGTAACA AAGTTTTTAC TGTTTTCACC AGAGCCTGTC ACATGAGGTC
38551 AGGTGTGGAA CATTGCAGTT GTGGTGTCAT GTCCGTGCTC AAAAAGTTTC
38601 AGATTGTAGA GCATTTTGGA TTTCAGATTT TTAGATTAGG GATGATCAAT
38651 CTACAGTACA GATGCTCCTT GACTTACAGT GGGTTTACAT GATAATGTTT
38701 CTTGTTTGAC TGAAACATTA TAAGTAATAT TTGATTTATT TCAGATGCTG
38751 CAGGTGTTCA AGAGCTAGAT GAAGGTATGC TGCCAAATTT ATGAATTAAT
38801 TCAATCATTG ATTTCTTGAA ATAAACTCTA ATTAATGAAC GGTATTCCTC
38851 TCAATTGCTT GGTTAATACA TGCTACATTA AATATTTTTT CTTATACACA
 
38901 TCTAGTGAAA GATGTGAAAA CATAAACATT CATAGTGAAG GGTATACTTA
38951 TGCTTTGTTA CTTCGTCATG TTCCATGGCT TTAAAAAAAT CACAAGAAGT
39001 CTGTGTATCT CTCTTTTTCT GGCCCTACAC TTTTCTTCTG CCACCCCAAT
39051 AGAACTGTCA GCCTGCACAC TGAAACTGTT CTCAGAAAAC AAAGGCATCA
39101 TCAACTTCTC AAGGTTAAGG TAGTGATTTA AGCTAACAGA CCCCACACTC
39151 ATGTGATAAT AATTGGTTAA GCAATTACAG GTCACAAGCA GTCACTTGAC
39201 CAGTGACATT TTAAATCTCT AGTCATTGAC TTTGTCATTG GTTTACTTTT
39251 GCACCTGGGA AAAGTTGAAA ATTCCTTAGC ATGGAATCAA AACTCTCACA
39301 TCAGTGTGGT TCTTGTCAGG TTATTCAGCC TTATCTTTCG CCACTTACCA
39351 TACTCTGCAA CTTTGTTCTA GCATCCAGAC AAACTAGACT ACACGGAGCT
39401 CCACAGTGAT CATCCTCACC TCCAGCTCTT TGCATTGACT TCTTCCCTCT
39451 ATCCTACATG TGTTTTCCTT CCCCTTCAGA TATCAACCTA TGAATTGCCT
39501 CTACCAAAAA GCCTACAATA TTGACACAAA CCTGGGCTAG TATCCCTTCT
39551 ATGTCTTCCA GTAAGTGGCG TCTTATGCCT GTCATTGTAC GTATGACTCT
39601 GCATGGGAAT TGCCTGTTTG TTTTTTCAGA TTATAACATA CAGTTGTTGA
39651 GGGGTGGACC GTATCATCTT TATCGTGTTA TTCCAGTGCT TGTCCTAGTA
39701 CCTTAGCACA TGGTTGCTGA ATACATGAAT GAAGAGTGAG AAACCAGAAG
39751 CTCTGATACT TAACTGCCAT GATAATGAAT TCAGTGTGCA ACTATGGGCG
39801 AATTATATTT AATAGTAATT GCATATTGTA CATATTTTTC ATTCTAATTA
39851 ACACTGATAA ACTTTTCAAC TTATACTGAC TTTCTCTTAG TTAACTGTGA
39901 AATCATTTAG ATAAAGAATA TAATCCTTTT TCACTCTAAC TTCTGAATTA
39951 TAATCTGAAT CCTCTATAGC AGGGGTCCCC AACCCACAGG CCACAGTCAG
40001 GTCCATGGAG TATACAGTTT AAGACCTCTG CTGAACAGCT ATACCTCCAT
40051 GCTTGTTTCC AGTGGCCATG ACACTTTATT TAAATATGTA AGTTTTATTA
40101 AGACTGAGCT CTTAAAAAGA AAAAACCAAG AACCTTAGAT ACAACTAGTG
40151 AAGAATTGAG ACCTGTCCAT ATTTAAAACC AAGCACACGA TACCACTTAA
40201 AAGGTTCCCC AGAAAGCCTC TATCCTGAAA TGCTTGAAAG TGAGGCAGTG
40251 CTGACTCTCG ATTATTACCA ATTGCATTAA ATATGACACT TGTTTTGTTT
40301 CTTTT~GGCT ATAAGGAGAA AATGTCATTT TGTATATGAG TGAGCACAGA
40351 GGAGAGAGAC TGGGGGAAAG AACAGAATGA GCTAATAATT TTTTACTAAA
40401 TACTCCAGTT CTCAGCTTTT TAAATCAACA GACAAAATGA ATCAGCTAAA
40451 CCTAAAATCT TTGTGAATAG TATAAATTGT CTTTTAAATT AAATGCATAT
40501 ATTTTTATGT TTTACTTTTT CAAGACAAAA GCAGGATATT AGTACAATAT
40551 AAGATTTATA GAGGAGCAAA TTTCTTGAGA TAGGAAACCC TTAAAAGCAG
40601 TATTTAAAGT GCTTAAATAC TGTCACATAT GTTTAATAAT CATAATACTT
40651 AATTGTGAGA ACTGGGAGCT CATGTTACTA CTAAAACCAA ATAAAAATTC
40701 AATACATATT TGTTAACTCA ATTTAAGGAT GTTTACCTTA ATACTGACAA
40751 AGTACGGATG GTAACACTGC CACACTGAGA ACAGGCAAAG TAATCTTCCT
40801 TCTGCTCCTT GGCCAAAACT GCCACAAACT ACACACATAT CCTGAAGTTA
40851 AGAAAGCAGA ACATATTTTA AATGGAGACT AAGCTAAAAA CCTACAAATT
40901 TACTTTAAAA ATACCTTCTT AACTAATATA GCTCTATAGC TAAATATTGG
40951 ATCACTTCTG TGTAGATGAG ATAAAGCAGA AATGTGCAAG GAGGAATTCA
41001 ATGAGGAAGA CAGTAAATTG TCAAGTTCAA ACCTGATTCA AAGTGAACTT
41051 GTCACTGCTA GAAAACAACA CAACCGTAGT GTGCATAGAG TTTTCTTCAT
 
41101 CATCCTTATT TGATGAAATA TCTGCAGTAG ACACCTATAA AAAGCAAAAT
41151 ACACAAAATA CGAAGTTATA TTTTTCACTT GTTTTACACT TAACTGAAAA
41201 GCTTCAGAAA ATTCATAATC AAAACATATA TTTTTGCTAA GGTCTAGAAT
41251 AACGATTCCA AATATTAATG CTAAGATACT ACAGTAAAAT GGAGTCATGA
41301 CATTTTATTA TTCAACTCAT TCTCTCTTTA GAGGTAGAAT TCTAATCAAG
41351 AAATTATAAA TGATAATATT ATAGGTATGT ACACACACAA ATCTATCACT
41401 ATTTTAATGA CACACACTTG GGATCTGCAA TGTAGTTAGT CTGAACTGAG
41451 ATGTCCTGCA AACATAAAAT ACAGCACATA ATTACATATT ACATGTTGAA
41501 ATGGTAATAT TTCAGATATA TCGGTCGAAA TGGAAGGCAT TAAAATTAAT
41551 TTCGTCTGTT CACTATAACC TTTATTTTGT TTCGAGAGGA GTTTCACTCT
41601 TGTTGACCAG GCCTGTTCTC CCTGCCTCAG CCTCCCAAGT AGCTGGGATT
41651 ACAGTTGTCC ACCACCATGC CCAGCTAATT TCTGTATTTT TAGTAGAGAC
41701 GGGGTTTCAC CATATTGGTC AGGCTGGTCT CTTAACTCCT GACCTCAAAT
41751 GATCCACTGC ACCCAGCTCA TTGTAACTTA TTAATGTGGC TACTAGGAAG
41801 TTTTAAATTG CATATGTGGT TCTCATTATA TTTCTGTTAG CACCGCTTTA
41851 GAATATTATT TTGAATAACA TCCAAATTTC AGTATTAGCC AAATGATTAT
41901 CAACCAATAT TGTTCAGTCT GGACTTAGTT CTATTTGACT AAATCAACTA
41951 AGTACCCACT GGTTTGTTAA TAATTTCTAG AGTGATTATG AAACAAAATA
42001 AAGCTCTGAA CTAGAAGTTG TAGAAGAAGA CAAGGAGGGC ACTGCCAAAA
42051 TCATAAAATA CAGTCCTCTT TCTTTAAAAA GCTTACAACC GAGGCCTGGA
42101 AAGACAGAGT TGAAACACAA CAGGTTATGT TCAAGGTCAA AAACATAACA
42151 CACCTGAATT ATTTTTCTTG AGGAACAATG GAAAGAAGAT TAACCAGCTG
42201 GGTGTAGTGG CTCATGCCTA TAATCTTAGC ACTTTGGGAG GCTAAAGTGG
42251 GTGGATTGCT TGAGCTCAGG AGTTTGAGAC CAGCCTGGGC AACTTGGTGA
42301 AATCCTGTCT CTACCAAAAA TACAAAAAAC AGCCGAGCAT GGTGGAACAC
42351 GCCTGTAGTC TCAGCTATTC AGGAGGCTGA GGCAGGAGAA TCACTTGAAC
42401 CCAGGAGGCA GAGGTTACAA GTGAGCCAAG ATTGTGCCAT TGCACTCCAG
42451 CCTGGGTGAC AGAGCAAGAC TCCATCTCAA AAAACAAAAA CAAAAACAAA
42501 ACAAAAAAAA AACCCAGCAA CCTTCAAAAT CAGAAACAAA TCAAACTCGA
42551 TGTCATCCTT TTTCTCTCCA CTGAGAAATG ACCACTGGTT ATCAAAAAGC
42601 ACTCCAATAT TTAAAGTGAT CTGACTCCCA GTCACAGCGT GACTGAATTC
42651 TTAGAAACAT ACAGCAGGTG TTAAGTATTT CTCTTGGACT GATTTATGAG
42701 AGAGGAAAGC CCTGAACATA TAGAAAATAC ATTTAAAAGC AGATTTTTTG
42751 TTTTTTTTTG GTTTGTTTGT TTTTGAGACA GAGTCTCGCT ATGTTGCCCA
42801 GGCTGGAGTG CAGTGGCGCA ATCTTGGCTC ACTGCAAGCT CTGTCTCCCA
42851 GGTTCATGGC ATTCTCCTGC CTCAGCCTCC TGAGTAGCTG GGACTACAGG
42901 CACCTGCCAC CATGCCAAGC TAATTTTTTG TATTTTTAGT AGAGACGGGG
42951 TTTCACCATG TTAGCCAGGA TGCCAGGATG GTCTCGATCT CCTGACCTCA
43001 TGATCCGCCC ACCTCGGCCT CCCAAAGTGC TGGGATTACA GGCATGAGCC
43051 ACCATGCCCA GCCTAAAAGC AGTTTTAATG GATAGTACTA ATGCTTTATA
43101 AGAGCAATTT ATAGTCATAT GAACCCTAAT GACTACAAGT GTTAATAATG
43151 CCAATATTCA TCATTAGGGA GTAAATAAAG CCATGACAAA TCCAAACATT
43201 AGAAAAGTAT GCAACATTTT AAAAGTAGGG AGGTAGAAAC TTGTATAGAC
43251 TGCCATGAAA GAAATTATCA AAGACACTGT TGAGTGAAAA AATAAATTGC
 
43301 AGAACAGTAT TTGAGGTATA GCACTATAAT ATAAAAACAT GCAAAGCCAT
43351 TACATGTAGT CTATGGGCAC ATATAATAGG CTGACTCATA AGAAATTGCT
43401 GCTTTTCATC AGTTCAGAAA TAATATTGGC AATTTCATAT GGATCAACCT
43451 AATATATAAA TATACCAAAC TGGTAACAGG GAAATAAGGA GGACTGAGGA
43501 GTTAGTAATG GTAAATTCTG ATATACCTAT AACGCTTTAA TTTTTTAATA
43551 GAGAAAATGT ATTGATGTGT TATATGCATA GCATTAACAA AATTAGCTTT
43601 CTAAGATTTT AGAGAATCAT CCAAGATGAT TCACAGAAGT AGAATCATCA
43651 TCATCAGTAA GAAATTAAGT GACTACTAAA AGTAATCATT AATTCAGTCA
43701 TAGGACTAAT GATGCATTGA CAAGGCTATT GAGATATATA ATTATGGAAA
43751 TGGCTAAAAT AGAGATAAAG GTATCTATTT CTACCTCCCA ACCACTAACA
43801 GAAAATTCAA CACATTATAC ACACTGAGCA GCTCAAAGAA ATTGTAAAGA
43851 TCCATTATTA TTTTTAAAAG GAAATTTAAA CAGTGAATGC TTTCACTGAA
43901 AATGATAAAC AATATATTCC CAGGATAAAC TGGAAATAAA GTCTGCAGTA
43951 GAAAACTACA AAGTCCCTAG ATTCAAGTGG GGGTGGGGAG TCATATTTGA
44001 ATAATAAGTG CAGAAAAACC AAAATATTTT AAAATAATTG TCCATGGAAG
44051 AAAGAAAACA GTATCATCTA GCTTGAAGAC CCATTGTTCT TATTTTATAA
44101 TTTATTTCAG ACCTTTAGAC TGTTAGAATA ATAAAACCTA ACTTGAGGGC
44151 AAAGGTAATC TTTGAGAAAA TATGTGCTAT TTTTGCCTGC ATAGATAATA
44201 GTGTGATTTA TCCAGAAGGT GATAGAAATA TCATTTTCCT AGACCACAGA
44251 TATAAGCCAA GGAGAACAGA AAGCTCTGAC CTAAACTTCA CAAGTGTCCC
44301 TTCCAAGCAG GGACATGTAA GAGTAAACAA AAAAAGAAGA TCAAATTAAA
44351 CTCAAAGTGG GAAAATAGGA AAAAATAAAG ATGAGAATAT AAATCAATAA
44401 AACAGAAAGG GGAAAGAAAA TAGAGAAAAG TCCATGAAAA CAAAGGCTGA
44451 CTCAAGAAGA TCAATAAGAT TGATAAATCT CTAGCCAGAC TGATCAGGAA
44501 AAAAATTAGA CACACATTAT TAGTATCAAG AATGAGGAAG GTGAAATCAC
44551 TACAGATTCT ACAGGTATTA AAATTATTAG AAACATTATG ATCAACTCCA
44601 TTCCTTTAAT TTGTGAAGAT AGACAAAATG AACAAATTTC TTGAAAGATG
44651 CAAATTTATG CAAGGAGAGA CAGATAACCT AAATAGGTAC CTATTAAAGA
44701 AATAAAATTT GTTGTTAAAA ACTGTCCCAC AGGCTGGGCA CTGGTGGCTC
44751 ATCCACTAAT CCCAGCACTT TGGAGACGGA TCACCTGAGG TCAGGAGTTC
44801 GAGACCAGCC TGGCTAACAT GGTGAAACCC CATCTCTACC CAAAACACAA
44851 AAATTAGCTA GGCATGTTGG TGCATGCCAG TAATCCCAGC TACTCAGAAG
44901 GCCTGAGGCA GGAGAATTGC TTGAACCTGG GAGGTGGAGG CTGTAGTGAG
44951 CAGAGATCAC GCCACTACAC TCCAGACTGG GCATGGTGGC TCACGCCTGT
45001 AATCCCAACA CTTTAGGAGG CCAAGGCAGG TGGATCACCT GAGGTCAGGA
45051 GTTCGAGACC AGCCTGGCCA ACATGGTGAA AAACTGTCTC TACTAAAAAG
45101 ACAAAAAAAG AAATTAGCCA GGCATGGTGG CAGGCACCTG TAATCCCAGC
45151 TACCCAGGGG GCTGAGGCAG GGGAATCACT TGAGCCTGGG AGGCAGTGGT
45201 TGCATGAGCT GAGATTGTGC CATTGCACTT GCAGCCTAGG CAACAAGAGC
45251 AAAACTGCAT CTCAAAAGAA AGAAAAAAAA AAGAAAACAC AACAAAAACC
45301 CCCCACAAAG AAAATTTCAG GCCAAGATGG TTTCACTAAT AATTTCATGT
45351 ATAATATAAG AAGATACATT TCCACTACTA CACAACTTTT CCAGAAAACT
45401 GAAGAGGAGA ATATACTTTC TGATTCATTC TATGAAGCTG GAGTTATGCT
45451 GATACCAAAA CCAGATGAAG ACATTACAAG AATGTAAGAC TACAGGCTGG
 
45501 GGAATGGTGA CTCATGCCTG TAATCCCAGC ATTTTGGGAA GCCAAGGCCG
45551 GGAAAATTGC TTGAGCTCAG AAGTTCGAGA CCAGCCTGGA CAACATAGTG
45601 AGATGCTGTC TCTATTAAAA AATTTAAAAA AGTAGTTGGG TGTGGTGACA
45651 CACACCTATG GTCCCAGCTA CTTGGGAGAC AGAGGTGGGA GGTCGAAGCT
45701 GGAGTTAGCT ATGATCGCAC CACTGCACTC CAGCCAGGAA TTATAATGAG
45751 AACCTGTATC AGAAAAAAAA GTGCACAGGT TTACAACCGT GGTGCATCCA
45801 GTTTTATTAA TAGCAAGAAC AGGGAGTACG CTGTTAAACA CAACAGCATA
45851 GATGAATCTC CAAATAATTT TGCTGAAATA AATCAGTCCA AAAAGCATAC
45901 AGTTCTGTAT GATTCCACTT ATATACAACT CTAGAAGATG CAAACTAATC
45951 TTGGGGACAA GGACGGATGG CAGGAGGAAT GCAGAAAATT ACAGAGGGAC
46001 ATGAAGAAAC ATTGGGAGAT GAATATATTC ACTATCATGA TTGTAGTATC
46051 GTTTTCAAGG GTGTATATGT ATATATCAAA GCTTATGGAA TTGTACATGT
46101 CAAATATAGC TTATATCAAC TATACCTCAA TAAGCCTGGT TTTAAAATTT
46151 TTCTTTTTGA AAAAAGGACG AGAATCTAAG CTTCCTTATT CCTGGTTTAG
46201 TAGTAAACTT TGAACAATTT CACCTGTCTC CTATACTTAA AATGACATTT
46251 CAGAATTTTA AAAACAGGAT TTTAATAAAA TAGCGAAGTT ATTACATAAA
46301 ATATTTGCTA GTAGTTAGCA ACTGTATTTG TAATACACAT ATAAATAAAG
46351 CCTCATAACA TGATAGTAAG CAAATATCAA TCTTAAAATT TTTTAAATAA
46401 AGGAGCAACT ATATTACATA CTGACTTTTT AGAAAGGGTT GGCATAGAAA
46451 GATAAGGAGT CAAAGAGGAA GGTAAGAAAA GAGGAAGGTA AGAAAAGAGG
46501 AAGGATGAGA AAGTAAATAT ACAAGAAAAT GTAACCAGTG GCTCAAAAAA
46551 AAAGCGAAGT AGGACAGTAA AATAAACATT TTGACCTATT TATATGACTC
46601 TTAAGTTCAA AATAACTTGC TATGAGATTT TCATCATTAA CTGACATTTA
46651 GATTAGATAA AATATACATG AAGCAAGCCT CACCCCAGGC AATACAACAG
46701 CTCCGATTCC ACTTTTCAGC TTTGACTTGC CTCGGCCACC TCGCCCTGAC
46751 AGTCCTGCAC CTCGAGGTCT CTGCTTTCCT GGAAATCCAG ACCCACGGCC
46801 CTATGTAACA GATTAGGAAA AGTCAACATT CTGTGTCAGC CCAAAATGAT
46851 TTTTAAATCC AAATGCCACT GAGATAAAAC ATTTTATTAA ATGTTATACA
46901 AACACTTCTT TAGATAAGTA TTAAGAAACC TGGCTTATTA TTTTTATCTT
46951 TAAAAGTATA CTGCACAACT TAAAAATCTA AATATAAAAT GCTTACAAAC
47001 TTAGAATCAT ACCTTAGGCC TGTCACTGTG AACGCTATCA GCAAGCCTTT
47051 GCATGATTTT TCTCTTTCCC ACTCCTACAT TCTCGGTGAC GACAACAACT
47101 ATAGCCTGAT CCAGATATTT TGAAGTGCAA CAAATTGTAT TCAATATAGA
47151 GTAAGGATAA GGAAGAACTC TCTCATTAAC TGGTCTCACG GTGATTACAG
47201 TAATAGCTAA CATCTATTGA GTGCTTACTA TGTACTAATC TAAGTATTTT
47251 TTACTCTCAA CAATCTCATA TAGTAGGTTT TATTATCCTT ATTTGAGATG
47301 AGCGTGCTGA GGAATAAAAT GGTTAAGTAA CTTGTCCAAG GTCGCTTAGC
47351 TAGCAAGTCT GGCTCCAGCG TCCCTGGGTT GAAAGCATAT TTGGACTGCT
47401 ACATCAGCAG AAATTTCGTT TTTGGCTAGT GTGTAACAGA TTCTTCCCTG
47451 TCATTAAAAA TTAAGTCAGT TTCCTTCACT ATTCCAACAG TTCTCTTATG
47501 AACTCAACAT TTCTACCTCA TTCACCCATT GTATTTAGAG GAAAATTTAT
47551 TATTATTGTT ATTACTTTTA TTTTTGAGAC AAGAACTTTA TCCATCACTC
47601 AGGTTGGACT GCCAGTGGTG TGATCACAGC TCATTGCAGC CTAGAACTCT
47651 TGGGCTCAAG TGATCCTCCT GCTTCTGCCT CCCGAAGTGC CAGGATTACG
 
47701 GGAGTAAGCC AAAGCATCCA GCCAGGAAAA ATTATTTGAG GATTACAGGA
47751 AAGCTGACAA AAGGCTTTGT GAAAGCTTTG CTTTAAATAA TCTGAATAAT
47801 AAATACTTGA AATGGAAATA ATTTATCTGA CTTCTTACAC AAGAAATAAA
47851 CCTATGGGAA AATGTGTTAA ATTCCCTGAT AATTTCAGAC ATTAAGTACC
47901 AGAGTATCGT GTTCCCTGCC CCCTCACCCT TGTTTGTACT AATTAATTAC
47951 TCCTTAAAGA AACCTGGCAC CTACCTAAGT AGATGAATTA TGTATATTTA
48001 AAATTATCCA GATGCTCAGG AAAATACTTA GACGTTTCCC TCACGACAAG
48051 TTAAACAATA TGTCATATAT TCAACTGATC GTCCCTTATC ATAGTTTGAA
48101 ATGAACTTAT TCCCTATTTA TCAGAATGGA CCCAAGTCAA AAATTAATGG
48151 TGATACTGTA AGCATTAAAT AGAAACACAT GAACAAAAGG AACGGGAGGA
48201 ATGGCTTTCT CCCTTTGGAT GTAATAAATA CAGCCAGCTC CCAGTTTCAA
48251 ACTGCCACTC CTGTCTTCTC TTACCCTGCT CTCCTTGAGA TCCCTTTTGA
48301 GAAGTGCATC AGCTTCTTTG CACAACAGAT GGATGGGGTC AGGTTAGTTT
48351 TTTGGGTTTT TTGTTTGTTA GTTTTTGAGA TGGAGTCTCG CTTCTTTGCC
48401 CAGGCTGGAG TGCAATGGTG TGATCTTGGC TCACTGCAAC CTCCGTCTCC
48451 TGGGATCAAG CAATTCTCCT GCCTCAGCCT CCCTAGCAGC TGGGATTACA
48501 GGCACATGCC ACCACGCACA GCTAAATCTC TTTGTATTTT TAAGTAGAGA
48551 CGGGGTTTCA CCACGTTGTC AAGTTTGGCC TCTAACTCCT GACCTCAGTG
48601 ATCTGCCCCC TCAGCCTCCC AAAGTGCTGG GATTACAGGC GTGAGCCACT
48651 ACCCCTGGCC GAGTTTGTTT TGTTATTAAA TTGGGATAAA AGAGTTTTGA
48701 AAAATTAAAT CAGTGATTAA AAATCAAGAC TACAGGTAAT TCTCTAAATT
48751 TATTTTCTTG AACATGAAAA TGGCTTGACC CAGAAAAAAA GTAAGTTAAA
48801 AATTGGTGGT CTATATTATC AAACTGTCAA ATGAGGTATA TTTATACCTC
48851 AATATCTTGG ATGATATCAG GGGGAGGTAG GGAGGTTAAA AAAATAGTTC
48901 TTCCAGTCAT GAAAGAAAAT AATCTAGAAT TCCTTAAAAT CCTTCATTAG
48951 GCTAAATTAA ACAGCCATAT TCCAGAATAT TAAATATAGA ATATGAAGAA
49001 AAACTGTCAT CTCCAGTCAA TGAAGTATTT TAACTTTTGA GTTAATACTT
49051 TTTCAAATTA ATTTTTTTAT CTTCAAAATG CATCACACTA CTTAACTCAC
49101 TTCAAGGACT GGCAGAGCCA TCAAACAATG TCATGGGGGA AAAAGCCTTT
49151 TAAGTCATTT TAAGGTATTA ATACAGACAA TGAAATCTCC CACAATTTTG
49201 GTATTTCTTC TCCATGAGAA CATAATAAAT TAATGGAGTT TTTTCTTTTG
49251 CTTTTTTCTA AGCAACAAAG TTTTATGATA TCATGAATGA AAAGGTCCTT
49301 AATTACCTTT TGGTCTCATG TCAAGGACTT CTCCCTCATA AAACCAGTAG
49351 TAATCACAAC AAAAGGAATT AACCATAAAA AGAGGTATTA AAAATGTATA
49401 CTTGATTTTT AAATGCAAGC ATATTATTTC TTTACATTAA AATTTTTAGA
49451 TTTAAAAAGT GTTTCTGGAA GCTCAATCTA GAAAAGAAAG ATTTAATTCT
49501 TAACATCCAG TAGGGCAAAA CAAATCAGAC AGAAATGATA TATGAAATGT
49551 AAATGCAATT TTATGTACCA CTTTGATGCT CCAAATGGCA CTGCCAGGAA
49601 GTGCCTGGGT TTAAAAATTT CCCGACCTCC TGAAATGTCT GGGGACCAGG
49651 AAGGTGGGCT CACTGTATTA TGGGTACTCC AAGCCTCCTA GGATATGGCA
49701 GTTGAGAAAA TAGATGTGTA AAACTCAGCA ACATAAAAGG TCAAAGCCAG
49751 CAACTAAGGA ATTTTAGGAC AGCAAAAACA AATGCAAACG TATGGAAATT
49801 TAGGACAAAT TGCTTCAAGG AAGGCAAAAT AAGCTAATCA CTAACAGTGA
49851 TTTAAACATT TAAGTATAAC AAATGTTTGC TGCTACAGAG ACATCACTAC
 
49901 AATGAAACAT TAAAAATTAA GGGTTTATAT GACATCAACA TTGACTCATG
49951 AACTGCAATT ACTGCACCAA GAAGTAAATA AAAGTCAATC ACACTTTAAG
50001 AATTGACACT AGAAGAAAGT ATTGGGGGGT TATTTTTCTT CTAACAACTA
50051 TCGCTCTACT TAAAAGGAGA AATGGATAAC CATAAGGGAT TCTATATTCT
50101 ATAGCTATTA ACAACCAAAA CCAGTAGGCC AAAGAATGCA ATGAGAAACA
50151 TAAGCAATCG ATAAATGCAT AAACTTTAAA CTGTAGAGAG CTGGTAACAT
50201 TAAAATGCAA ATACCATTAT AATCTTAGCA TTTAATCACT CTTTCTTCAG
50251 TGACCACTAG TTGTCGGTTT GGTTTCGGTT TTTACTTAGG GAAATGAATA
50301 CTTTATGGAA ATTACATCCA ATGGACAAAA GTGAAGAAAC GTTAAAGCAA
50351 ATTGTCCTAA ATTTGCAAAT TAAAATGCCT TAAGTGCCTG ATAAATTATA
50401 TAGAAAGTAG TATCTTATTA AAATCTATAT AACTAAAACT AAAGCGTTTT
50451 ACTTCCAAAC AACCACATTC AGCAATACCC TGAACTAATC TGAAGATGCT
50501 AAACAGCATA AAGAAAAATG TTTACTCCAC AAAAATAAAA TTTTAAAGAA
50551 AAACAAGACA AATGTCAAAC AATAAAAGAA TATATTTTGA CTTAGGTAAT
50601 TCAATGGTGC ATGCATAATT TTACCAATCA AGTAACCAAA ACTTAAGACA
50651 AGGTACGTAG TACTTACCAG GTTTCTAGAA TATCATCAAA TTAACAAGTA
50701 CTATCTCATT AATCACATGA AAATACCACC AGGAATTAAA ATAACCAGAA
50751 ATAAGAATGT GACTGACTAC TCTTGGGGTA AGAGATAGGA AATGGAATAA
50801 CAGTATTTTG AGAAAAGCCA CACAAGCAAT AGACTGGTTT CATTTTTAAG
50851 TCACAAACTC AACCCGCACA CATTGAAGTC CAGCAATCCA ACTCATTCTC
50901 TCCAGCAAAC ACTATTTTTT TTCCCTTCCA GGATTACCCT CAGGTGTTCT
50951 TTCTTACCCA TCAAATCTCT AACCTAGCTC AGGTAATCAC TATGCTGATT
51001 TCACTGAGCA GCAACCACTG CAGGTCAATT ACCTCACTTT CATAATTTCA
51051 AATCAACTTG AATCTGGCCT ATGTCTTCTT CCCTACTATT AGTAGGGAAC
51101 TGTTGTCAAA AAGCCATTTC TCTTTTATCA TTTTACTCAA GCTCCTGACA
51151 TCTGGCATAC TGTTCTGCTG GATTTGGCAG CAGCCCACCT GTGGATATTT
51201 CACTGACTTC CTCGCTAATC TTTCTTCATC TCCTGTACGT GTTCTTCCTC
51251 TATTTGACTT CCAAAGCCCT GGAGTTTCCC AGGGCTTGAG CCTAGATCCT
51301 AAATGGTATT ATCTACAATG AGGGCATCTT GGAAGTTGAT GCTTCCCACA
51351 TTCTTATTTC TGGTTTTAAT CAAAGATCTC CAAAGTAGCA GGATTTTCAA
51401 CTTCCTTCTT AGCATTTCCA TTTTCTCACA CAAAACCCCT CTTAAGTTTG
51451 CCAAGTTTAA AATTTCTCCC TTAACCGGTT TCATCCCAGT CTTCCCCATC
51501 CTTATAAATG GCATCCAGTC CCTTGTCACT CTCCTCTGCT CCTTCAGTCC
51551 TCTCCCCCAA CACTTTGTCA ATGCAAATCA TCAGCAAATT CAAGACTTTT
51601 TATCTTCAAG TTTTGTCTCC AATCCTTCTA CTTCTTGTCA CTTCCACCAC
51651 TAGCAGCACA CTCGTGGCCA CCACCATCTC TCACCTAACC TGCTACAGAG
51701 CCTACTGCTG GTTATGCTTC TCCCACCCTG CATATACTCA AACCTTTCCC
51751 CACAGCAGCC AGAGGAACTT TTCAAAAGTA CAAATGTGAT CAAGCAAGTC
51801 ACTATTCTAT TTTAAACCTT TCAATTGCTT CTCAGAGCAC TTAAAATACA
51851 AACCTTTCCC AGTGCCTTTC TCCAACTCAT TTTGTGGCAC TTGTCTCCCC
51901 TAGTGCTATG CTCCAGCTAT TCTGGCTTCC TTGCTGTCCT TCAAACATGC
51951 CAAGCTCTGT ATCAATCAGA GCCCAGCTGA CTCAGAACAC TTCACTATAC
52001 CATTCTGGCC CTTCAATAAA TGTCTGGCAT TTTCTTTCTT TCTTTTTTTT
52051 TTTGAGATGG AGTCTCACTC TGTCACCCAG GCTAGAGTGT GGTGGTGTGA
 
52101 TCTCGGCTCA CTGCAACCTC TACCTCCCAG GTTCAAGCAG TTCTCCTGCC
52151 TCAAGCCTCC TGAGTAGCTG GGATTACAGG CATGCACCAC CAGGCCCGGC
52201 TAATTTTTGT ATTTTAGGAG CAATAACATG TTGGTCAGGC TGGTCTCAAA
52251 CACCTGACCT TGTGATCCAA CCAGGCTTGG CATCCCAAAG TGCTGGGATT
52301 ACAGATGTGA TCCACCACGG CCAGCCATGT CTGGCGTTTT CTAACCCTTC
52351 AGGTATGGCC TTACATATTT TCTCATCAGG AGACCTTTCC TTAGTGCTCC
52401 CAGTGAACCA TCATCTCTCT TACCACACCA TTAGTGCCTC TCATTGCATC
52451 ACCACCTAGG ACGTTTTATT GTTTGTAATT TTATTTAGTG GTAGGCTGTG
52501 GGGCAAGAGA TACCTAAATT ACTTCTCTCA ATATTTCCAT TGGTTTTTCT
52551 CTGAGATTTG GCTAACATAC ATCTTAAATT CTTTTTAGTC AATAGTTTAA
52601 TATTATGTTG GCAGTAGACA GTTCTAAAAA ATATGAGACA GAAAGTCATA
52651 CTCTAAGATA AAAATAATGT TGAATAATTT TATTTCAGAA ATAAGAATTT
52701 TCTGGTAATT ATGTATTATT CATCATAATT TAAAAGTTAG CTTTGATGCC
52751 AAAATTTTAT CTCACTTTGA AAAGACAGAC TTTGTGTAAT TTTATAGTTC
52801 TGAAGAAAAA TTATCATTAT ACTTAGATAT TCTGACAAAT TATCTAGTAT
52851 ATTCCTTAGA CCTTAAAACT AAAATATAGC TATAATTATC AAAGTTTAAA
52901 TTATCCTTCA GATCTTAGCT TTGATCAAGA ATTTACACAA ACCATTCAGA
52951 AAAGGTTACC TGCCCCTGTA ATGTATGGGC TCTTCTCCTA CAATCCAGTT
53001 GCAGCACTTT TTACACTGAA TTAAAGATCA TGCCCCTTTC TGTCCACTGC
53051 TACGTTAAGC ACACTGTTCA GCACTTACTG AACTTTTTTT CTGCTGAGCA
53101 TCTATTATCT TTTTTTGCAT GTGTGTATGA ATTCATCAAT AAACAATGAG
53151 CTATGATATG AAACACACTA ATGCCTCCAC CAGTAATAAC CTAGAATCTT
53201 GAATTTCTTC ACTTAAAAAA AAGTTACTAA AAATTACTAA AAAAATCTAT
53251 ATAATGCCTC CTTAATCTAC AAACTGAATT AAAAGCAAAA TAGTTCCTAA
53301 TGTAATCATT ATTAAGTTGT AAATAGAATC AACTTGCTAT CAAATACTAC
53351 AGAAACTAAA AAAACACTAC CTGGCAGGGA CTGAATTTGA ACCCAAGAAA
53401 AATAATTTAA CCCAAAAATA GTTTAATATT AGCATTAAGT TTCTTTCTGC
53451 TTTGAGGCCA ATTTCTCAGG TAAACTGTCC AAGCTAGTAA ATCATACAAG
53501 GACTGTGGGA TGGGGAAATG AGAACAATAA TGACAAGTGG AGGATAGGCT
53551 ATTTAGAAGC AACTTTCTAT TAAAAGTCAA ATTCTATTGC TGATTGTTAA
53601 ATACGATCAA AGTCATTCTT ACAGCCCAAG AGTACTATCA GTTTTAAAAG
53651 CAGCCATACA AGGTTTGGTG TCTCAAAGGC AAACTTTACA CCTCCAAATC
53701 TTAAATCAAC CCCGGCCCCA CACTCTTCCA ACATACTATG TAAAACCAAG
53751 GTTGCTTATA AACAGTAAGT CTAATTTAGG AAAAGTGAAA GCAATGAAGA
53801 CAGAGGGCAG AAAGGAAGAT AACTGGTAAA AGAAACAAAA GAGTTTACAG
53851 TTTGTCTTTT TGGTAAATAC AATCACTTGT ATACACTGGA GCTACAGCAG
53901 CAAACACTTC ACTCTTTAGG GAATTCTTAA AAGAAGTCTC ATATATAAAA
53951 TTGAGTTCAA TATCAAGTAG AACAAGAAAT TAGACCCAAT CCTGTTTTGA
54001 TAACTACAGG AACAGCTATT ATCAGCTTTA GCCTTCAATA ATTTCACAAT
54051 ACAAATTAGG TAGTGCTAGC AACAGTAATG TCTCCAAAAA TACCACAACA
54101 AGCACAAGTG AACTAAAAGC AAAGTCTGGT ACTTCTAATC TGCTGTGTTG
54151 TTAGCTTGGT GTTCCAAAGG GGTAGGTATT TTTTAGGTTT AACACTATGG
54201 TTCTCTGGCT ACTTTTAAAA CTGAAAGTAC TCTTCTTATA ATATAAAGTA
54251 AACTTCTAAT AATTTGTCCA AGAGATATCA AAATACAGTA CCTATGTTTC
 
54301 CATTTATGAA ATTTTCTTGA AATATTTACT GCAGGCTGGG CATGGTGGCT
54351 CACCCCTGTA ATCCCAGCAC TTAGGGAGGA CAAGGCGGGT GGATCACCTG
54401 AGGTCAGGAT TTCGAGACCA GCCTAACCAA TATGGTGAAA CACCATCTCT
54451 ACTAAAAATA TAAAAAAAAT TAGCCAGGTG TGTTGACGTG CACCTGTAGT
54501 CCCCGCTACT CAGGAAGCTG AGACAGGAGA ACTGCTTGAG CCAGGCAGGC
54551 AGAGGCTGCA GTGAGCGAGG TCGTGCCACA ACTCCAGCCT GGGTGACAGA
54601 GCAAGACTCA ATCTCAAAGA AATAAATAAA AATAAAATAA ATATCTACTG
54651 CAAATAATAC GTTTCATAAA ATGCTTAATA AAAACTTTCA ATACCACCAT
54701 GAAAGCAATT AGAACATGAG GGTGCCTGAA GTAGATCATA GTAGTGTGAT
54751 TACATTCAAT ATCTTTCTAT AAGACGTGAT GATACATACA GGGTAAATCA
54801 TACAAGAAGA CCTAAGATTA TTATCTCAGA ATTTCCTAAT TAGAATGGGA
54851 ATCAATTGTG CAATGAGAGA AATGGACTGA AGACAGAAAT AAAAACAAAT
54901 TTTATGATTT AATATGACAA ATTTAAAATT TCCACTTTAT TTTCCACCAT
54951 TTTTAAAGGG CATTCCTTAT AGTATTAATT TTAATGCATT TTTAAAAATC
55001 AGTAAGACAA GTAGCACATT TCTCTAGTTC AGGACTCCAC TTTCAGGGTT
55051 ACAGTTAAAG ATGATCCTAG AAGTGCATTT TAATTGGTCT AGGGGAGGGT
55101 CATTAGCTTG ATGTTCTAAA ATCTACTGAG TTGATTACAA TATGTGTCAG
55151 TGTCAATAAC CACTTT.CTT AAACCCAAGT CGCAAAGCAC ATATACCTAT
55201 AGAAATGTCG AAAATGGGCC AGGTGCTGTG GGGCTCACAC CTGTAATCCC
55251 AGCACTCTGG GAGACCCGAG ATGGGAGGAT CACTTGAGGC CAGGACTTTG
55301 TAAGGAGCCT GGTCTACACA GCAAGACCCC CATCTCTATA AAAAAATAAA
55351 AAATATTTTT AAATGCTGAA AAATGAAATA CTAAATTATT TATGAATATA
55401 TAACTTCAAT AAAAGATGAG AAAATACAAC TAGGTCATTA GGACAATTTG
55451 TATGAAACAA TATCCAATTT GCTGTCTGTT ACTTCTACCT TATCATGTCT
55501 AATTTTTCCT CATCTTTGTG AAATCCTGCA TCTACAATAG GCAAAAAAAC
55551 AAAAAACAAA AGGAATCTTA TTCTATTCAC AGCCATCAGA AAAATGTGTT
55601 ACTATTCCTG TAGTCAGTTA GGTTCTAAAT CATACATTTG AGATATATCT
55651 CAAACATGTC ATAAAGAAAA TAAGCATCAT ATTTCTAACT AAATAGCACC
55701 TGAAATATTC CATATGAAAG GAAAAATACG AAAAACTAAA CTTTTAATTA
55751 CTAACCTCCT TAAAGGAAAA TAATTCTATA TGATTTGATA TTTCATAATG
55801 GATGTCCAAT ACCTGAGATA TAAATTCCAT TTTCTGTGTC CTAACATCTC
55851 ACCTTCAGAA AATCTCCAAT ATTGGGTTAT TTGGATTATA TATTTAAAAC
55901 TGAATATACT TGAAGATGTG AATGATTTGT TGCCACAACA AATGGTTTAC
55951 AATTATCTGC ATTAAAATGT GTTCCATGAA CAACAGAAAA AGAATATTTC
56001 TACTTCAGTC CTTAAAAATT GTGAAAGCAC AGCCACAGTC TTAGTTTGTC
56051 GATCTTCAAT TCTTTTGGTA TTTTAATTTC ACCAATAATT CTGGAACTGC
56101 TGGTTTTGTA TTTCTCAAAA TGTTTGTCTT GCTCATGCAT CTTGCTTACA
56151 TCACAATCAC AGCTAGGAAA AGCAGCTTAT TTTACCGGCA GTCCCCGTGT
56201 GAAGAATATG TAGAAGCACC AGTAAATAAA AAAGGAAGTT TATCCAGAAT
56251 GCCTAATTCA AAGAAATTCA TGTAACATGA ATACTGAACT TCAGTATTTC
56301 TAATATACGT ATATACACAT TCCTGCTAAC TGTAGAATAT TCTCCTGCAA
56351 GTTTATGGTC CCTGTGCCTC ACCAATGGAA GATCTATCTT GTACTATTCA
56401 ATTCATACTC TCCCTAATAA CAGCACTATA TTAGCAGGTA TCATCATCAA
56451 GTGAAGGCTG CTTGCTAGAT AACTAAAACC AAGATTGGAA AACTAAAAAG
 
56501 CTGGAGGGAG GGAAAACACA GCCTACAAAA TGCTTACACG ATTAGCAGCA
56551 AATGATGAAG TAGTTACAAG AGGGGCCGAA GAATAGAAAG ATTATCATCG
56601 GATTTGGGAA TTCAAAGGCA GCTCAGCAAA ATACTAGGAC ATGGCTCATA
56651 TAAGATGGAA ATATAAGATG GAATAAGCCT GGAAATACAC CCTCCTCCCC
56701 AATATTTCAG AACATAAAGT CTACAGAAAG AGAGTCCTAA TGTATTGAAC
56751 CTGGTTTCTC AATTGAAACA AAAGTCTTCA AGGAAGGAGA GATACGTAAA
56801 ATTTAACAGG AATACATATG AAGCAGTATC TTTAAAGAAT GTAAATATAT
56851 CAACCAAAAG TAAACTTGAG ATTCAAATTT CCATTGCTAA AGTCTTTTCA
56901 GAGTCAGAAC GACATAATTA TTATATAACT ATTCAAAACA AAACAAAAAA
56951 ACTTACTGGA AAAAAGAACA ACTGGTTTGA GAAGAAAATC TTTTCATGGT
57001 AGAGCAGAAA TAACTGTCAA GGAAAATACT TAGAAAAAGT CATATATAAA
57051 GAGTGGCCTA GTTTTACTGC ACACGTCTTT GCCATGCAGA AACAGCTAAA
57101 TCCCATCTGA CAACTACTTT CCCTTTCGTG TACCTTGTCT TTAGAGAATA
57151 AAATATATCT GGGTTTGATA TAGAGAGAGA CTAATTTTCA CATTAAAAAT
57201 GATTTTCTTA AAAGCCTGGC CTTACAGTTT AAAAATTATA CGATTAACAA
57251 TCAAGATATT ATTCCTATAA AAAGCCAAAC TTTAATCCAT TTAAATCATG
57301 AAACTTAAAA CTTCACTTGA AGCAATTTCA ATCATGAAAC TTAAAACTCC
57351 AAATTACTAT TGTGAATTAA CACTTCTCCA CTATGTATCT TGCAATTTTA
57401 AAAAAACACT TCAGAAAGTA GCATATTTGT AAAACAATAA ATAATTCATA
57451 CCTATATTAT CCTCATTTTG CCATGCAGTT TTACTTAAAT CTCACTTAGA
57501 AATGGAACTC ATTCTTAAAG GGTAGAATGC ACATGATGTA AAGCAGACAC
57551 AGGAAGATTT ATCTTTACTT ACTGAAAGTC AATAGTACAA TTCTAAGATT
57601 TTTTCCATGA AACTACTGAA ATAAGTCCTA CTTGAAAAAA GAAATCCCAC
57651 AAGAGTTAGG AATTAATAAA AACACCAAAA TGAGAAACCC CCCAGCATGG
57701 AAAATTCCTG TCATAAATGT TGATGTTTAT CCAGTTAAAA ATATATACAA
57751 TGAATAATAT CAATAAATAT ATTAGAAGGA AATAATAGAT GTCAGTTACT
57801 GTACCCAAAA CTATACTGTA TTTTAGGCTC TATATTTACT GCAGCACAGA
57851 ACTAAAAGAA TTTTTAAAGC TCTTGCTATT AGAAACTATT ATCCATAGGG
57901 TGCATCTTTA TGCACCAGCT GAGTTGAAAT TCCAAATAAG ATTCCCTTCC
57951 AAATTTAATA AATGGCATTC TTCATTAACA GCATTTAAAA AATAATGGCT
58001 GAAACATATA ATCAACATTA CACTGAAGTT CTATCTGAAG ACAGAAAAAG
58051 TTGCCATCCA CACCAAAGCT ACACATATAC CTTCTGATTT TATAGAATTG
58101 TTCCAATATC TTTTCTGTCA ATATCATTAA AATCAACCTA CCTGTTTGGA
58151 CCGAGGTCTA CCAGGAGAAA ATTTTCTCTT AGTAATAGCT GGTTTACCCA
58201 TGCCAGTTTC TTGGAGTGAC TGAGATGTAA GTTGTTGGCA TGATGTTTCC
58251 AGCAGACGAA CTAAGAGCTG AAGGGTAATT ATGCAGCATG TCATGCGAAG
58301 GCAAGTCTGA AGAAGGTGTT GGGGAGGAAG ACACATCTGC CTTGCTTATG
58351 TCTGCTGATA ATGAAAATGA TGACTCTGTC TCAGATGATA ACTTTATAGA
58401 TTTGCCTCCT TGGTATGAAA CATCTTTCAC ACAACCCTCA ATTGTAGGAG
58451 TCATTTCAGA GTCCATCAAT CCAGTAGAAA GTTCAGAATC TTATTTCTGT
58501 TCCTTTTCTC CTTGTAGCCT TTCTATAACC AACTGTTCCT CAGGACATAA
58551 TGAAGTGCTT TCCTCATGTG GGGACACTAA GGTTTCTAGT GGGAAGAGTG
58601 ACAGATTCAA TGACAAATTT TGGAGGCCTT GATTCTTCTT TTGGATCCAC
58651 TGTTTTAGGT TCCTCTAACA ACTGCAGTTG CTCTGCTGCA CAGTGATCTG
 
58701 GTGTGTAACG ACTTCAATGT TTTCCGGCAC TCCCATTTTA TCTTCAATTT
58751 GATCTCCCCC ACAAATATGT TTCACTTCAG AAGACATTTT CATTTCTTCA
58801 CTATCAGCCT CATTAGAATT CTGTTTTTCC AATTCGGGTA TTCACTGTAT
58851 GTTGGGATGA TATTGCCAAA ATTCAAGAAC ATTTTGTTAT GGTAATGTAC
58901 AACCAAATTT TTAAATTTTC TAACTATAGA TATATAAAAC ATTTGGCTAC
58951 ACTAGAACTT AAATCAGAAG GTATTCATCA AAGCAGACAA TTATTAAGTG
59001 AATGAAAGCC AAAGTACATA AGGATTTATG GATTAGAATC ANCCNCNCAC
59051 CAGAAAACCT AATGAAAACA TGTTTTACAA CACAAGTACT TCCAAATGGA
59101 TTGAAAATTA CACAGGAAAA GCTCTCATAC TCATTTTTTA GTTAAGAAAA
59151 TGAGGATCAA TCATGTTATG TGAGGTACCT AAGTTAAAAA GTAAAGACTG
59201 ATCTAGAATG TGTAGGCATT TTCTAATAGT AAATACCCAG ATGCGACAAA
59251 GTTACTATAG ACTCAAAAGC ACAGACAGAA AAGATTTGCT AACCACCAAT
59301 ATTTATTTTG CTTAACCATA CCTGCCAACC TAGCCAAAAT ATTCTTTGGA
59351 TATAATCGTT GTTCTTCATC ATTGTCTGTA AATTAAAAGA CTAATCTCAG
59401 GAAATCTATA AACTTAGATA TCAGAAACAT AGTCACCGGT AAATTTTAAA
59451 TCAGGATACC AGTATGTTAA ACCTTTAACT CAATCTCATC AAGGTAATTT
59501 TTAAGTTATA GTAAAAAGAC ACTGCTAGCC TCATTTACCA ATCAGTGACT
59551 AAAATTTATC TATTTATCTA TGTTCTTTTT TTTTTTGAAA CAGGATCTCA
59601 CTCTGTGACC CAAGCTGGAG TGCAGTAGCA GAAACATGGC TCACTGCAGC
59651 CTCAACCTCC TGGGCTCAAG TGGTCCTCCC ACCTCAGCCT CCCAAGCTGC
59701 TGGGACTGCA GGCATGTGTC ACCATGCCCA GGCCTATACA TGTTCTGATA
59751 CCAAACTGAT ACCAAACTGA AATCTCATCT TCTTTCCCAC TTTATTGAAA
59801 GACTCCAGGT AAGGATAAAA GAAAACTCCC GAATACCAGA TCTGATAGTT
59851 AGTCCAAAAT ATTTTTAATC ATGACCTCCC CCCTCCAAAA AAAAAAGCCT
59901 TTCAAGAGGA ACCTAACCTA TCTTTAGATC CATTGTCAAT TCTTTTCCAG
59951 AATCTCTTAA CTAAAAGATT TAGTTACTGT AATGTATATA CATCCACGCA
60001 ATTGCAAAAA TGTCTTTGAG ATAAAGACAT TATCTTATAA CTCAGTCCAA
60051 TTAGACAATA TGAACATACT GCTTACCAGC AATGAGAAGA CTATCTGTGT
60101 CCTCTATACC GTCAGTGTAG GGAAAGGCTT TCTGAATATG ACTCAAAAAT
60151 CCAGAAGCAA AAGAAGATTA ATAATTCTAA CTCCACAAAA AGAAACTTTT
60201 GCATGGCAAA AAAAAATAAT AAAAACAAAG TCAAAAGACA CATAATACAG
60251 CCTGGATAAC ATGGTAAGAC CCTGTCTCTA CAAAAGTAAA AAATTAGCTG
60301 GGCTTCGTGG TACACATAAC TCCTAGCTAA CTCGGGAGGC TGAGGCAGGA
60351 AGATTGCTTG AGCCCAGGTC AAGGCTACAG TGAACTATGA CCATACCAAT
60401 GTATTATGGC TGGGGTGACA GGGTGAGACA CTGTCTCACA CAAATAAGCA
60451 AAAAAAAAAA AAAAAAAAAA AAAAAAAAAC AAGCTAGCAG AAAGTATCTG
60501 CATCATGTAA CACATATAAA GGCTTACATC CCTAACTTAT AAAGAACTCT
60551 TAAGTTAGGG AAAAATGCCA AAATCTTTAA TAACAGGCAA ATCACATGAA
60601 CAGACAATTC ATGAGAAGAA ATAAAAACTG TCCCTTTAAC ACATGAAAAA
60651 ACGATCAACT TCACTCTTAG GATAAACAAA AATTAAAACT TCTTTGATAC
60701 ACTACTTTTT ACCCAGATGA CAGACAAACT TTCAAAAGCT AAACAAGTCA
60751 TCCTGTTTGC AAAGACGTAG GAAAAGTTCT CTCACACATC TCTGGTGGCA
60801 ATGCAAAGTA CCATAAGCCC CAAAAAAGGG AATTTCACAG TATCTAACAA
60851 AGCTACATAT GTGTACACTC CTGAGGCAAT AATCCTAGGA ATTTACCCTA
 
60901 CAGATTTGTC CCTGAGAATT CAAAAACACA CATGCACAAA GCCAGGGAGT
60951 GTAAAATCAT TTATAATGCA AAAATGTCAT AACCTTACTG AAATACCCAA
61001 TTACAGGCCA CTTGGGTTGA GTAACCGATT AGCACAGATA CACAATGGAG
61051 TGCTATTGCA GCTGTTTAAA AGGAGAATGA GATCTCTTGA ACTTGCCTAA
61101 CTTGCCTGGA AGTGACTTCC AGATTTTATT ACATGAAAAA AGCATCATGC
61151 TAAAGAACAT ATACAACATG CTACCTTCAC TGAAAGAGCA ATATATCCTC
61201 TTATTCTTAG ATGAAAAATA ACAGGAGGTT TAAAACAGAT AACAATGAAG
61251 TTCGTTACTT CCAAGAGGCT GGGGGAAATG CGGTATAAGT ATTGACAAGA
61301 GACAGTGACA ATTCTGAGCA AAATTTCATA TAGTTTTTGA GTTTTGAAAG
61351 TATACTAATA CCTTCTATAT TTAAAAAATT AAATCACTAA GAAAAAGGGG
61401 AATAAAATCT ATTACTGAGA GCAAACTGAA GCAGACTTGC TTTTTAGATT
61451 GAGCAACTGA ATAAATGTGC TGCTCTACAA CCCAGAATTT TTATTGTAGA
61501 AGAACAGACA TGCAAGAATG AAAAGGCAAA GAACTCAGTG GAGAAGGATA
61551 AGAACTGGAG GTGCTGGTAT AAATTCATTA TTTCTAAGTG ATGTATATGT
61601 ATATGAACAT TTATGTGTAT ATACATAACG TGTAGGGGTG TTCAATCTTT
61651 TGGCTTTCCT GGAGCACATT GATAGAAGAA TCGTCTTGGG CCACACACAA
61701 GATACACTAA CACTANTGAC AACTGATGAG CTACACACAC ACACACACAC
61751 ACACACACAC ACACACACAC ACACACCCAA AAAAAAAACC TCATAATGTT
61801 TTAAGAAATT TTATGAATTT CTGTTGGGCC ACATTCAAAG CCATCCTGGA
61851 CCACATGTAG CCTGCGGGCC ATGGGTTGGA CAAACTTCTG TAAGTGTATA
61901 CACATGCATG CATTTCGTGA CACCACCTGC TGAAAGGGTG AAGAAGTAAA
61951 CACCCTCCAG TAGCAATGAG CACACCCGGC ACCCATTCCC CACTAAAGGA
62001 ATCTGGGTTA CTTATTTAAC AATGGACTTA TTTCAGGACT CGGGCATGGG
62051 AGGTATAAGA TGAGCTTGTA ACATCTTGTC ACACCAAAAA GTAATGAAGT
62101 TGTTCAAAAA AGCGGTGAGG GCAGAACACC AGTGCCAGCG TTGAAGGAGC
62151 AACTGACAGC AAGTATGTGA CTATTTGAGC CAAAAAATAA TTAGGTAATG
62201 AGTTGTAAAT GATTGAAGAT AATAGGAGTT CATGAGTCAA TATTAATAGT
62251 AAGCTTAACA TGTGAAAGAA GTGTTCTTGC TTACAAAAGA AAGCAGAGGG
62301 CTAACTGGTA AATGTGGTGG GAGTGCTAGA GCTGGAAATG CATCATTTGC
62351 AACCAAAAGA AAGCATGTTA AAATGGCATC ATCAGGCAGT AAGTTTGTTG
62401 TTGTTTCTGT TGTTGTTGTT GTTGTTGCTG TTTTTTGAGA TAAGAAGTCT
62451 ATCTCTGTCA CCCAGGCTGG AGTGAAGTAG CATGAGTCCA ACTCACTGCA
62501 ACCTCACCTC CTGGGTTCAA GCGATTCTCC GGTCTCAGCA TCTGAGTAGC
62551 TGGGATTACA TGGCACCCGC TACCATGCCT GACCTAATTT TTGTATTTTT
62601 TAGTACAGAC AGGGGTTCAC CATGTTGGCT AGGCTGATCT TGAACTCCTG
62651 ACCTCAGGTG ATCCACCCAC TTTAGCCTTC CAAAGTGCTG GGATTACAGG
62701 TGTGAGCCAC CACACCCGCC TAGGGAGTAA GTTTTTCAAA ATCTTTAAGG
62751 AAATTCTGAG GAAAGCAAGA TATTTGCATG GTCTTAAAGT ATCCTCACAT
62801 ACTGCTTTAT TAGATACAAA GGAGGGGGGA ATAGAAATTG GGCAACAGCC
62851 TGACCAGGTG ATTCTCATGA GGGAAAGTGG AATAATATGT GCTTCCAGTT
62901 GTGATACCCT GAGGATGGAT CAGCATTTTA CAGTATTCTG GCCAATAATG
62951 CATGCCTCTC ATCTAATTAT AAGGGGTTAT CAAAAACAAA ATGGGGAAAT
63001 TTCTGTTTGA ACAAAGAAAA AAAACAAATA TATCGAGAAC TCTATTCTTC
63051 ATAAATACCA ATGACAAAAA CTAAATCCTA GAACCTACTT GGAGGAAGGG
 
63101 GTAGATTATG AGGGACATAA GATCAAATGA CATAACTGTA ATATGGCTGG
63151 TACATTAGAT AAACATACGG CATTGATGTA AGTTTAGGAA TTTGATAGCA
63201 GGATTGTGAT TTTTAAAGAA TATTCCTACT ATTAAGAAAT ACATACTGAA
63251 ATGGTGGTAA AAAGGCACAA TGTATGTAAT TAACCCTCAA ATAGTAGGGG
63301 GGTTGGGGGA ATAGAGACAC ACAGACACAC ACACATACAC ACACACACAC
63351 ACAGAGAAAG TGAGAGAGGG CATGCCCAAT GATAATGAAA TGCTAACAAC
63401 AGATATATCT AAGTAAATGG GATATGGGTA TTATTTTTAT TTTTTCCAAC
63451 TTTTAGAGTT TGAACTTATT TTCAAGTAAA AGCTTTCTAA AAACGCATAA
63501 CATACCAAAG AACAGACATT CTTGAGATAA AAGTAGGAAA AAGAAACCTA
63551 TCTGACAATG ACTGGTTTAC AATAAAGATC CTTAAAAGAT TAACCCCACT
63601 CCAGCTCACC AGGGAGCAGC CCCAAACTAG ACTGACAGCC CCGTGGGGGC
63651 ACACTGATTT GCCCCAACAT CTCCAGCCTT CACTACAGTG GTGTTCGCAC
63701 ACAGTAAACA CTCAAATGTT AAATGCAAAC AAATGAGCAA ATTATGACTC
63751 AAGACTACAG AGAGAACTAC AGAAGAATTA AACAGCAGAG AAAGATGATC
63801 ATTCTTCAAA TGGTTCAAAG GACAAACCTT TCAATCTATG AAAAACTCTA
63851 TGGTACCTGT TTCTGTACTT ACTTTGTGTT AGGCATTGTG CTGGGCACCT
63901 TTATAGATAC CACTTCATTA AATCCCAACA TCCCTGTGAG GTAAGTACCG
63951 TTAACCTTCT ATACACTGAC TTTAGGGAAG TTAAACAGGT AGCCAAAGAT
64001 CATCAATTTA GTAACTGAAA AAACTGCAGT TTGACTCCAC AGCATAAGCT
64051 TTTAACCACC ACACTATAGT GCCTTCATAG AACAGAATAA AAAAATTAAG
64101 TATCCAATAG TATTCCCAGA AATTATGTAA TCCTGTCACT CTCAAAAGAG
64151 AATAGCAGAT TACAAAGAGC AAATCAAAAA GAAGCACACC AAAGAGGAGA
64201 CAGAACAAGC TGAGAAAGAC ACCCAGAATT CTCTACCAGT TCTCAAGACA
64251 ATGAAAACCA TAATAAAGAA GATTAAGAGC AAGAGACAAA AATTATCCAG
64301 TAGTTACTAG ATATTATTAG AGAGTGTAAA GAAGGTAATT TTTTAAGTAA
64351 ATATATTTTT TAAAAAAGGT GCTGGGGTGG GGGGTGAAGG CGGGGATGGG
64401 GATTCACACA TCTCCCAAAG CTTTCAGGAA GAAAAAAAGG ATTATGATTA
64451 AATTTTTTCA AGTTAAGATT TTTCTCGGTG ACAGCATAAA CTGAAAAAAC
64501 TGAAGAAAAT GTTTTCAGAG TATAAAAAAG AAAAATTTGT AATATGAAAC
64551 ATTATACTGT GCCAAGTTCT TATAGCCTAA AAGGAACAGT CTCTTTCCGA
64601 CATGTAGGGT TTCAAAAACT TTATAATTAT TTTTAAAGGA TCACAACCAA
64651 GACTCCGAAA GATTGAGGGT AAAGTTGGAA TTAACAGAGG AAATATATTT
64701 TTGCAAGAAA ATTGTGACAA GGTTTACAAT CTAGGTTAAT CAAATCAAAC
64751 AGAGAAAAGG GGAATAAAAA CTTTATTTTC AGTGGGGAAT GGGAGAAAAT
64801 TTATATCTTT AATATTTATA ATCATTTACA TTCACAAAAT GAAGATTCTG
64851 AATTATAAAT CATTGCAGGA AGTAAAAGAA GAGAAAAATA GAAAGAAAAG
64901 AAAGTTTTGC CTCAAAACAG CCAAGCATTT CAATTACAAA CCGTCAATCC
64951 AAACAGTTAC CTCTATTAAG CTTTTCAAAA GATACATTTT TATAACACAA
65001 AAGAATCCAA AATAAATGTG AAAAGATAAC CATTCAACTG TTAAACTAAC
65051 ACCCTACGTG GCCATAGTGT CAGGCAAAGC AAAATACCTG CCCTTCCTGA
65101 TCCCTCAACA AAAAACACAT TACATAGAAT AAAGAACAAT TTTATACTGG
65151 TAAAAAACAC AATATATTAA CACTGGCCTG ACCTACTTGC TAAATAACAG
65201 CCACAAATTA TTTGTTTAAA AATATGATAA ATTGATAGGA ACAATTAATA
65251 GTGGTCAATT GTAACTTGTT GAATCAACAA GTCAAAAAAT AAGGTAAAAG
 
65301 GACTTCATCT AACTAAATGA AATTAATAAA CACAAATGCT ACACCTTTAA
65351 AAAGTCAATA CTTCAAGTAT CAAAATATTT ATTTTAAAAA ACCTACCTTA
65401 GTTAAATTCT CTGATTAAAT GAAATGTAAG CATAAATTAA CATACTTCAG
65451 CACAACTTAA AAAAAAGTTA TTCGCTNAGG GATATACATA CATTAAAGGT
65501 TACAAATACA ATAAACTTGG CTTCAGTCAA AGCTATAGCC TAAGGTAAAC
65551 TTATAGGCTT AAATGTCCTT TTTCCTTAGG AAAGAAAATG AATTACAAAT
65601 TCAACTTCAT ATTAAAAGTA AAAAGGCAAG TAGAGGAACT GAGTGAAGAT
65651 AAGAGTGTTA ATATCCAATC AGTCAAACAG CAGAGTTTTC CTTTCCAATG
65701 ATGTCATTCA GCAAGAGAAG GAATCTGAAC AACCTGAGGA AGCAGGCCAG
65751 AGTTCATCAC AATCCTTCAG CCCTCAGGTC CCACTTCCCA GGTAGCCACA
65801 GCTGAGCACA GTTACCCAGC AGCTCTTTTT CCCCCCAGGA GAGAAAGCCC
65851 CTCCCCTCAA CCCCCACCAC TCAGGCAGAT GCCTAGTAGA ACTTGCTGAA
65901 GACCACAACA GAACCTGGTA ATACTGCTTG CAGTTCTAGG TGCCAAACCT
65951 CACTGGCGAG GACAGGGTGC AGGTACAGAA TCTCTCTGCG TCTGCTCCCA
66001 CATCCTCCCC ACGCCAGGGA CCTGGGGTTC TGGCGACATC ACCCTCCTTT
66051 TCCAGGCCCT CCACCTTCAC CCTAGAAAAA GCAGTTCCGA GAAGGGCAAT
66101 GACAATGCTG TGCCTTCCAC TCCCGCACAC GGGCCATCAC GCCCTTTTCA
66151 GCTCCAGGGA AAACCCGCTT CAGGCCCCAT GCAGCACCTG AGGGTCATCT
66201 TCATCCCTCA ACCTCGCGAC ACCAGCAGCC CAGGAAGACT AAGCTTAAAA
66251 GCTAAACCGC ACCTTGGATT CCAAGGGCTA TGTCCACTAC CCCACTGCCC
66301 CCAACCCGGC TCTGAACGCC TCACCCTGAA GGGGCAGAAG CCAAGTGAGG
66351 TAGGAAGTTA GTTAATGAGT TCATCGGCAT TTTTTATAAT AGCTCAAAAT
66401 TTTGAAGGAC CCAAATGTTC ACCAACAGGT GATTCAATAC ACAAATTGTG
66451 GTATCTCCAG CACTCGGTAA TACAAAAGAA TCACTACTGA TCTGTGCAGC
66501 AATAGGGATG CATTTCAAAA TAATTACACT GAGTTAAAGA AGCCAGACCA
66551 AAAACGAGTA TATACTATAT GAAGCTTTTA TTAAAAATTC TAGGAAGTGA
66601 ATACTAATCT ATAGTGAAAA AGCAGACTAG GAAGACAGAG GGTTTGGGTT
66651 TGAACAGCCA CAAAGCAAGC CAAGAGGCAC CAGGAAACTT TGACGGTGAT
66701 GACTATGTTC ATTATCTTGA AGAGTGTACA GTTTACTGTA TGTCAGTTAT
66751 ATGTCAATAA AGCTGTTTTT AAAAAGCTGA TGGTAATGTA TAGATTTGAA
66801 TAGATATCCG ATTTGTCTGT TCTCCATATA AAACATACAG AGGATTTAAA
66851 AAAGTAAACA ACTTACTTTG CATTTCCAGC CATTGGTTGG TACAGATTTC
66901 GTAATTCGTT GAAGACAAAA AGTATGATAC CCTTTGTCAC ACGTATCACA
66951 CACTAGCATC TTGCTATCTT CTCCCGATTG TCTAAAAAGT AAGATAGCAT
67001 TAATGATGGC TTATCTTTAA ACTTATGTTT TGCAACATAA GTAATCATGA
67051 AAATCATCAG TCCTGGGAAC CGCACAGTTC ATAAATACCT CAGTATCTGT
67101 TTTGTCTCTG CAGATAGTAC CAGAGGTACC CTCCATAAGG ATTACCTCCT
67151 AAATGGTGAT CTTTACCCCA ATGCTCACCT ATGGTTAACT TGCTCTAGCC
67201 ATTTTAGGTT TCCTGCAAAT GAGGAAGGGT TAAGGCAAGA TGAATAGCCT
67251 CTGATCTTTA CTTTTAAAAT ATAATTTTTG AGAGGAAGGC GCTGTAACTG
67301 CTATGTGCTA CTGTGTGGAA AGATCAAATC TTGGCACAGC AAATTGAAAA
67351 GACTTGTCAT ATAACGTAAA GAGGATCAAA TTTGACATCA GATCTCTATT
67401 TGTATCCAGG CTGCTGTTTT CTAGCTGTGT CACTAACAAC TTAGACTAAA
67451 GCTCTCTGAG TTTCAGGTAT AAAACGGGAA TAATAAACAT TACTGTGAGA
 
67501 ATTAAATGTA ATGCAGGTTC AAGTGCTTAG AATGTCTATT AAGCAATTAA
67551 ATGCTGAATA ATTATTTCAA AAGATGAACC TTGTCATAAT TATTAAGAAT
67601 TATAATTGAA AACCTAAAAC CTTTCTTTAA ACACTCTTCT GCCCCCAAAG
67651 ATTGGGCCCC TGTCCTACAG CATTATCTCT GTTGAGATTT TTGACTATAA
67701 TTACTTTCAG ATACTATGAT TTTAAATTCA AGAGGAGGGG AAGAGAAAAT
67751 ACAGGGAAAG CAAGAAGAGC TGAGAGAGGA ATCTCTGAAT GAAATCTGGG
67801 GGATAAACAG AAGTCAACAG GAAGATGGGT CAGTTGGCCT CCAATTATTA
67851 TTCAGTTCAA CCTCTCCATA AAAAGTCACA TGTTTTATCT CTTCAGCTAC
67901 ATGCAAAGAT CTTGGAGAAC AAGTACTCTT CCTTCAAGTT CTTTTAATCC
67951 TTCCCAAGAA ACTGAGTGCA GATACAAGGT ATCGAATAAA TGTTTACTGA
68001 ATATTTTATC TAATTTTTAC CAAAATATGA AGGTACATAG TCCTATTTAT
68051 GTCACGTAAC TCTAAGATAT TGACGCTTAT TTCTTATAAA ACTAAATAAT
68101 AAAAACAGAC TGATCATGGC AGTTGGGCAT AATTCTTGCT GGTAACAACA
68151 AAAATTCTCA GCCTGTAAGT GAAGATGCTG GTAACATCCC CAAATTATCA
68201 TGCTCATTAG AATCATGGGG GTGCAAGCGG AAATCACAGA AAAGACTGGA
68251 GAAATGACCA AGAGATGAAA TACAATAGGA AAAGATAACC CCCTTACAAA
68301 TTTGTAAATG CCACATATTT CATTTTGGAA TTATGCTGAC CAGGATCTAC
68351 TCAAGGTGGC TTTTAGAGAG ATGCAGATGT ACATTAAAAT AATAGTAGAA
68401 AGTGTCCTTC CTGGAATGGA AATTCAAACA CATGACTGAC GTTTTGACTC
68451 AACTCTAATT CTTCTACGTC TGTAGTGAGA GGTTTAAATC CTATCACATC
68501 CCCTCCCCAG TGCCCCTGAA GACAAGTTAC TTTATTCTTC TCATGCTTGT
68551 TTTAAATTGT TAATATCGCT TATTTTTTAA ACTGTTGGTA AATTTAAATT
68601 AGCTCTTTAG ACGACCAGAA CAATTTAAAT GATTAAAATC AGTTTTGTTT
68651 AGACTTCTTT CAAAATAATA TAAACGTTAA TATCCAGTAA TTTTTTTAAG
68701 TATGATGAAG TCACTCTGAA TGAATACAGC CAAAATATGA ACAACCTCTA
68751 TTATATTACA CAAACTTTTA GCGCCTAGCA ATAGGGTGCC TCTTAAACCC
68801 AATACACAGC TTTGAATTGA AATGAAAACT TACTTGCAGT TCTGGCACAC
68851 TTTGCACTCA GGACATTGCC AACCTGCACG TTTTAAAGGA GTAACCGCTA
68901 TATCCAGGCA CATTCCATGA TAGTGCTGAC CACAAGTAGT ACAAAAGAAC
68951 TGATCTAAGA GGTCTCCCGG GCTGTCGCAC ACTGCACAGT TTGCATCTTC
69001 CTTCGCTATA ATTAACAGTA AAACAATGAA ATTGTTGTAT AAGAATTTAA
69051 ATTTTTTCTG ACTACACAGT AAAATCATTT GAAAGGATTT GCATCATGAA
69101 CCTTTCAGAC ATTTGAAGTT ATTCACATTG AATTGTCATC TAATCAAAAA
69151 GAGGTATCAA TAAAAACACT GTAGGGTATT TAATCTAAAT GTAACCACAT
69201 TAAATTTAAT CGTATAGTTT TGCAATTATT TGGTGGCTAT ATCTACTTAA
69251 GCCATAAGCA GGTCTTTCTT TAATTTTCTA TTCCTAATGC CAATGATAAT
69301 GCTGGATACC TAATTAATGC TCAATATATG CTCACTGCAA AGAACCAAAC
69351 AAGGAAAATA CCAACTAAGA AATGCTTTGC AGTGTAACAT AAACATTTTC
69401 TTTGAATGCT AAATATATTT TGAAATTACA AAATCAGACA AGAGACAAAA
69451 TTAAGAAACG TTAATATCAA TTAGACTAAG ATGCTTCTTA AGCATAAACA
69501 GACTATTCAA ATCACATAAG GGCTTTTTTT TGATTACTGG AAAATGTCTT
69551 ATGTGGAAGA ACATTAAAGA CAGAAGCTAC ATATTCATAC ACAAACACAT
69601 TACTTAATAT ACTAATTCTA TATTCATCTA TATTAATTTC TAGTTGTAAA
69651 AGCATTAAAA TCCACATAAT TATGGACATC ATCCATCCCT AAATGTGGAT
 
69701 TAAAAGAAAA TGTACTAACA GTTCTTTTAC CATATAAAAC AGAAAATATA
69751 AGGATAAACT GCCCTGCCAA ATATACAGTT AAACTTATGC ATATTGCAAA
69801 ATAAGGAAAA TAATGTATTC TAAAATCATA CCAATCTTAG ATAATGTATA
69851 AAAAATATGC CTGTATATAA CAAGTATCAC AAAGTATATG AATCTTTTCA
69901 TTTTTTGAAG GTTTGATCAT TCCTATCTAC TATTCTGACT CATATGTTCT
69951 TTCTTTTCCA GTCAAGCTTA TCATACCAAC ATTTGATAAT AATATATAAA
70001 TATGCCTGTA TTTAACAAGT ATAACAAAGT ATATGAACCA TTATTTTTCC
70051 CTTTTTTTTT AGACAGTTTC ACTCTGTCAC CAAGGCTAGA GAGCAATGGC
70101 GCGATCTAGG CTTACTGTAA CCTCCGCCTC CCAGGTTCAA GGGATTCTCA
70151 CGCCTCAGCC TCCTCAGTAG CTGGGATTAC TGGAGTGTGC CACCATGCCC
70201 ACCTAATTTT TATATTTTTA GTAGAGACGA TGTTTCACCA CATAGCCCAG
70251 GCTGGTCTCA AACTCGTGAG CTCAAGCAAT CCTCCTGCCT CACCCTCCAA
70301 AAGTGCTGGG ATTACAGGTG TCACCTACCG CACTCAGCCT TTTTCCCATT
70351 TTTTGAAGGT TTGGTCAGTC CTATCAATTT CTCTCTGACT CATACGTTCT
70401 TTCTTTTCCA GTCAAGCATC TTGAAACAGA GTAAAAATAT ATTTCTTTTA
70451 TTTGTACCGG AGTCTGCAAA CTTTTCCTGA AAAGGGCCAG ATAGTAAATA
70501 TTTTAGGCTT TGTGAGCCAT AGCAGTCTCT GACAAAAAGT CAGACCTTTT
70551 GTTTTAATTT ACACTCTTTT AAAAATGTAA AAATAATTCC TCGCTTGAGG
70601 GCAGTACAAA AACAGGCTTC AAAATGTGGA CGCTGGCTGG ACCTCTGACA
70651 TACTTCAGGA AGGCTGGCTT AGCACCTAAT ACTCATCTGA AGCTGCATTA
70701 GTGAGGCTGC CAATGCCCAT AGGAAACCCG ATTTAGTCTT TGGCTTTTTT
70751 GCACTTTGTA GCATTTGCAT TGCTAGCCAC TCCCACATTA AAACGCCCCT
70801 TTCCTTAGTT TCTATCACGG CAACTCTCTC CTAATTTCTC CTTAGTTTTC
70851 CCCCACTTTC ATCTCATCCT CCTTTGTTGG CTTTTGTACC TATACAATCT
70901 TTCTAAATGC TGGAGTTTCT TAGGATTTCA TCCTTTTCCT TCTTTCCTAA
70951 TCCCCCATAG TTTCCCTTTG TGATCACATC AACTCTCACG CCATAAAATG
71001 CCACCATGAT TCCCAAATCT GTATTATTGG CCCTAGAATT CTTTCATAAA
71051 CCCCAAACCT TTATTCTTCA AGTCTTACTG GATCTCAATA GCCAAGTGTT
71101 CTAAACACTC AATCTCAACT GCTCTAAAGT GAAACTTTTT TAAAAACCCG
71151 AAACAGCATC CATTTTTCTC AAGAATAGAT AAAGGCAGAA ACCTTAGTAT
71201 CATTGTTGAC TCTGAACTCA GCTTTGACCC AACACATCCA GCCAGATCCC
71251 AATTCTTATG TATTAAACTT CCTACACATC ATTCTTTCTT TATATGTTTA
71301 TAGTTTAGTC TATCATACTA GGTTGGTGCA AAAGTAACTG CAGTTTTTGA
71351 CATTACTTTT AATGGCAAAG ACTGCAATTA CCTTTGCACC TACCTAATAC
71401 TCTGTCTCGC TAACTGCAGT ATCTTCTGCA TCAAATCATC TCTACTTACG
71451 GTTCTGACAT CCTCTCTAAA TCATTTTCTA CACATCCAGA ACAATCTTTC
71501 AAAAATGTAA ATCTAATTGT TAACCTCTGT ATAAATTAAA ACTCCACCAA
71551 TACCTCATTT TTCACCTAAA AGAAAATCAG AATTTTCAAG TATGATGCAA
71601 AAAGGTCCTT ATCTCCCTTT CCAACTGAAT CTCAGAACCA CTCACTATTT
71651 CGTGGTCATG GTGGGTTGTC TTCCATGCAC CTCACATAAC ATGCTATTCA
71701 TGTCTGTGTG CTTTTACACA TTCTGTTCCC TCTGCTTGAA ATTTTGTTTT
71751 TCTGCTGTGC TCCCAGCCTT CAAAACTCTG CTCATGGCTG GGCACAGAGG
71801 CTCATGCCTG TAATCCCAGC ACTCTGGGAG GCCAAAGCAG ATGGATCACC
71851 TGAGGTCAGG AGTTCGAGAC CAGCCGGGCC AACATGGTGA AACCCCATCT
 
71901 CTACTAAAAA TACAAATTAG CAGGGTGTGG TGGCACATGC CTGTAGTCCC
71951 AGCTACTCAG GAGGCTGAGG CAGGAGAATC GCTTGAACCA AGGAGGTGGA
72001 GGCTGCAGTG ACTAGAGTTT ATGCCACGGC ACTCCAGCCT GGGGGACAGA
72051 GTGAGCACCT GTCTCAAAAA AAAAATCTGC ACATTATTTC CTGAATACCT
72101 ATTACCACAC CCAAATACCC ATTTAGTTGT CGAATATTAC TTTTACCCAT
72151 ATGCTTACAA GAAACTTCTG TTACATAATC ATTTATTTAC AGCACAACTC
72201 TGTGAATCCT TGAAGGACAG GAGCCTTAAC TTTTTTAACT TTGTATCCCC
72251 AACTGCCTAA CATGAATCAA GTCTTATTGA ACATATTTTA AGTGAAGGAC
72301 TTACTGCTAA AATAACTTTT AAATGGCTCT TTAAGTGATA GGCATTTAAA
72351 ATAAATCATT AATCTAACAA TGGAAGCATC ATAAATTGGT TCATTTTTGA
72401 TAGAAAGGAC ATAACTGGGA TGTTATGTTT CTTAATATTA AGAGAGTACT
72451 AGTATTTATC TTTCAAAATA TGTACATTGA AAAATTGATC TCTTTTTGCT
72501 CACTGTAAAA CTTTTAAAAA TTGGGGGAAT ACTCAACTAA TTATCAGAAA
72551 CTAATTATCA GATAATCAAT TCATTTTTCT ATTATACTCT CCTAGGAACA
72601 CTAAAATGCT TCTGTGAAAC TTTCTAGAGT TTGCTCCCCT GACCACTTCA
72651 AGTTGCCATG GTAGCTACTA GCGACATGTG GTTTCTGAAC AAGTGAAATG
72701 TAATTAGTTC AAATCGAGGT ATGCAGTAAG CATAAAATGC ACACTGGATT
72751 CCAAAATCTT AATGGGGAAA AAGATCTCAA AATTTGAAAT ATTACTATTT
72801 TGGATTACTG AATTAAATAA AATTTACTAC AATTGTTCCC AAATGTTTCC
72851 TTTTCCTCAA ATTTTTAACA TACCCACTAG AAAAATTACT TATGTTGCTC
72901 ACATTATATT TATACTGCAC AGCACTGTTA TATGCAACAA AGTTCACTCT
72951 GCAACAAGAC AACTGTCATT GTCACAAAAA TCACTTTTTA GACTATGATG
73001 ATCTCCACCA ATGCCTTACT GACAACCTTG CTGTGTTATC TGATTACTAT
73051 GGATCATTGG TAATAAATTA GTCAAAGAAA AACATCCATG TAATACATCT
73101 GGTGCAAAAA CACTTAATAT GCCTTATGTG AAAAACAATA TTATTTTTAA
73151 AAGCACTACC ACAAAGAAAT GAGGTTGGTT CCAACCCTCC CAGTCAGGGA
73201 GAAGATGTGT GGGAATACTG AAACAACCTT ATGGAGGTCA TGTATAAGAA
73251 GATTATCGAT ACTACTGGAT AGGTTTGGTG TCATCACAGA AATTAAATAT
73301 GAATATTTAG ACACACATGT CTGTAAGCCA GAGACATTAA GTTTAAAGCA
73351 ATAATCAACT AAGAAATTTT TCTTTAAACA GTTGAAAAAG CAAAAAGACA
73401 GGCTATAACT AAAGATAATT TAAGAGGGGA GAGGAGTAAA ATCCAGGTCT
73451 ATTTGGCTTC TAAGATCAGG CTCTTGACTA CTAATCAAAT TGACTCCTCA
73501 GCAAACTATA CTACTGATCA ATGAAAGTGA TGAATATTTG AGTATACACA
73551 AAAGTAGACA TGAAATTCCA ACAGTGGCAT ACAGTGAAGC TAAATGAAGT
73601 AAAGGGAGCA AGGTTCTTAG GCAGGAGAAA AGTGAGAGGA AACAAAATGA
73651 TCATCTTTAA AGAAGGTCAT TTCCTAAGAG ACAGCTGTAA AAAAAGAAGA
73701 GATAAATAAA AAAGACAAAA ACATTGAGCT TAAATTAGAA CAGAGCTTAT
73751 CACAGATCCT TAAACTTGAT GAAACTGTTG CTTAAGAAAG TCCAGACAAC
73801 ACATAAGAAT AAAAGCATTG GTGAACAGCA AGAAAAGTCA AAATAGCTGT
73851 CATACAGTGT AAATTGAAGA AGGACCAAAT CAGCATTGCT TTCTAATTTC
73901 CATCAAACCC TATCATCTGC CAATTTTCAT TACTGGCAGG ACACTGGGCT
73951 GAGTGATTTG TCTATAAAAG AGAATCTCTG GAAAGAAAAA ATAAATCATG
74001 GATGTCATCC AAAGAGGCTA GACCGTATCA TTGGCAGGTA GATAAGAAAG
74051 GTCAGAGGAC TGACAGAATT TAGGGCAAAA GAAAAGCAGC AGGACTTAAT
 
74101 GTCAAGAAAT ACAAAGATAA CCAGAGAAAA GAGGCTGGAG TTAAAGCTTA
74151 AGTGAAGTAA AAGGAATTAA AGCTAAATGA TGAGTAAATA CATTGAGGAA
74201 CAGGGGGTGA ATTAAGATAT GTAGCATTTC CATGTTTAAA CCTTGCAAAA
74251 ACTTCTCATT GCAGACCGAA CAGGACCCAA ACTCCTTCCC TTGCTTATAA
74301 GCCTACATGA TCTAGCCTTT GTTCGCCTTG ATGATGTCTC TACTACCCTC
74351 ACCTAGTAGG CTACTATGCT CCAAGTAAAT GATGTCTCTA CTACCCTCAC
74401 ATAGTAGGCT ACTATGCTCC AAGTAAACGA CATGCTGTCT GTTCATGAAT
74451 CATGCCAAGT CCACTCCTCA AGGTCTGCGT AGCGGTCTGT TCCCTCTCCC
74501 TGGAATGTTC TTTCCTCTTA TCTCGCATGA TTGGCTTCTT ACCATTCAGT
74551 TTTCCTATTC AGGAAGTATA TCTGGTTGTA TCTCCTAGAT GGATTTTTAG
74601 TGTAAAATTT CCAGACAAGG TTAATGTATC CTTGTATCAT CATGAAAATA
74651 ATGTTGCAAC AANGACCAGC CATTTAACTA TGACAGAGCA CCCTAAAAGT
74701 GTTTCCTGAT GAAAATCTTG GCCTTCTTCA CAGATACTCT CTTTAGTGGA
74751 TTTCTTTATA ATGAGATACA TGCTTTTGTT AAATTTCTCA ACTTTTATAA
74801 AAAGAGGTCA ACCTTACTCC TAACTCAGTC AACTATCACA GCTACTGCTC
74851 TGCCCTTTAG GAAGCATCAG AAAATACATA TACTCAATAT GTATTATCAT
74901 TCTCATAACT TAAGCCTTAG ATATGAGGTA AAATAACTAC CCTTTTTAAA
74951 GCAATTAATA TATACAAGGC ACAATGTTAT GTGAATTCCA TTCATTGTGT
75001 TCATTTAACC CTCATAATAA CACTATGAGT TTAAGACAGT ATCATTTTAA
75051 AAGCATAAAA AGTAACTTCT GCCATGACAC ACAATTAGGA TAGGAAGAAA
75101 TATTTATAAA AGATGTTAAA AAGGTGACAA AACATCCTAT CTGCAATATT
75151 CTTTAAATTA TATCCTTGGA ACTTAATCCA AGATTATGAT CTTCTTAACA
75201 GATGGGTCAG AACGCTGTGT GGTTAGAATG CACTGTTTAA GATCCCCAAG
75251 GAGGCCAGGC ACGATGGCTC ACACCTCTGT ATCCCAGCAG TTTGGGAGGC
75301 TGAGACGGGC AGACCACGAG GTCAGGAGTC CGAGACAAAT CTAGCCAACA
75351 TAGTGAAACC CCGTCTCTAC TAAAAATACA AAAAATTAGC CGGGTGTTAT
75401 GATGTGCACC TGTAATCCCA CCTACTTGGG AGGCTGAGGC AGGAGAAATC
75451 ACCTGAACCC AGAAAGCGGA GTTTTCAGTG AGCCAAGGCC ATGCCACTGC
75501 ATTCCAGCCA GGGTGACAGA GCGAGACTCC ATCTCAAAAA AAAAAAAAAA
75551 AAAAAAAAAC CAAAGCAAAT ATAATTTAGT CATTTAGGCC TTAATTTTAT
75601 ACCACTGACT TATTTTGAAG GCTGCTATAA GAAACAGCCC TATGAAACTG
75651 GTATTTTCCT ACTGCAAGGT GGCTACTTTA AGACAATTTT TCATTGCATT
75701 CTATCAAGGG ATGTCTTATT ATTATATCAT TATATCAAGT GATGTTATAA
75751 ATAGTAAGAA TCAGATTAAG GGCTCATATG TCCTTCTTTG TATTGACTGT
75801 TGAAAAGGTA TGGGGCCAAA TTTGTAGTTT GTCTGGAATT ACATATTTTT
75851 GGGGGTCTCT ATTATCTTCA TACTTATCCT ATCTAAATTT TCCATTGCCA
75901 AATTTCCTTA CTTATTTTTA GTTTTATCCT ATTGCTCATG TATTTTTATG
75951 TCTCCATAAG TCTATTTTGG AAAAAGGCAG AGTACTCATA ATTTTAGTAT
76001 ATCTTTTAGC TTTATGTTGC CATAAACCTT TCATTATATA CATGATCAAC
76051 AACAGCAAAT TATCTCACTT CAGTATTTAG TTTATTATTT TACAAACTGA
76101 TTTATGATTG CTAACATGTA ACTGAAGGTA TACACTATTA GAACACAGTT
76151 TTCAGTAGAA AGTAGCACTG CCATTGAGTA AAAAAATGTT CTAACATTAG
76201 AGCAACATTC TTATACAAGT TTGCATGTTG TTTACTGAGG TCTAAAGCAT
76251 GACTACACAA AAGGCTGAAT AAAATTCAGA TTCTTACATA CACATAAAAT
 
76301 TGTTTTATTG AGATGACAAA GTATATTTAT TATGCCACCC AGAATATAAT
76351 CCACTCTGAT AACTGCCAGT GTATGCACTT GCTGAAGTAA CTCAGTACAT
76401 AAATGGTAGC CACAACAGTT GCTGTGCATG AAAGTTCTTC TCTTCCAGAT
76451 TGAAGAGTGT ACAATCTAAA GCATTTTAAA ACTTTAAATC CCTTATTAGC
76501 TTAAATATAA TTTAAAATTT TAGTTTGCCG TACCTATAAT TTGTCTGTAC
76551 ACTAGGTTAC TAAGGGTGAT ATGATTACAT ATGTGGATAC AAAATAATTT
76601 TAATGGAAAA TGAAATTAGG GTACTCAACA AAGATAAAGG GTAATGATCA
76651 TGTACACTAA CCGTATTTGA GATTAGTTTA AGCCTGGGGT AGCTATACTT
76701 ATGTTTCACA GACCTTGAGA AGATAGGGAA AAAAAGCTTT TATCAACATT
76751 GCTAAGGAAC AGGTAAAAGC TAACATTAGG TAACTAAGAG GTGACATAAA
76801 AAAGACTGAA TAAAATATCA TGGAGGTTTC ATAATAAGAT TGGAAATTCC
76851 ATAGACTAGG AGAGAAAAGA TCCCAAAATA TACATGCTCA TTGGGAAAAC
76901 AGCTAGTAAG AACAAGGAGA GATCTCTATT TAATGATACA ATAGTAGAGT
76951 TATAATTTCC TGTATATTGT AAATTTCAAG CATTTAAACA TTTTCATTGA
77001 ATTATAAAAT ATTATTTGTA AAAGAAAGAA AAACAGCACA ACTGCAGATT
77051 ACAGATGACT AAGATAGATG AATCATGAAA AGGTGCTAGC AGAGATTTCT
77101 ATCACACCTA TCAGGGATAC ACAATTTCCA AGAATTTCAG AAGTGTTTGG
77151 TGTTCCTATT AACATAAATC CGGAAATAAC ACCTGAGTGA ACTGTCTTCT
77201 AATTCTTCAA CTGGATGGCT TTTTAGTGTA AAAGATGTTG AATACTGATT
77251 GACTTTTTAA TAATTTTATA GTATATGTCA GAAATATTGC ACAGTCCCTA
77301 TTTACATCAT TCTACAGTGG TTTTTAAAAT GTTTTAAGAA TAAAAAACAT
77351 GAAAACTTTA TTTGATTTTT CTGAGGAAAT AACTTTTTGG ATTTAATTTC
77401 AATGAAACCG TTGATAACAT TTCCCTCCCC AACAATCTCT GGCAACGATC
77451 CCTCAGATTT TAATGATTAT GTATTATTAC CTTTTAATAC AAGTAGAATA
77501 ACACTCAGGG AATTTACAAC ATTTGTTATT TTCAGTAAAT ACATTGGTTG
77551 AAGTTTAAAA GTCTATCCGT AGTAAACTTA CATCTTTCAG GAGCTTGGTC
77601 AATGTGTTCT GGACAAAGCA GGAAGATGTG ACTGAAATCC TGAAAGGAGC
77651 CGGCTCCTGC AGCACAAGGA TAATGATACA TCTGGGTACA TTTCTCTTCA
77701 CAGCATTTGA TAGTGGCTCC AAAGTGCTTA CAAAATGCAC ATTGCTGAAA
77751 GGGGTAAAGG AGAGAAATCT CTTTATAAAA CCTTGAAAAG GAATATTTAA
77801 ATATAAGCTG GGAAGGTATA AAAAACTCTC TGTAACATCA CAAGTAAACA
77851 AATTGAACCT GCAAAATATT AAACAAAGGA TTCATTAAAA ATAATAAAAT
77901 CTACATTACT CAATTTAGTG CTTTGTGTGC TACCAACTCA TCCTTCCATT
77951 CAAATTAGAA AGTTAGAATT TCATTCCTTA TATTTTCAAA AATAAATTGT
78001 GAAGCATTTT AGAAACAAAA CCTAAAATTT TTTTTTAAAA GCAAATAGTA
78051 ATATGGTTAA AGGGGCAGGT TTCTATATTG AGGATTATTA TAAAGTTTTT
78101 AAATCCTACC AAAACTAGTA ATAGGAACAT ATATTATTTA TGAGACATAT
78151 TACTATTTTT TACCCTGCCT AAAAATAAAT ACAAATAAAT TCATCAATTA
78201 TAAGTTAACA GGGACACAAA TGGTTAAAGA CTCACACACA AAAAAAACAA
78251 AACTACATAC TTCAATGTAG CAATCAACTT CAAATTTCTT AACAAAAGAT
78301 GGAAATGTTG GGGAAAAAAT TAGTCATCTG GTATCTTTCC CATTTCAACC
78351 TGCCTCCATT ATCTTGCAAG TGGTAAAATG CACAGAAATA AGCCTCAAAC
78401 AAGAGGGGCA GTCTAGGGCA AGTGAACACA TAAGTCGGAA GAAATTATGT
78451 AAAATGTTGC ATTTACTTAT TCAGTTTTCC CTTAGAATGA TTCACAAACT
 
78501 CTTCCTCATT CTCCCAAGTC CATTTTGAGT ATCATTTTCT TTGAAGAGAG
78551 TCTGATGGGC CCTGTACTAT ACAGTATGAA ATCTCTCTGT GGGAAATGAC
78601 TATCTAACAT AAATTTTTGT TTACACCGTT ACATGGTACC TACTTGCTTA
78651 TGCCATTACA TGATCAGTTT ACCTTTTTCT CAACCTAATC CAAGATCCTT
78701 CAATTGAGGC ACTATACTAT CTTTGTATCC AAAGCACCAA AAATGCTGCT
78751 TCAAACAGGC CCTAATAGAT AGGTGTTCCT ATACATATAC CAAAAAGACT
78801 TAACTTTTGG TGATCTTGTT TGTGAGTGTG GCTCATAAAC AGCTTAGTTG
78851 AGATAACTGG AGCCTCATGT AGCAGAGACA GTTGGACCCT GCTAACATTA
78901 CTGTGGATAT CTTCACATGT TACTACATTG ACTTTATATT CTGCTAATTA
78951 ACCAGGGACT ACAGTAGTTA AAATTATAAT TGTTTTCAAT GTTTTATGTG
79001 TAAATCTGTA TCTCACATAC TATCAAACTC TTCCTCACTG TCATCAGTCT
79051 ACTGCATTGA ATCCAACATA ACAAAGCTAA ATGACTCCTG AGGGCTGAAT
79101 CAGAAAGAAG AAAAGAAAGA GATACAAAAC TTTAGTCGGC CCGGTGGCTC
79151 ACACCTGTAA TCCCAGCACT TTGGAAGGCC AAGGCGGGCG GATCACGAGG
79201 TCAGGAGATC GAGACCATCC TGGCTGATAC AGTGAAACTC CATCTCTACT
79251 GAAAATACAA AAAATTAGCT GGACGTGGTG GTGGGCACCT GTAGTCCCAG
79301 CTACTCAGGA GGCTGAAGCA GGAGAAGCTT CTAAATAACT CATAAACACT
79351 AATTACTGTT GTGACACTTT AATTTTATAC AATATTTATA AGTATACAGA
79401 ATAACATTTC AGTGCTATTT TGGCACTCAA GGGTATTAAT GCATTAGAAA
79451 CACAGAAAAA AATAAATATT TGTCTTCATT GATAAAGTGT AATAACCAGC
79501 TTTTACAAAA CAGGTAATTT TTTATATTGT AACTCCCAAG GTATCTAAAT
79551 TTGTTTAGTG CATTAACAGA ACCCAGAATT AACTACAAAT ACAACACCTT
79601 GATAGTCTAA ATGTCCAAAC AGAAAGGTCA CGTAAATGTC GAAAGAAAAA
79651 GTACAATCAA TCTGTTGAAA AATGTTTATA ACAGCTCATT AACATAACTA
79701 ATTTCTGCTA AATTGTTAAA ATCTAAAATC GCTTATTTTG ATACCATATG
79751 GCAAGTTTTG CAGAAGCTTT TTGCACTAAA AATAATCTTG GCAGATAAAA
79801 TATAATGAGA GGTAAGTATG TAATAACAGA AAATAATTTA AATTGACCAG
79851 CTTCTCAATG TCTTTCTCAT TTTAGAAACA CCAATATGTT CTCTTAATCA
79901 TATCTGCAAT GAAAAATATT ATATTAGCTA AACTATTATG AAAAGTACTG
79951 GAATATATCT AACATACTAA TTTACAATAT CATATAAAGA TATCAGTGTA
80001 TAAAAGAAGG AAAATAAAAC ACTACATTTA GGAGAACGCT TTAAACCCAT
80051 ATGTTACAAT TAAATAGCTA ATGAAGAGCA CTCGGCATTA AAAGAAAATG
80101 TTTCCTATAT ATACACAGTC TGGAATGTAT CTTCCTAAAC CAAATAAAAA
80151 GTGCTTGTAA AATCATAGAC AATATCAAAG AATAGATTTC TACAGGGAAC
80201 TTCTCAAATG TCAACACTCT TATACACTAC ACATAAAAGA ATCAAACAAG
80251 TTAAAATATT AATAATATAA TAAGTAATAT ATAATACATA AGTAACAATT
80301 ACAAAGCTAA TTAACATTTG GGGAGGAGGG ATGAAGTAAA CAGCTCTTGG
80351 CCGGGCATGG TGGCTCACGC TTTTAACCCC AGCACTTTCG GAGGCTGAGG
80401 CAGGTGGATC ATTTGAGCTC AGGAGTTCGA AACCAGCCTG GGCAACATGG
80451 TGAAACTCCA ACTCTACAAA AAGTACAATA ATTAGGTAGG CATGGTGGCA
80501 TGCACCTATA GTCCCAGCTA CTCATGAGCC TGGGGTGGGA AAATTGTTTG
80551 AGCCCAGGAG GTTGAGGCTG CAGTGAGCTG TGATCATGCT ACTGCACTCT
80601 AGCCTGGGCA ATGAAAGTGA GAACCTGTCT ATAAAAATTA AAAAAAAAAA
80651 GCCCTTAAAT GGTTTATCAA CTAAATGGTT TATTTGAATC AATAATGAAA
 
80701 ATTCCTAGAC AGCATTTTTT CTTTTCACTT TTATTCATCA TTAAAAAGAC
80751 ACGGAGTAAG AAAAGGAAAA AAAGTAACAT GTTATAATAA TATTTTCCCA
80801 TATTAATCCA AGAACACAAT AACAACAACA AAAACTCAGT GAGAACTCAT
80851 ATTACCAATT TTAAGAATGA GTCTGAGGAA TTTTCAAAAC TTTTATTTAA
80901 TAGATTGAAA TAATCTGTCA AAATGTATCT ATAAAACACA ACAGAATGAA
80951 CAAAATTTCT GAGATTAAAG TTGTATCATT AAAAAATACC AAGAAGTTAA
81001 ATATTCCACA GTTCATTTAA TTAATTATCC AGTATTTATT CTGTACCAAC
81051 TATAGCAATG GTTTTAGGCT ACGTGCTCTA ATAAATAAGT ATTGTAAGGC
81101 TCCCCATGCT CCAAATTTGT TTTTAATCTT CACTAATCTG TTTTTCGTGT
81151 TAGGCAACTA GGATTCTCCC AGGATGCAGT AATAAAGAAC AAAGATGGAA
81201 AGCAAGAGAA AGAGGTAGAA TGACATGAAA ATCTGATCCA GAAGGTACAA
81251 CGTCTGTTTT ATATGAGTAC CAGAAAGGGA GAAAATAGGA GGGATTATAA
81301 TCACCAAAAT TCTAAAGGAA AAGTTATGTA TATATCTAAT AATAAAAGCT
81351 AACATTTAAT TAAACATAAT CCTCATTCAA CAGATGAGGA ATCTAAAACC
81401 CAAAGAGTCA TTATCACTGT TCCACTTATC TCTATATCCC TACAAAAATA
81451 CTCACTCTTG ATCTGTAACA ATGCCTCCTT AAACTCCCTG AATGGCAGCT
81501 TCAGGTAAAA ACTTCCCAGA AAGTTTGAAA CAACAAGGGT AAAAGGAAAA
81551 TTCTTGGAAC CACATAAAAA AGAAAACAGA TCATTACAAA AAAAGAAGAA
81601 TCAGGCATCA GTTCTCATTA CCAATACTGA ATTAAAAAGA AACCCCCAAA
81651 AAATCATTAT TTTCCTAGGT CTATAAAAAA TTATTTAAAA CCAAAATTTT
81701 AATGTCTGTT AAGCTAACAT TCAAGTCCAT GGATAACAAA TGTAAATGGG
81751 TTAAATTCTC TAGTAAAAGA CAGCAACTTT CAAAATATGC AAAATATACT
81801 ACTTAACACA TACACATATT TTACATGTAA CTATACTACA GACTTCATTT
81851 ATGTATGTGT GTGTGTGTAC ACATATATAA TATAAAATGA ATGAAATACT
81901 TTGGATATTT GTCCCCACCA AAATTTCATG TTGAAATGTA ATCCCCGATG
81951 TTGGAGGTGG GGCCTTATGG GAGGTGTTTG TTTGGGTCAT GGGGGCAAAT
82001 CCCTCATGGC TTGGTGCTGT ACTTGCAATA ACAAGTGAGT TCTCACAAGA
82051 TCTGGTTGTT CAAAAGTGTA TGGCATCTCC ACCCGTCTCC TTTGCTCCTG
82101 TTCTTGCCAT GTGAGATGCC TGCTCCCCCT TCTCTTTCCA CCATTATTTC
82151 AAACTTTTTT TTTAAATTTA TTATTTATTA TTATTTTTTT TTTGAGACAG
82201 AGTTTCACTC TGTTGCCCAG GCTGGAGGGC AATGGCCCGA TCTCAGCTCA
82251 CTGCAACCTC CACCCCCCAG GTTCAAGCGA TTCTCCTGCC TCAGTCTCCT
82301 GAGTAGCTGG GATTACAGGC ACCTGCCACC ACACCTGGCT AAATGATTTC
82351 AAACTTCTTG AAGCCCTCAC CAGAAGCAGA TGCCAGCACC ACACTTCCTG
82401 TACTGTCTGC AGAATCGTGA GCCAATTAAA CCTCTTTTTG TTATCAATTA
82451 CCCAGCGACA GGTATTTCTT TACAACAACA CAGGAGCGGC TGTATATATA
82501 TATATATTCA TACATATGCA CAATGTACAG TGACATACAC AATTCAAAAA
82551 CAGCCTTGAG GGGAAAAAAC ACAAACGCTA TCCAAAAAAT GCAGGTATAG
82601 CAATAATTGC AAAGAAACAG AATTTAAACT AAAAAGCAGT GAATAAGTCA
82651 AAGATAATAA TTCACAATAA CATAAGTTTT ATGTTACAGT GTAAATTCCA
82701 AAAGAAGCCA TAGTCATTAA AGTTTATACA CCTAATAAAT ATATGTCACA
82751 TATAGAAAGT AAAGCTGAGT AGAAATAACA TGCATAGATT AGACTTTAAC
82801 ATAGCTCTCA AATCAGGATA TTAAATAGAT ATGAAATAAA TAATAACAAT
82851 GATCGGGTTT GATTCAAACA GACACATAAA ATACACACAG ATCTCTGAAT
 
82901 TCCAAAAATA GAGAAAACAT TCTATTCAAA TGTACACAGA ATTTTCAAAA
82951 AAAAATTCAC ATGTATGGTC ATAAAGAAAT GTCCACATAT TTTAAAAAGC
83001 AGGTATTTCC TAGGTTATAT CCAGTAAAAG CAGATGCGTC CCTAGACCTA
83051 ATACAATAAA AATTAGAAAT TCACAATATA AGGATAAACT CCCAAATCTA
83101 TCCACTGGCA AAATTTAAAA GTCCTTTCTA TATAACGGCA GAGTTTCAAT
83151 CAAGAAGAAA AATCATGTAT TATTTTTAAA TGATTAAGGA AACATCAAAA
83201 AAATCTCTGA GACGTGGCAA ATGTGGTATT CAGAGGAAAA AAATACAGCT
83251 TCAAACATGC AAAACTGTAA GACTAAAGAA TTACAATAAA TAATGCCCTA
83301 TTCAAAAATT AAGCCAATAA GAATCAACAT ATAACCCATG AGATTCGCTA
83351 TCTAGGGAAG TAAACCCACC CTAATACTGG GTTTATTAAA ATGCTGTAAT
83401 GAGATCGGAG GGTTAAACAA CTTTTCGGTT TAGGAAGTGA AATAAAGCTG
83451 ACTGAAATAA AACCTAATCT ATGAAATTAT TCTGTATATG AATTGACCTG
83501 AAGTAATGAA CAGTTCAATG ACTGACTCAT GACTCTTGTT CTGGTCCAGG
83551 CTAATCATTC TAGTTGGCAT ACTATTAAAC ACACATTTCT GATTGAAACA
83601 TCTGCTAAAA AAATAACATC AAACGACATT ACAAAATCAA AATGGAAACT
83651 GCTAAGAGTG GGATTTTTCT CCTGCCAGTA TCTTTCCTTT CTGCCACATC
83701 CTCCTCCCTG CTGCCACAGC AATCTTTCTA AAGGAATACA AATATATTCT
83751 GAATCACACA CAAACCCCTC AATACAGAAT GTGTGGTAGG CAGTATTCTA
83801 AAATGCCCGC ATACCACCAA CCCCACCAAA TTTCCTACCC TAATACAGAG
83851 GCTGTAAATA TGATGAAATA TCACGGCAAG GTAATGCCAT TAATATACTA
83901 TATGGCAAAG GAATTCTGCA GATGTGATTA AAGCCATCAA TCAACTGCCT
83951 TTGAATTAAT CAAAAGGGAG ATTTGCCTAA TCTAATCAAA TAACCTCTTT
84001 GAAAGCGGAG TTTTATCCAG CTACTAGCAA AAGGGAAATT CATAGCAAGG
84051 GAACATTTCA GTACACTGTT GCTATGTTTG AAGATTAAAA GAGCCAGGAG
84101 CAATAACCAA AGAGTGGCCT CTAGGAACTG AGGGTGACCC AAGCCAATAG
84151 GCAGTAAGAA AATGGTGAGC TCAGCAGGGT GTGGTGGCTC ATGCCTGTAA
84201 TCCCAGCACT TTGGGAGGCC AAGGCAGGAG GATCACGATG TCATGAGATC
84251 GAGACCATCC TGGCTAACAC GGTAAAACCC CGTTTCTACT AAAAATACAA
84301 AAAAAAAAAA TTAGCCGGCA TGGTGGTGCA CGCCTGTAAT CCCGGCCACT
84351 TGGGAGGCTG AGGCATGAGA ATCACTTGAA CCCAGGAGGC AGAGGTTGCA
84401 GTGAGCCGAG ACTGCACCAC TGCACTCCAG CCTGTGCGAC AGAGCAAGAC
84451 TCTGTCTCAA AAAAAAAAAA AAAAGAAAAG AAAAAGAAAA TGGCAAGCTC
84501 AGCCCTACAA CTTCATAGAA CTGAATTCCT CCCACACCTG AATGCCCCAG
84551 AAGAGGATTC ACCCCCGGAG CCTCACCCAG GTAACACCTT GATTTGAATC
84601 CTGTGAGACC TTAAACAGAG AGCACAGTTG AGCCTGCCCT GAGTTCTGAC
84651 CTAAGTAAAT GGGTGCTGTT TTAAGATATT AAGTTTATGG TAATTGCTTT
84701 ATAATAATAG ATAACAAATA GAGCAGCATT TCAAGGCCCT CCCTGCTTTG
84751 GTCTCTGCCA AATCACTATT AGATTTTTAA GATTTTTTTT TACACATTAT
84801 ACTTACATTA CACAATGTCT TAACACTATG AAGTAAAAAT GTAAATATTT
84851 TAAACTCTGG CTTGGAAATG AGGAGGCAGA ATCTCAGAAA TTTTCCTAAA
84901 ATAAAATAGG AGAGGCAAGC TTTCTGAGTC CACAAAAATG CCCTCTACCT
84951 ATTGGGTCAA AGAAAAAGTA ATTTCTCTAT TTGTAACAAC ATACATAAAT
85001 TATAAACCCT GAAGAAATAG GTCCCTAATA GCTATAATCT TTAACATAAC
85051 CATAAATAAA AACATGAATA AATCTGACTA CATAAAAATG TAAAAACTTC
 
85101 TATGTGGTGA GAACACACAT TACAGAAAAA AAATCAAATG ACAAAAAATA
85151 ACCTCCAATT TGGATTACAA ATATCTAGTA TCCTGGATAT ATACAAATAA
85201 AGCATGTAAG TTATAAAAAT GAGTATAATT TTAAAATAGA CAAGGATACT
85251 TTATAGAATT TCATAATTTA AGGAAGACAT AAAGTTAGCC AATAAACTAT
85301 GGGTAATATG CAAGAATCAG TAAGCATTAT TATTTGCCAG CAGCTAAGCC
85351 AAGTACTTTA TAAGAATTAC TTTATTCCCA TCTATGCCAG GCACAGTGGC
85401 TCACGCATGT AATCCCAACA CTTCGGAAGG CAGAGGTGGG TGGATCACCT
85451 GAGGTCAGGA GTTTTAGACC ACCCTGGCCA ACATGGTGAG ACCACGTTTC
85501 TATTAAAAAT ACAAAAATTA GCTGGGCCTG ATGGCCAGGT GCCTGTAATC
85551 CCAGCTACTT GGAAGGCTGA GGCAGGAGAA TCAGTTGAAC CCAGGAGGTA
85601 GAGGTTGCAG TGAGCCGAGA TCATGCCACT GCACTCCAGC CTGGGCAACA
85651 GAGTGAGACT CCATCTCAAA AACAAAAACA AAAACAAAAA CAAAAACAAA
85701 GAAATTACTT TATTCCCATC TAACCACAAC CACTTTAGGT AGATATCACT
85751 ATTTTTCATT TTACAAATGA AGCAGAGAGG TTAAATTTCC TTGTTCAAGT
85801 CCATAGAGTC AGGAAATGAA AAAGGAACTG AACTCAAGGA GTTCATAATT
85851 ATTTATATAC AAGTAATAAA GACAAATTCA TTGACTATGG AAATGTACTG
85901 AGTATTTTGT CTTGTTTACA TAATGGTTTT TATTTAGGTT GTAAGAAAAT
85951 AACATATAAC CAGGCACGGT GGCTCGTGCA TGTAATCCCA GCACTTTGGG
86001 AGACCAAGGC AGGCGGATCA CTTGAGACCA GGAGTTCGAG ACCAACCTGG
86051 CCAACATGGC GAAACCCCAT TTCTACCAAA AAAATACAAA AATTAGCCAG
86101 GCGTGGTGGT GCACACCTGT AATCCTAGCT ACTTGAGAGG CTGAGACAGG
86151 AGAATCGTTT GAACCCGGGA GGTGGAGGTT GCAGTGAGCC AAGATTGCAC
86201 CACTGCACTC CAGCCTGGGT GACAGAGCTG GACTTTGTCT CAAAAAAATA
86251 AAAAATAAAA AAGAAAGTAA GAAAAGAAAG TAGCCACTTT GGGGTTAGAA
86301 TCCATAATAC TCTCAGTGTC AACCAAAAGA AAGGTACTGC AATGTTCACA
86351 GAACACAATT CATAATGACC TCAAATTGAA AGTCCTCAAA ATGCCCAACG
86401 GTAAAATAAA TAATTTATAG TACTCTTAAC AACAGAATAC TATATATCAA
86451 TGAGAAAAAT AAACGACTGG AAGGAACAAT TTGGATTTAT CTCATAAATA
86501 TATACGTGAA AGATGACAGA AAACAGTACT TAGTAGCTCA ATTATACGAA
86551 GTACAAAGCC AATTATGCAA AGTACAAACA TAGGCAAGAC TCACCTATGT
86601 TGTTACTAGA TGATAGTGCA TGTCTCTGGG GAGTTAGGAA TAGCAGCCAA
86651 CAGGAGGCTT GAATGGAATA TCTGGGGCGC TATTATTTGT TTCCTGATGT
86701 GATTTAGTAT ATAGGTTCTA ATATTTTGGG TATTCCTAGA AATGTGTACC
86751 TATTATTCAC TTGCTCTATA TATAGAGAGA GCTATATAGC TCTCTCTCTA
86801 TATATATATA TAGCTATATA TATAGCAAAG TGAATTTTAT ATATAATTCT
86851 TCAACATATT TTTAGAAAAA TTAAGTTTTT TTAGAAAATT AAGAGAATTT
86901 TAGATAAAAA CTCAACTTTT AATGGAAAAT ATTGCCTTTT TCTTTCTCAC
86951 ATGAAATCCT CTATCAAGGT TAGTATCCTA CCTGGCATCT CTCCAATACT
87001 GGGTTAAGAG TTTTTCTTCT CAAGTAACCT GTATTATATT AAGATGATAT
87051 TAACTGAAAT AATCAGATAG ATGACTGCGC CATAAAGCTA ATTCTAAGGT
87101 ATCTGTCCTT AGCCAAAAGC AGAAAAGGGA TACACATAAC TTCCACATAA
87151 AACATCCACC CCCATTTCTT TGGTTATTCT TAAAAGCTCA TAATGTATAC
87201 AATGATAAAA TTTATTGTTT CAAATTAGGG TTTTTGAAAT TGAAATATTA
87251 ATTTAAACAC GTTTGCTTAA CACAACTTTA AAGTTAATAA GAAAACACTG
 
87301 CATATTACAG AAGAGTTAAA TACTTCAAAA AGTGGGAAAG AATTTAAAAG
87351 CTTTCACTTG AGTAGGAGTA AACACAAACA CACTAAGAAT CTAGTTGGAT
87401 ACACTTAAAT CATGTCTTTT AAGAACATAA GAACAGATTA TTTGCTTTCT
87451 CTTTAGTCCA AAACCTTGGT GTAATTTTAG TCCAAATTTG AAACTAACAG
87501 AAGAATACTC AGCTTCTTAA AAGTTTTATT TCAAGAACAG TAAAAGAACT
87551 TCTGGTCTTA CAAACATTAC ACAGATAAAA CGAGTTTCTC CAAACAGATG
87601 TATGTAAAAA TCTAGTACAT ATACCCCCAA GTTGAGTCAA TAAGGTGAAC
87651 CAGATTCTGG TTGAATGGAA TCCTTGAAAT ATAACTTTGG TGAACAAACT
87701 GTTTGTGTTT TCCACTTTTC TTAAAAAAGA AGTGATAAAG CAGATTTCCA
87751 GTAACTGCCA GCTGACAAAA TGCTACTTAC CAGGTAAAAA CCACTACCAA
87801 AAATCAGAAT CACAGAAGAG CTATCAGTAA AATTATTAAT GCCACAAATT
87851 AACATGATGT TAACAGCTGA TTTATGCATC AAAAGAGTAT TTATATAATC
87901 ACAGCAAATA ACTCAAATCT TTTACATCAC TAGTAAACTC AAAAAATATA
87951 GCAAAAAGCA ATAACATTAA ATTTCATTAT TGATCTACTA GCTTTAACTA
88001 TTTAATACTA GTACACTTAG TTTTGATTAC TTTGAAATTG AAGCAAAAAC
88051 AATAATCTTT TCTTCTTTTA ATGACTTTTT CAGTTATAGG AAAGATGACA
88101 TTAAAAGAAA ATATACAACT ATAGCTTAGC TACAGGTATA AAATTGTTTT
88151 AAAGATATGT AATTGTGCTA TTGAATTAAA CTGAAAACAT AACTATCAAC
88201 TCATGATTTT GAAAAAAAAT CTTTTTTATG TTTGGTTTTG GTAAAAGGAG
88251 AGACAAATAA GTCAGAAGAA CAGAAAATAC AGAAATAGGC AAACTTTCCC
88301 ATATTCAATT AATTTTTGAG CAAGGGGCCA AGGTAATTCA AGAGAGCAAT
88351 AAATAATCTT TTAGAATGAT GCTGAACTGC CAAATAATTC ATATGGAAAA
88401 CAAAGAAACA TGAATCTTAT CTTGTATTCT CTTATCATAT GTGTAATTTA
88451 ACCATAAATG GATTTTTAAA CCTACAAGTA ACACTGCGGC AAGCAAAGAT
88501 TTTTAATAAA GCATAAAGAG CATAATTTTT TCAACTTCAA AAAATTAACA
88551 AATGACAAAT GTCATCAAAA TGAAACCCTC TGCTGTTCAA TAAACATTGC
88601 TAAATAAATG AAAACAAGTC ACACATTAGG CAAAAATATT TATAAAATGC
88651 ATCTCCAATA ACTGACTTCT GTGAATAATA TATAAAAAAA ACTTAGAACT
88701 CAAGAAGATG ACAAGCAAAT CAATTGGAAA AAAATGGTGG TAGAGACAAA
88751 ATCTTTAACA GACATGTCAC CAAAGATACA CAAATGGCAA AAAAAGACAT
88801 AAAAAGAGGC TCAATCTAAT TAATCATCAG AGAATGCAAA ATCAAACCCA
88851 CAATAAATTG CACTAAATGC CACCAGATGG CTAAAATTTA GAAATAGTGG
88901 CAATAGCCAA TGCTGGCAAC AATGTACAGC AAATGGAACT CTCAGATCTC
88951 TAGAAATGGA AAAGTATACA GACACTTTTT AAGAAACTGC XTGAAACTGT
89001 TTTCCAAAGT TAAGAATATT ATTTCCAACA AGTCAGAAAT TCCAGTCCTA
89051 GGTATTTACC CAAGAAACAT GAAAAGATAC ATCTACAACG AAGCATGTTA
89101 CATAAATGCT CACAGCAGCT TTATATACTG GCCAAAAATT GAACGAAACT
89151 TAACTGTAAG CTAGTGAATG GACCAATCTC AAAATGATTA TGCTAAGCAA
89201 AAATAAGGCA GATGGAAAAG ATTCTATACT GTATGGTTCC ATTCATATAT
89251 TAGTCTAGAA AAGGCAAAAC TATAGGAAAG CAACAGATCG GTGATTGCCA
89301 GGAGCTGGAG TGGGAGGTAG GCAGTAACTT CAAAAGGTCA TGAGCGAATT
89351 TATGGGTAAA GGGTCTATTC TGTATCTTGA CTATGATGGT AATTACATGA
89401 CTGTTCATAT TTACCAAATC ACATTCAATA CACACCTTAG AAAGGGTGAA
89451 TTATAGTGTA TGCAAAATAT ACCTTAACAA AACAATGACT CTACACATAA
 
89501 CTTTATTTAT TTATTGGGTA ACAGCATAGG GACTAATATC AGAATAACTT
89551 TCCTAAAATG TCTCAAATCT GAAGAAAATG TAAAAGCTAA CTAATTGAAG
89601 TTACCTGTAA ATAACCAAAA ACTGGCTGGA ATGGGAGGAC ATTTACCCT
89651 TGGAAAAAAA GCGGAATGTC ATTGGCTAAA ATCTATATTT AAATAGAGTT
89701 TCAGTCTGGG CACGATGGCT CCTGCCTGTA ATCCCAGCAC TCTGGAAGGC
89751 CGAGGAAGGT GGACCACCTA AGGCCAGGAT TTTGAGAGCA GCCTAGCCAA
89801 CATGGTGAAA CCCCACCTCT ACTAAAAATA TAAAAATTAG CTGGGTGTGG
89851 TGGTGGGCAC CTTATAGTCC CGGCTACTTG AGAGGCGGAG GCATGAGAAT
89901 CGCTTGAACC TGGGAGGTGG AGGTGGCTGT GAGCCGAGAT CGCGTCATTG
89951 CACTCCAGCC TGGGCGACAG AGACCCTGTC TCAAAAGAAA AGAAAAGAAA
90001 ATTCTACAAC TAATAAGTAT AGTAATTTCA ATGAAAATTT TAGTGGATGG
90051 GCTTAACAAG AGATTGGTAG AAAATTGGTC CATCTCCTTG AAAATACATG
90101 AATAGTGTAT CTATTCTGAG GAACTAAAAG TAAAAACACT GGGAAGAAGA
90151 TGAACAGACC CTCAGTAATC TACAAAATAT CAAATATACT AACATTCATG
90201 TAATTAGAGT TACAGAGGAA GAGGAGAGAG AAATGAGACA GAAAAAGAAT
90251 AAGAAATAAA AGTCAAGCTT TTCACAAATT TGGTGAAAAA TATCAACTTA
90301 CACTTTCAAG AAGCTCAGCA ACCCCCAAGC AGGATGAACA CAAAGAAAAC
90351 CACACCTAGG CACATCATAG TCAGACTGCT GAAAACCAAA GATAAAGAGA
90401 AAATCCTGAA AGCAGCCAGA GAATATCAAA ACATTATTAT ATACATGGGA
90451 ATAATGATAC TACTGACACC ACACCTGACT TCTTATTAGA AACCATGGAA
90501 GACAGGAAAC AATGCAACAT CTTTTAAATG CTTGAAGAAG AAAAACATAA
90551 TCATCCCAGA ACTCTATATT CAGGGAAAAA AAGGCAAAAA TGCATGCAGA
90601 CTTCCACTTC CAGACAAAAT GCGAGAAAAT GTACTTTCCC TGTTCTTTCC
90651 ACTAAATACA GCTTTAAAAA CCCTTGGCGG TGATACATAA GACAAACAAA
90701 GGAAGACAAC AAAATGTAGA GAGACGGCAG ACCAATTAGG GCTTCAGGTG
90751 CCAAGAAACA CAAGAGGATG AATTCCCTAG GTTTCCTTTC TGACTCATAT
90801 ATACAAAAAA GTTGCTAGAG AAGCAGACAA CTCAGAAACA CCAACAGGCA
90851 CACACATACG TAAAACTCCA AGAAAATTCT GCTGTCTCTT GTCAAAAAAG
90901 CAGGGAAGCG GCAGGCTACT AAGAAAAAAA AAACTCACAG ACAACAACCA
90951 ACCAGTTTAA GCCAAAGGGG ACAAATAAAA CCCCAAGCCA TATACCTTTT
91001 GCAAAGACTT AAAAGGAGGA CATAGTCAGC CATTCCCCAC TCCTACCCAC
91051 AGACAACATG ATACCACAGC AGGCCAAGGG GGGACACAGG ACACTCATAC
91101 CCACTGAGCA GCAACGCGGC TTCCTCCCTC TAGAGTGGGA GTGGAAAAAA
91151 AGTGGGGAGC CTGAGTTCAA CACCCACCCA ATGAGAAGGG CATATTCCTA
91201 TCCCTCCCTC TACAGAGATA TGACAGCAGA GGCCTACTGG AGAATCCAAA
91251 CTCAAAGCAC TGCTCAGTGA TAACTTAACC ACCCCTCCAT CCCAGTGTCA
91301 GTGGAGGCCA AGTGAAAAGC AGGAACCTTT TGAGCCAGGG TGGTATTCAC
91351 AAAGGTATAA TGCAAGTCCA GAGTTTTCAT TCCTACTCAG CATTTACTAG
91401 GTGTACGTCT CCATCAATGA GTCAACAAAG GCCTACTATG AAACTGGAAT
91451 CTTCACATAC ACCTGGGGGT AGTAACAAGG CTATGCCCCA CCTCCACCCC
91501 AAAAGCAAAT TTTCAGATAA ATCCTACTAA AAGGGGGGAT TTAAATAAAA
91551 TGCAGAGCTT CATAACACAA CACTTAAAAT GCAAAGGATT CAACCAAATA
91601 TAACATGTCA TACCATACTA AGAAGCAGGA AAATCTGAAC AGGAATTTGA
91651 AAATATAGTC AAAAGGTGCA AACAATGAGT ATTCATAGAT GTCATAATTA
 
91701 TCTATCAAAG ATTTTAAACT ATATATTATA AAACTGCTTC AACAAGCAAT
91751 TACAGCCAGA CACAGTGGCT CACGCCTGTA ATTCCAGAAC TTTGGGAGGC
91801 AGAGGTGGTT GGATCACTTG AGGTCAGGAG TTCGAGACCA GCCTGGCCAA
91851 CATCGTGAAA CCCCATCTGT ACTAAAAATA CAAAAATTAC CCAGGCATGG
91901 TGGTTCACAC CTGTGATTCC AGCTACTCGG GAGGCTGAGG CATGAGAATC
91951 ACTTGAGCCC AGGAAGCAGA GGTTGCAGTG AGCTGAGATC ATTCTACTGC
92001 ATTCCAGCCT GGGCAACAGA GCAAGAGTCC GCCTCAAAAA AAAAAAAAAA
92051 AAAAAAAAAG CAATAACAAA CATAATTTTG AAACAAAGGA AACAATACAA
92101 TGTTTCGCAT AGAAATCAAG AAAGAAACAA ATACCAAGTT GAAAATTAAA
92151 AAATATGCTA GCAATAATAA AATACAAACT AAAGCTGAAC ACAAAATGGG
92201 GGAGATTACC CAACCTAGAG AAGAAAATGA ACTGATTAAA AAAAAAAAAA
92251 AGTGGCCAGG TGCGGTTGCT CAGGCCTGTA ATCCTAGCAC TTTGGGAGGC
92301 CGAGATGGGT GGATCACCTC AGGTGAGGAG TTCAAGACCA GCCTGGACAA
92351 CATGGCAAAA CCCTGTCTCT AATAAAAATA CAAAAATTAA CTGGGCATGG
92401 TGGCACACAC CTATAATTCC AGCTACTCAG GAGGCTGAGG CAGGAGAATC
92451 ACTTAGACCC AGGGGCGGAG GCTGCAGTGA GCTGAGATTG TACCACTTCA
92501 CTTCAGCCTG GGCAAAAGAG CAAAACTCTG ACAATAAATA AACAAATAAA
92551 TATATATATA TTTTTTAAAA AAAGGCATTA AAGACACTTG TGGTACTATA
92601 ATAAATGATG CAACATTCCT GTCACTGGAA TCTCAGAAAA GGAACATGAT
92651 GCAGCTGAAC AATTATTCGA AGACCCACAG ATTCAAGAAA TTGAATAAAT
92701 CCTAAGAGGA TACACACAAA TAAATCAATG CTAATATATA TCATAAACTT
92751 NTGAAAACTA ATGACTAAAC TTAAAAACCT TGNGATAAAT AAGAGAAATG
92801 ACACCTTATC CACAGATGAA AAACAACTCA NATGACAGCA GATTTCTCAC
92851 CCGAAACCAT GGAGGCCAGA ACAAAGCAGC ATAACATTTT TGTAAGTCCT
92901 AAAAGAACTG ACAACAAAGA ATTCTATATC CAGCAAAAAC ATCCATTAGT
92951 AATGAAAGGG AAATCAAGAA TTTGTGAGAT GAATGAGAAC TAAAAATTAG
93001 TCATGAGACA AAACTACACC AAAAGAATAG TGAGAAAAGT TATTGAACCA
93051 GAAAGGAGAA TGATAAAAAG AAGGATTCTT AGAACATCCT GAAGGAAGAA
93101 AGAACAAACA AATAGTAAAA CTATGGGCAA TAAAACAGAC TTTACTCTTC
93151 CTTGAGTTTC CAAAATAATG TTTAACAATT GAAGCAAAAA TTGTTACATG
93201 ATGCAAAAGC TATGGTGGGT AAAACTGTTG ATGCATTTCC AAAACTGAAG
93251 GCAGCTGGCA CCAAGCTGTA TTAGAAGTTG TCACATTCTC AACTGGTACT
93301 ACAACTACAG TAAAAATAAT GTAGAGTATC ATTGAGAATA TCACTGATGA
93351 AGCAGCAAAA ATCTTTAATG CTTTAAGTCT TGATCCATGA ATACACCATC
93401 TTTTAATTAT CTATGTGACA AAATGAGAAA TGAAGCGTAA AATAATTGTT
93451 ACACCACAGT TTTTCTTGAG GAAAGAATTT GTGATCATGT GATTTATAAA
93501 CTGAAATAAC CTGCTTTTTT AATGGAACAC TGTTTTTACA TGTAAGAACA
93551 ACTAAGAAAC CACAATTATT AAAACTTGAT TATCTGACAT ATTTCTTCAA
93601 AAATGAATAA AGTGAGCCTG CCACTTCAAG GAAAACAACT AACATGAGTG
93651 GACAATAAGA AGATTTGAAA ACCTTACAAC CAACAATGTA AGCTTAACTA
93701 ATTCCAATAC TTAAAGACTT TTCTGATGAG AGCAGTTGAT TATAACAAAT
93751 GTGAATTTTT GTTATTAACT AAAAATGTGT AGGGACTAGG TAAGCTGGCT
93801 ACACTTGTAA TCCCAGCACT TCGGGATCAC TGGAGGTCAG GAGTTCAAGA
93851 CAAGCCTAGC CAACATAGTG AAACCCCATC TCTACTAAAA ATATGAAAAT
 
93901 TAGCTGGGCC TGGTGGTGCA TGCCTATAGT CCCAGCTACT CAGGAGGCTG
93951 AGGCATGAGC ATCACCTGAA CCCAGGAGAC GGAAGTGAGC TGAGATCGCA
94001 CCACTGCATT TCCAGCCTGG GCAACAGAGT GAGATTCTGT CTCAAACAAA
94051 CAAAACAAAA CAAAACAAAC AAACAAAAAC AACCGTATCA ATAGTTGGAA
94101 TATCTGGATC ACTCAGTGAA CCAATATTTT CCAAAGACCA CTTAGTAATC
94151 TTATAAAATC ATATATGGGT AAAAGATCCC CTGAAATTGC AAGGTAGTCT
94201 AATAAATTAT ATATATATAT ATATATATAT ATATTTTGTC TCAGAGATAA
94251 AGTCTCACTC TGTTACCCAC GCTGGAGTGC AGTGGCACAA TCTCGGCTCA
94301 CTGCAACCTC CACCTCCTGG GTTCAAGAGA TTCTCTGCCT CGGCCTCCCC
94351 CGTAGTTGGG ATTGTAGGTG CCTGTTACAA TGCCTGGCTA ATTTTTGTAT
94401 TTTTAGTAGA GACGGGGTTT CACAATCTTG GCCAGGCTGG TCTTCAGCTC
94451 CTGACCTCGT GATCCACCCA CTTCAGCCTC CCAAAGTGCT GGGATTACAG
94501 GCGTGAACCA CTGCGCCCAG CCCATAAATT ATAATTTTAA AGGATAAAGA
94551 TATAAAGTTC ATTGATGGGA TTGCAGTCTT CAGACTGCAC CAAACCACTT
94601 GAAAAACTTG AGAAACTACC ACTTCTCAAG CTTTGGTTTT AGTATCAAAC
94651 AGGAATATTC ATTAGGGCTT GTGCTTTCAT TCTTACAGTA TATTTTAATA
94701 AACAGAAGTT ATTTCTCAAA TGTAGTTGAA TGCTCCAATA TTTTCCTTTA
94751 CGTTCAGTGC TGTATCTCAA GAACTCTTTT ACAAAAATAT TATCCTATAT
94801 TATCTTTTTA AGTATAAATA AAAATAATTA TCTTTTTAAA AGCTTATGAG
94851 TTTAGGTGGA AATATGAAGG GCCAAAAGGA ATTGGAAGAC ATTTTCCTAC
94901 TATTTGTTCT TTCTCTTGAT TTTCATGATT CATTATTCTT GTAGAAGTGA
94951 CCTTACCACA GTATAAGGTT CACTTATAAT CACTGTTCTG CCTTGTAATT
95001 TTAGGTGAAT TTGTTACAAA ATATTATGAA ATCTATAGGA AAAGCCAACT
95051 TTCAAAGCTA TTAAGCATTT GACTTAACAG TTGAGGGCAA TATTATTCAG
95101 AAAAATTTCA ATCTCATTCC TCAGCGCAAA AAACTGAAAT AAAACTTTAC
95151 CACTGCCAAG TCAGTAAATT GCTATGTAGA ATGGCAATTC AGTATATTCT
95201 GCCTCTATAT CTAACAAAAA TTCATAGGAT TTGATGATGA AGTCCACAAA
95251 TGCCAGTCAA AATCACTGCT GACACTATTA TATAATAGTT CACTAAAACA
95301 TGACAGATTT AAATATTTTC TGCAAAGTAC CTGCTAATAA ATAATACAAT
95351 AAATAATCAG GCTTTAAACA CCTGACATTT AACAAGCTTT GTAAATTTGT
95401 CCAACTCAAG TCTTTCTGCT CCACAGTTAT TTTTGCCACT ATCAACTGTA
95451 ACACATCTTA GCGGATTCCA CTTCAGGTTG TACTAAGTTA GTGTTTGCTC
95501 AACTTTTCTG AAAATATTTT CTCTTGTAGT TGTTTCACAT AGACTATGCA
95551 TAAACACTAA TTCTTCGGCA TCTTCAAACT CAGCACTGAC TTCTGGAATA
95601 TGTAACAACT GAACAGTACT GGTACTGGTA ACACCTATTC ATCCATCAAA
95651 AGCCTAAGAA ACTCCATGAA AAATCATTCA CCCTTCTTTC CAAAATGGCT
95701 TGATATTACT CCCAATGTCC TTAACTTTTA GAGCAATTAC TCTCGCTGAA
95751 AGGTTAATAG TCTTTTTAAA AGTTTATTTT CTATGGACAC ATCTTTGGTT
95801 ACCGTAATGA AACATAATTT AATTACCTCC TGTTGGTCAT GGTTTTCCTT
95851 CCTTGGCTCA CACATCAGCC ATTGAAAAAA CTTACTTTAA TTATAGCCTA
95901 ATTTCCATTT TTTGTTTTTG AGAAGAAATT CTGCTGTGAT AAAATTTTGT
95951 TTTAATTTAC TATTTTTTTT CCTACCTTTT AGTTAAGTTA ATCCCTGTGG
96001 TTGGGAAAAT TGTAAAAGTG CCCATTCTGG CAATGTTGCC ATATATAATA
96051 TTCTTTTAGC CAGCCATAGT CTTCTAGCAT AACCAATATA ATGCTTTTGC
 
96101 TATCTAATTT GTTAATAATA ATATACATTC CACTATGCCA ATAAAGTGTT
96151 ACATATAAAG TCCACTTTTC TCCTTTTCTT GTTTTAACAT GATAGGTAAG
96201 TATGCACTGG TAATGAGAGA AATAAATAAG ATGCCAGGGC AATATGTGTG
96251 GTGCACACAC ACACACACAT ACTGACAAAC AGTAACTATG TCATTGTGAT
96301 TCGTAGCATG CAGAGCAGCA ATTTGAAATA TATGTGCTCT CTGCAGCAAC
96351 CAACTCTGAC ACTGTAGTGC AACGGAAGTC ATATATAATG CCCAAACAAT
96401 TCAGGGAAAG GAAAAGTCAC GTGCCTCTCA TTTTAACTGA AAAGCTACAA
96451 ATGATTAATC TTAGTGGGGA AGGAATATCA AAAAGTGAGA AAGGCCTAAA
96501 GCTAGGCCTC TTGTACCACT CAGACAATTA TGAATGTAAA CGAAAATTCT
96551 TGAATAAAAT TAAAAGCACT ATGTCAGTGA ACATGAAACA ATCTTATTGC
96601 TAATATGAAG AAAGTTTTAG TGGTCCATAT GAAAGATCAA ACCAGCCACA
96651 ACATTCCCTT AAGCAAAAAA CCTAATCCAC AGCAAGGCTC TAAGCTTCTT
96701 CAGTTCTATG AAGGCTGAGA GAGGTGAGAA AACTGCAGCA GAAAAACTGG
96751 AAGCAAGTAG AAGTTGGCTC ATGAGGAAAA ATGCTCTTGT CATAACATAA
96801 AAGTGGAAAG TGAAGCAGCA AGTGCTGACA TGGAAGCTGT AGCAAGTTAT
96851 CCAGAATATC TATCTAAGAT CATTGCTGGA GGTGGCTATA CTAAACAAGG
96901 TGTTTTAAGT AGACAAAAGA GCCTTCCATT AGAAGAAGAT GCCATCTAGG
96951 ACTTTCACAG CTAGAGAGGG AAAATCAATG CCTTGCTTCA AAGTTCAGGC
97001 TGGGGCCACA CCTGTTATTC CAGCACTTTG AGAGGCCAAG GTAAGAGAAT
97051 CACTTGAGCC CTAGGAGTTC AAGGCCAGCC TGGACAAAAA GGCGAGACTC
97101 TTGTCTCTTT TAAAAATAAA TAAATAAATA AATAAATAAA TAAATAAATA
97151 AAAACTGACT CTCTTGTTAG GGGCTAATGC AGCTGGTAAC TTGACGTTGA
97201 AGCTAATGCT CATTTGCCAC TCAAAAAGCC CTAGAGCTCT TAAGAATTAT
97251 GCTATATCTA ATCTGCTTGC TATCTACATA TGGAACAACA GTCTCCTGGA
97301 TGACAGCACA TCTGTTTAGA GGTTGGCTTA CTGAATATTT TCAGTCCACT
97351 GTCAAGACCT ACTGCTAAGG AAAAAACATT AATTTCAAAA TATTAGCACT
97401 CACTAACAAT GGACCAGGTC ATTAGAGGGC TTTGATGGAT ATGTTCAGAA
97451 AGAAGAATGT TACTTTCATG CCTGCCAGTA CAATACAACA TCTATCCTGC
97501 AGCCCATGCA TCAATGAGTA ATTTCAATTT TTAAGTCTTA CTGTTTAAGA
97551 AATACATTTC AGCCAGGCAC GGCAGCTCAT GCCTGTAATC CCAGCACTCT
97601 GGGACACAAA GGCAGGAGGA TCACTTGAAG CCGGGAGTTC GAGAACACCC
97651 TGGCCAACAT GGCAAAACCC CATCTCTACT AAAAATACAA AAATTAGCCA
97701 GGCGTGGTGG CACCCACCTG TAGTCCCAGA TACTCGGGGG GCTGAGTCAG
97751 GAGAATCGCT TGAACCGGGA GGCAGAGGTT GCAGTAAACC AAGATCACAC
97801 CACTGCACTC CAGCCAGGCT GAGAAAGCGA GACTCCATCT CAAAAAAAAA
97851 AGTGATTCCT GTAGTAGATT TGGGCAAAAT AAACTGAAAA CCTTCTGGAA
97901 AGGCTTCACC ACCGTAGATG TCATTAAGAA CATTCATAAT TCATGAGAGG
97951 AAGTCAAAAT ATCAACATGA ACAGGTGTTT GGAAGAAGTT GATTCCAACT
98001 CTCATGGATG ACTTTGAGAG GTTCAAGACT CAAGTGGAGG AAGTCACTGC
98051 AGATGTAGTG GAAACAGNAT GAGAATTAGA AGTGGAGCCT GAAGATGTGA
98101 CTGAATTGCT GCAATCTCAT GATAAAACTT GCACAGATTA GGAGTTGTTC
98151 CTTACAGATG ACCCAAGAAA GTGGTTTCTT AAAGATGGAA ACTACTCCTG
98201 GTGAAAAGGC TGTGAAATTA TTGAATGACA ACAAAGGATT TAGAATACTA
98251 TATAACGTTA GTTGTTAAAG CAGTGGCAGG GTTTGAGCTG ACTCCAATTT
 
98301 TGAAAAAAAG TTCTACTTTT GGGTAAAAAA TGCTATCAAA CAGCATCAAG
98351 TGCTACAGAG AAATCTTTCA TGAAAGAAAG AGGCAATCAA TGTAGCAAAC
98401 TTCATTGTTG TCTTATTGTA AGAAACTGCC AGAGGCACCC CAACCTTCAG
98451 CAATCACTGC TGGCATCAGT CAGCAGCCAC CAACATCCTC TATCAGCAAA
98501 AAATCTTACA ATTTACTGAA AGCTCAGATG ATCAGTAGCA TTTTTTAGCA
98551 ATAAAGTATT TTTAAATTAA AGTATGCACA TTGGTTTTTT AAATATAACG
98601 CTATTGAACA TTTAATAGAC TGAAGTAGAA TATAAACATA ATATTTACAT
98651 ACACTGGTAA ACCAAAATAT TTGTGGCCTT GCTTTATGGT GATATTCACT
98701 TTATTGTGGT AGTCTATAAT GGAACCTACA GTATCTCCAA AGGTATGCTT
98751 TGTATTTACC TTTGGTACCA AATTCTAGAT ATACATCTGC ATATACCTGG
98801 GTATTTAACT TCCTGGTCTC ATGTTCCTTA AATAGTTAAT AAGAAAGAGT
98851 AATTATGAAC CATGCAGACC AATTAACAAG CCTAACTTCC AATATTTTCT
98901 TCACTCTGAA ATAAGTATTT CCAACATTGT ATTATGTCAG AAGTTCTCGG
98951 TTTTGGGGGT GCTGTATCTT AATTTTTCTG TACTCTATTT TCAACTTCAC
99001 TGACATAATA CAAGCTCTAT CTTCCTTACA AATTTATAAG GAATCAAATG
99051 AAATGAAATA TATAAAAGTT GTCCTCTTTC TCCCATCTTC AAAAAACTTC
99101 CCCCTGCATT TCATTTCCTG TTAGCCCTCT CTCCTTCAAC AGCCTGGTCT
99151 GTAGAAAGAG TCCATACATG GCCTCCCTCC ACTCCCTCAT CTTCCATTCA
99201 CTCCTTGTTC CAAGGCCTTC CCAACACCAC TGAAACTTTT TTCAATGCCC
99251 TTTTTTTAAT TTTTTTTTAA AAGCAAATTC TGCCCCCAAC TTTTCATTCT
99301 TTCAGTTGAA CCACTTTTTC CAAGACACTG CAGATGTAAA AAATACGTAG
99351 TTAAGGCCAG TGTCCAGTGG CTCATGCCTG TATTCCCAGC ACTGTGGGAG
99401 TCCAAGGCAG GAAGATCACA GGTCTGGAGT TCAAGACCAG CCTGGTCAAC
99451 ATGGTGAAAC CTCATCTCTA CTAAATATAC AAAAATAAGT CAGGCATGGT
99501 GGCAGGCACC TGTAATCCCA GCTACTCAGG AGGCTGAGGC AGGAGAATTG
99551 CTTGAACCCA GGAGGCGGAG GTTGCAGTGA GCCAAGATCG CACCATTGAC
99601 TCCAGCCTGG GGCAAAAAGC AAGACTGTGT GTGTGTTTGG AGGGGGGGAC
99651 CTGTTAATAA AAAGAAAAGA AAACAACTCA ATTTCAAAAT GGGCAAAGGA
99701 GGCCTGCCAT GGTGGCTCCT ATCTATAATC CCAGCACTTT CAGAGGCCAA
99751 GGCGGGTGGA TCACCTGAGG TCAGGAGTTC AAGACCAGCC TGGCCAACAT
99801 GGCAAAATCC CGTCTCTACT AAAAACACAA AAATTAGCCA GGCATGGTGG
99851 TGCACGCCTG TAGTCTCAGC TACTCAGGAG GCTGAGGCAG GACAATCACC
99901 TGAACCCAGA AAGTGGAGGT TGCAGTGAGC CGAGATCATG CCACTGCACT
99951 CCAGCCTAGG TGACAAGAGT AAGTCTCTGT CTCCAAAAAA AACAAACAAA
100001 AATGGGCAAA GGAGCCAAGC ATGGTGGTGC ACACATAGAG TCCCAGCTAC
100051 TGAGAGGCTG AAGTAGGAGT ATCACTTGAG CTAGAGGATC ACTTGACCCC
100101 AGGAGTTCAA GGCTGCAGTG AGCTAGAACT GTGCCACTGC AATCCAGCCT
100151 GTGTGACAGA GTAAGACTCC AAAAAAGACA AGGGCAAAGA CATGACGAGA
100201 GAGAAAAAAT AAGTATAAAA GTAAAGACAG AGAGAGAGAG ACTTCAAAGA
100251 GGAGGACAGG GCAAGACACA GTGGCCCATG CCTGTAATCC CAGCACTTTG
100301 GGAGGCTATA ATGGGAGGAT CACTTGAGCC CATGGGTTCG AGACCAGCCT
100351 AGGCAACACA GCGAGACCCC AGTATCTACA AAAACTAGCC GGGAATGGCT
100401 GGGCATGGTG GCTCACACCT GTAATCCTAG CACTTTGGGA GGCCGAGGCA
100451 GGCGGATAGC CTGAGCTCAG GAATTAGAGA CCCGCTGGGT AACAAGGAGA
 
100501 AACCCCATCT CTACTAAAAT ATAAAAACTT AGCCAGGGGT GGCAGCATGC
100551 ACCTGTAGTC CCAGCTACTT GGGAGGCTGA GGCAGGAGAA TCACTTGAAC
100601 CCAGGAGGCA GAAGTTGCAC GTGAGCTGAG ATCACACCAC TACACTACTG
100651 CACTCCAGGC TGGGCAACAC AGCTACGACT CCCTCTCAAA AAAAAAATGT
100701 AAAACTAAGC TGGGCACAGT GGCTCACACC TATAATCCCA TCACTTTGGG
100751 AAGCCGAGGC AGGCAAGATC ACCTGAGGTC AGGAGTTCAA GGCCAACCTG
100801 ACCACTATGA TGAAACCCCA CCTCTACTAA AAACTCAGAA ATTAGCTGAG
100851 CATGGGGGCA TGCGACTATA ATCCCAGCTA CTCGAGAGGC AGAGACAGGA
100901 GAATCGCTTG AACCTGGGAG GAAGAGGTTG CAGTGAGCCG AGATCACACC
100951 ATTGCACTCC AGCCTGGGCA ACAAGAGTGA AACTCTGTCT CAAAAAAAAA
101001 AAAAAAAAAA AAAAAACTAG CCGGGCACAG TATTGTGCAC CTGTATTCCC
101051 AGCTACTTGG GAAGCTAAGA TGGGAGGGTC ATTTGGGCCC AGAAATTCGA
101101 AGCTGCAGTC AACTATGATT GTGCCACCGC GCTCCTGTCT AAGTAACAGA
101151 ATGAGATATT GTAATTTAAA AAATAAAAGG ATAGGTAGGA TTTACCGACT
101201 GAAAGATGAG AGTGGAAAAA GGAAGGAGGG ATAGCAAGAA TGGCGGGCAG
101251 GTAGACAGAA AGGTAAAATT CAGCCATGTT CAAAAGGGAA CAAATAAAAG
101301 CATTGAAAGG TAAGACAGAC CTAGTATATT TATGACTTGA ATCCCAAGCT
101351 AAGAAACTGG AACATAATTT AGCAGAGAGA CTCCAAAAGT TTTACAGCAA
101401 AAAGTATTAG AATCATTTAT ATACTGGGAA AAAATAGCCT CCTCATTTTA
101451 ATAATCCAGA TTGGGAAATC AGATCTAAAA CAGATGTTCA ATGGACATCC
101501 CATTTCTTTT GTAAAAGCAA CTTGAAAGGT CAGACAGCAA GTTGTGTTCT
101551 GTTTTTAATT CACAATGAAA ATCTGACTAC TCATTTATTC AACAAATATT
101601 TGAGTACCAG CTATGTGCTA GGCACTGTTC TAAACACGCC AATAACATTT
101651 ATTTAGTGGT ACTGATTCTG TACTCTAGAA CAATGGTTCT TAACTGGGGA
101701 GAGGGAGCAA TTTTTGCTGC CCAAGGGACA TCTGGCAATG TCTAAAGATA
101751 TTTTTGGTTG TCACAGGGGC AGGGGGGAGG GGAGGGGCGC GGGTGTGCTA
101801 CTATATCTAG TGGATGGAGG CCAGAAATGC TGCTTAACAT CCTACACACA
101851 GGATATACTG CCCGTGACAA GGAATTAGCT AGCCCAAAAT GTCAATAGTG
101901 CCAAAGTTGA ATAAACCTGT TCAAGAGCAA CACATTTCTA TTCCCTCTTC
101951 CATAGTAACT GTTAATAAAC ATTAGTTCCA TTTTCTACTA TTTCTGTTCT
102001 ACAATAGAGG CTTTAAAATA TCTTAAGAAA TGTAACACCA TTTTAATAAT
102051 TTGGCAAGTA GAAGTAAAGA TACCATGAAT CCTGAGATTT CACTTTCAAA
102101 AATGTATTAA GCTACATTTA TTTTACAATA AAACTTTTAG GAACAAAGTT
102151 GTCTTCTCCT TCANACACTG TTCTCAAAAA CTGTGCTAAA AGCCTTGAGT
102201 AATGTTTGAT AACTCCTTTT ATTTTGGGGT CCTTATAGCT GTTAATAACC
102251 ACATTCTGCC AGTTCTTTCC ATGTGAATGC CTCACATTTC AATGGATCTT
102301 TTTATATACC AATGTATTAT CTTGAAACAT TTTACTAACT GGTTTTTCTG
102351 AAGCTCAATG TCCTTGTCTC CTTCCCTCCC AAGCTATCCC TACCCATTAA
102401 TACTGTACTT TTCACCTGCT CAAAAGTCCA TATGGTTGCC TGTAACATAC
102451 CAAATAAGAA CCTAATTCCT AAAGCTTGAC ATTCAAATCC TTTTTTCAAC
102501 TAGCTACAAT CCACTTTTCC AACTCTTTTT TCCACTGCTC TCCCTTAGGA
102551 GTAGCCTTTT TTAGCAAAAT GACTTATTCT GAGATACACA AATAACCTTT
102601 CCAGATCAGA GACATCAAAA GCCATTCAGG CTTCACTGAG ACAAAAGATA
102651 TGCACACAAC TCAGAATAAC AGTAAGGAAT CAACTTATAG GGAAACCAGA
 
102701 TGTAGCCATG CTCTTTAAAT ATTGAGAGAT AACCCCGTTG GCTCATGCCA
102751 CTGTCCTTAT ATATAACAGC CCGTATTTGC CTTTCTACTT TTCTGATCTC
102801 CTTATACTTC AAATGTACAA TCTTTTCAAA AACCCAAACA CAGAGTCACC
102851 TCAGTCACCA CTNNTTGCCC TTCTCCTACC GCACCCAATG CCTGNGGCTT
102901 TCAGGTCATT TATTTTGCAN TAAACTTGTT ATCTCAATTT TACTGGTTCT
102951 CTTTTAGATT CAGACAGCTG AGATTATGAT AGATTCATTA GTAAGCTGAA
103001 TGGGGAAGGA AAGGATATTT ACATTCCCAG GCCAATTTTC AATGCTTTTT
103051 GGAAGATTCT GTTAAATTCA TAATTTCCAG AACTGGCAAG GAATAGGTAA
103101 ATATATACAC TCATATATAA CTGGTAAGAC TATAAATTGG TATAATTATC
103151 CTACAGTGAT ATCTGCCAAC ATTGAAAGTC TTTAAAATCT GTATACCCTT
103201 TGGCCTAGGA ATTCAACTTG AAAATTTATC ATGAGTAAAT TATAAAATGT
103251 ACAAAGGTTT AACTATAAAG ACACTCATCT CTGCATTGTT TCTAACTCTC
103301 AAAGATTGAG GGGGAGGGGA AAATCCCTTG TCCAATAAAA AAAGACTATT
103351 CCATGAATCA TTTATTTAAG TAATGTAATA CTTTGCAACT ACTAAAATCC
103401 TATAGTATCA TGGTTATTGA CATGTAACTC TTTTAATGGC TTTCTTTCCT
103451 AAGCTAAAAA AATACAAACA TGATTCCACA TAATGTATTT CAAAAATTGG
103501 TATACATTTT TCTACTTTTC TATATTTTCT ACACATCTAT ATATAATTTG
103551 TGTGTGTGTG TGTGTGTGTG TGTGTGTGTG TGTGTATGCA TGCATGTAAA
103601 TAAAATATAC CTCTAAGTCT TTGTAACCTT AAGATGGTGG AGCAAAATAT
103651 TTTTATTTTC TGTTGTTGAT TATTTAAAAT TTAGCTATAT TAAATCTTTT
103701 CTGATTTAGT AAACATACAT AAGGCCTATT TATAGTTGAA TGGCAGTCTT
103751 ACAAAACCTA AGCAACCTTG GTTTACTCTA TCTGTGGGAA AAGCATTTTT
103801 AGTAGAATGC TTGCGCATCT TCCTACATTC CTGAATCAAC CAGTATTTAT
103851 CATCAACTAT GTTGGTGACT CTCACTTTGA CATAGGCTGA ACCAGAAATC
103901 CTAGGAAAAA CTGATGACAG AAAGCTAAGG GGAAGGATAG GAAACCGGGA
103951 AACCCCGGGA AAGGAGGGAT TATATTACAC ATGTTATGGG AACTAGTTTG
104001 TATGTGCGGG GCACAGAGCA TACACTTAGA TAAGAAGGGG GTCATAAAAC
104051 CAGAAAGGCA ATAAATAGAA TGTTATGAGC ATGAACTCTA GACGCAAACT
104101 GTTCAGGTTC AATCTCACCT CTGCTTCTTA CTACATTTAT TACCTTAAGC
104151 AAGTCACTTA ACTTCTCCCT GCCGTTTCTG CCACATCTGT ATAATGGGGA
104201 TAACAGACCC TACCTTAGAG GGCTGTCACA CTAATGAATT CTGAGAATAG
104251 TCCCTGGTAC TACAGTTAAG TGCTCATTAG TTGTCCACAC TCATCATCTT
104301 CATTACTCCC ATTACTATGA TGTGAAGAGT TGTGCTGTTA GGACGGGAGA
104351 AGCAACTTTT CAACACCAAT CACTTTTTTA GACGGCCTCC TTTCTAGCCT
104401 TGTTAGTTGC CCAAATTCTG AAAAAAACAA GAACTAAGAC TCTAGAAGGA
104451 GATCCGAGAG GCAGACGTGT GTCTCTGTGT GTGTTTGTGT GTGTGTGTAG
104501 GTGCGTGTGC ATGTAGACGA AGGAGGAATC AGGAGTAATG CCAAGAATGA
104551 GAATCAAATC TGTATAAGCA GTTCAGAAAA GGGGCATCAT GAGTCGCTCT
104601 TCTCAGTGCT GCCCCAAGGG TTCCACAGTT TGCTCCTTCC ATCATTCACT
104651 TGGATAAAAC CTTTCAATGT CTCTCCATGG CCTTCAGAGT TAAATCAAAA
104701 CTATTCCTTG GAATGACTTA AAACCCATAC TATCTCTAAA GCCTCATCTC
104751 TTGGCTCTCT CGCACCAAGC TCACTGAGCT TCCAGCCCTA CCAAATTTCT
104801 TCAGTAACAA CCTCTCACAT CTCAGAGCCT TTGCATATGC TGCTCCCTCT
104851 GCCTGAAAAG TAACCTCTAC CTCCTCCAAT AGCAAACTGC TACTCAACCT
 
104901 TCAAAACACA GCTCAAGGAT AACTCCAGAG CTCTTTCGTG ATTTCAAGCT
104951 AAATGACTCT GCTATATTAT CCCCCATGTG CATAACACCA CCACTACCAC
105001 CACCACCATC ACCACTAAAA CCACGACCAC CACTACTCAC GCACTACCAC
105051 CACCACCACC ACCAACACCA ACACCACCAC CATGACCACT ACCACTACCA
105101 CCACCACCAC CATCACCACT ACTCACCACC ACCACTACCA CCACCAACAC
105151 CAACACCAAC ACCACCACCA TCACCACTAC CACTACCACC ACCACCAACA
105201 CCATCACCAC TACGACCACG ACCACCACCA CTACCACCAC CACCACCACC
105251 ACTACCACCA CCACCACCAT CACCACTAAA ACCACGACCA CCACCACCAC
105301 CATCACCACC ATCACCACCA CCACCACCAC CATCACGCAC CACCACCACC
105351 ACCACCATCA CCACCACCAC CACCATCACC ACCACTATCA CCACCACCAC
105401 AATGACCAGC ACCACCATTA GCACCAGCAT CACCACGAG. .........GG
105451 TTCAAGCAAT TCTCTGCCTC AGCCTCCCAA GTAGCTGGGA TTACAGGTGC
105501 CTGCCACCAT GCCTGGCTAA TTTTTGTAGT TTTAGTAGAG ATGGGGTTTC
105551 ACCATCTTAG CCAGGCTGGT CTTGAACTCC TGACCTCGTG ATCCATCTGC
105601 CTCAGCCTCC CAAAGTGCTG GGATTACAGG CGTGAGCCGC TGTTCCTGGC
105651 AGAGATTCTA AGTTTTTTGA GAATAGTGAC TGTGTTTCTT GGGTCATGGC
105701 TCTATCTTAT ACATTTGGCA CAGTGTGTGT GTGTAGCAGG TGTTTAATAT
105751 TTGTTAAACA CAAGTGGTCC TTAATATCAA ACAGCATGGG ATTTAAGACA
105801 TGAACTTAGG TTTGAGTCTC AGCACTTTCA CTTCTTAGTT GTATGACCTT
105851 GGTCATTTAA TCTCTTTGAT CTTGAATTTC CTTATCCGAA AAATAGGGGA
105901 CATACTAATT AACATCTATT GTGATGGGTT GTAATGAGAA TTAAATGACA
105951 ATGTGTACAA AATACCTAAC TTGATTGGAT TTGTGGTACG CAGTAGCTAT
106001 AATGACCATT TTAACCCCAA TATGAGAAGG ATTCACTCGT CACACTGTTG
106051 TATACTTCGT AGCTATTACT TCTTTAATCC CCAAGGACTT GTTTAACAAA
106101 GTGTTCTTCA GTTTCTACTT CCCAATTCCT TTGTGGAACT GGAAAAATTT
106151 AAAATATCTT AACATAATAT TTTATTTCAA ATGACAAACA GTAAGGTAAA
106201 ATGTGGTTTT TCTTGGCAAC ATATGGTAGA ATGATGTCTA GAATATTTAG
106251 TTATGCATTT AATACTTTTT TTCTTTACCA TTTTTTAAAA AAATATTTTA
106301 TTTTAGATTC GGGGGTACAC ATGCAGGTTC GTTACATGGC TAGATTATGT
106351 AATGCCGAGG TTTGGCCTGC TAGCACAGCC ATCATCCAAA GTGATCCTAG
106401 TACCCAATAG GTAGTTTTCA ACCTGTGTAC CTCCTCTTCT ACCTTCTCTT
106451 TTGGAATCTC TAGTCTATTA CTTCCATCTT TATGTTCACA TGTACTCATT
106501 GTTTAGCTCC CACTTAGAAA TGAGACCATG TGGTATTTGA TTTTCTGGTT
106551 CTGAGTTACT TCTTTTAGGA CAAAGGATGA AAAAGAATGT AGCCTCCAGC
106601 TCCATCCATG TTGCTGCGAA GGACATGATT CCATTCTTTT TTATGGCTGA
106651 rTATATTTTT ACCTTTTTTG TGAGGGGGAG AGGATTTAAA TATGGACCAG
106701 CATAATGAAG AAAATATCTG TAATCCCACT ATTCACATAA CCCTTGCTAG
106751 TTTAAAACAA GTTCATGTAG GCTGAGCATG GTGGCTCACA CCTATAATCC
106801 CAGCACTTTG GGAGTCCAAG GCTGGAGGAT CACTTGAGTT TAAGAGTTTA
106851 AGACCAGCTT GAGTAACATA GTGAAACACC ATGTCTACAA AAAATAAAAA
106901 TTAGCCAGGT ATGGTGGTGT ATGCCTGTAG TCCCAGCTAC TTGGGAGGAT
106951 TGCTTGAGGC CAAGAGTTCG AGGCTGCAGT AAGCTGTGAT TGTGCCACTG
107001 CACTCCACCA GGTGTTTAAA AAAAAAAGTA AATAATTACA AAATTTAAAT
107051 AGTGTAAACT AGAGGCCTCT CCTATTAGTT TGCTAGAGTT ACCATAACAA
 
107101 AATACCACAA ACTGAGTGAC ATAAGTGACA CTATTTTTTT TTTTTTGAGA
107151 TGCAGTCTCA CTCTGTCACC AGGCTGCACG ATCTCGGCTC ACTGCAGCCT
107201 CGGCCTCCCA GGTTCAAGTG ATTCTCCTAC CTCGGCCTCC CGAGTAGCTG
107251 GGACTTCCAG CCTCCAGAAT TGAGAAATTT AATTTCTTTT AATTTTAAAA
107301 AATTAAGAGT GACACTTGGC CGGGCGCAGT GGCTCATGCC TATAATCCCA
107351 GCACTTTGGG AGGCTGAGGC GGGCAGATCA CGAGGCCAGG AGATCGAGAC
107401 TATCCTGGCT AACACAGTGA AACCCCGTCT CTACTAAAAA TACAAAAAGT
107451 TGGTCGGGCG TGGTGGCTCA TGCCTGTAAT CCCAGCACTT TGGGAGTCCG
107501 AGGTAGGCGG ATAACCTGAG GTTGGGAGTT TCAGACCAGT ATGACCAACA
107551 CGGAGAAACC CTGTGTCTAC TAAAAATACA AAATTAGCTG TGCATGGTGG
107601 TGCATGCTTG TAA
<210> 2
   <211> 11162bp
   <212> DNA
   <213>
<400> gene vector sequenence

1 GGGCGAATTG GGCCCGACGT CGCATGCTCC TCTAGACTCG AGGAATTCTA
51 CCGGGTAGGG GAGGCGCTTT TCCCAAGGCA GTCTGGAGCA TGCGCTTAAG
101 CAGCCCCGCT GGGCACTTGG CGCTACACAA GTGGCCTCTG GCCTCGCACA
151 CATTCCACAT CCACCGGTAG GCGCCAACCG GCTCCGTTCT TTGGTGGCCC
201 CTTCGCGCCA CCTTCTACTC CTCCCCTAGT CAGGAAGTTC CCCCCCGCCC
251 CGCAGCTCGC GTCGTGCAGG ACGTGACAAA TGGAAGTAGC ACGTCTCACT
301 AGTCTCGTGC AGATGGACAG CACCGCTGAG CAATGGAAGC GGGTAGGCCT
351 TTGGGGCAGC GGCCAATAGC AGCTTTGCTC CTTCGCTTTC TGGGCTCAGA
401 GGCTGGGAAG GGGTGGGTCC GGGGGCGGGC TCAGGGGCGG GCTCAGGGGC
451 GGGGCGGGCG CCCGAAGGTC CTCCGGAGGC CCGGCATTCT GACGCTTCAA
501 AAGCGCACGT CTGCCGCGCT GTTCTCCTCT TCCTCATCTC CGGCCTTTCG
551 ACCTGCAGCG ACCCGCTTAA CAGCGTCAAC AGCGTGCCGC AGATCTTGGT
601 GGCGTGAAAC TCCCGCACCT CTTCGGCAAG CGCCTTGTAG AAGCGCGTAT
651 GGCTTCGTAC CCCTGCCATC AACACGCGTC TGCGTTCGAC CAGGCTGCGC
701 GTTCTCGCGG CCATAGCAAC CGACGTACGG CGTTGCGCCC TCGCCGGCAG
751 CAAGAAGCCA CGGAAGTCCG CCTGGAGCAG AAAATGCCCA CGCTACTGCG
801 GGTTTATATA GACGGTCCTC ACGGGATGGG GAAAACCACC ACCACGCAAC
851 TGCTGGTGGC CCTGGGTTCG CGCGACGATA TCGTCTACGT ACCCGAGCCC
901 GATGACTTAC TGGCAGGTGC TGGGGGCTTC CGAGACAATC GCGAACATCT
951 ACACCACACA ACACCGCCTC GACCAGGGTG AGATATCGGC CGGGGACGCG
1001 GCGGTGGTAA TGACAAGCGC CCAGATAACA ATGGGCATGC CTTATGCCGT
1051 GACCGACGCC GTTCTGGCTC CTCATATCGG GGGGGAGGCT GGGAGCTCAC
1101 ATGCCCCGCC CCCGGCCCTC ACCCTCATCT TCGACCGCCA TCCCATCGCC
1151 GCCCTCCTGT GCTACCCGGC CGCGCGATAC CTTATGGGCA GCATGACCCC

1201 CCAGGCCGTG CTGGCGTTCG TGGCCCTCAT CCCGCCGACC TTGCCCGGCA
1251 CAAACATCGT GTTGGGGGCC CTTCCGGAGG ACAGACACAT CGACCGCCTG
1301 GCCAAACGCC AGCGCCCCGG CGAGCGGCTT GACCTGGCTA TGCTGGCCGC
1351 GATTCGCCGC GTTTACGGGC TGCTTGCCAA TACGGTGCGG TATCTGCAGG
1401 GCGGCGGGTC GTGGCGGGAG GATTGGGGAC AGCTTTCGGG GACGGCCGTG
1451 CCCGCCCCAG GGTGCCGAGC CCCAGAGCAA CGCGGGCCCA CGACCCCATA
1501 TCGGGGACAC GTTATTTACC CTGTTTCGGG CCCCCGAGTT GCTGGCCCCC
1551 AACGGCGACC TGTACAACGT GTTTGCCTGG GCCTTGGACG TCTTGGCCAA
1601 ACGCCTCCGT CCCATGCACG TCTTTATCCT GGATTACGAC CAATCGCCCG
1651 CCGGCTGCCG GGACGCCCTG CTGCAACTTA CCTCCGGGAT GGTCCAGACC
1701 CACGTCACCA CCCCCGGCTC CATACCGACG ATCTGCGACC TGGCGCGCAC
1751 GTTTGCCCGG GAGATGGGGG AGGCTAACTG AAACACGGAA GGAGACAATA
1801 CCGGAAGGAA CCCGCGCTAT GACGGCAATA AAAAGACAGA ATAAAACGCA
1851 CGGGTGTTGG GTCGTTTGTT CATAAACGCG GGGTTCGGTC CCAGGGCTGG
1901 CACTCTGTCG ATACCCCACC GAGACCCCAT TGGGGCCAAT ACGCCCGCGT
1951 TTCTTCCTTT TCCCCACCCC ACCCCCCAAG TTCGGGTGAA GGCCCAGGGC
2001 TCGCAGCCAA CGTCGGGGCG GCAAGCCCTG CCATAGCCAC GGGCCCCGTG
2051 GGTTAGGGAC GGGGTCCCCC ATGGGGAATG GTTTATGGTT CGTGGGGGTT
2101 ATTATTTTGG GCGTTGCGTG GGGTCAGGTC CACGACTGGA CTGAGCAGAC
2151 AGACCCATGG TTTTTGGATG GCCTGGGCAT GGACCGCATG TACTGGCGCG
2201 ACACGAACAC CGGGCGTCTG TGGCTGCCAA ACACCCCCGA CCCCCAAAAA
2251 CCACCGCGCG GATTTCTGGC GCCGCCGGAC GAACTAAACC TGACTACGGC
2301 ATCTCTGCCC CTTCTTCGCT GGTACGAGGA GCGCTTTTGT TTTGTATTGG
2351 TCACCACGGC CGAGTTTCCG CGGGACCCCG GCCAGGACCT GCAGAAATTG
2401 ATGATCTATT AAACAATAAA GATGTCCACT AAAATGGAAG TTTTTCCTGT
2451 CATACTTTGT TAAGAAGGGT GAGAACAGAG TACCTACATT TTGAATGGAA
2501 GGATTGGAGC TACGGGGGTG GGGGTGGGGT GGGATTAGAT AAATGCCTGC
2551 TCTTTACTGA AGGCTCTTTA CTATTGCTTT ATGATAATGT TTCATAGTTG
2601 GATATCATAA TTTAAACAAG CAAAACCAAA TTAAGGGCCA GCTCATTCCT
2651 CCCACTCATG ATCTATAGAT CTATAGATCT CTCGTGGGAT CATTGTTTTT
2701 CTCTTGATTC CCACTTTGTG GTTCTAAGTA CTGTGGTTTC CAAATGTGTC
2751 AGTTTCATAG CCTGAAGAAC GAGATCAGCA GCCTCTGTTC CACATACACT
2801 TCATTCTCAG TATTGTTTTG CCAAGTTCTA ATTCCATCAG AAGCTCCTTA
2851 ATTTTATACC ACTGACTTAT TTTGAAGGCT GCTATAAGAA ACAGCCCTAT
2901 GAAACTGGTA TTTTCCTACT GCAAGGTGGC TACTTTAAGA CAATTTTTCA
2951 TTGCATTCTA TCAAGGGATG TCTTATTATT ATATCATTAT ATCAAGTGAT
3001 GTTATAAATA GTAAGAATCA GATTAAGGGC TCATATGTCC TTCTTTGTAT
3051 TGACTGTTGA AAAGGTATGG GGCCAAATTT GTAGTTTGTC TGGAATTACA
3101 TATTTTTGGG GGTCTCTATT ATCTTCATAC TTATCCTATC TAAATTTTCC
3151 ATTGCCAAAT TTCCTTACTT ATTTTAGTTT TTATCCTATT GCTCATGTAT
3201 TTTTATGTCT CCATAAGTCT ATTTTGGAAA AAGGCAGAGT ACTCATAATT
3251 TTAGTATATC TTTTAGCTTT ATGTTGCCAT AAACCTTTCA TTATATACAT
3301 GATCAACAAC AGCAAATTAT CTCACTTCAG TATTTAGTTT ATTATTTTAC
3351 AAACTGATTT ATGATTGCTA ACATGTAACT GAAGGTATAC ACTATTAGAA

3401 CACAGTTTTC AGTAGAAAGT AGCACTGCCA TTGAGTAAAA AAATGTTCTA
3451 ACATTAGAGC AACATTCTTA TACAAGTTTG CATGTTGTTT ACTGAGGTCT
3501 AAAGCATGAC TACACAAAAG GCTGAATAAA ATTCAGATTC TTACATACAC
3551 ATAAAATTGT TTTATTGAGA TGACAAAGTA TATTTATTAT GCCACCCAGA
3601 ATATAATCCA CTCTGATAAC TGCCAGTGTA TGCACTTGCT GAAGTAACTC
3651 AGTACATAAA TGGTAGCCAC AACAGTTGCT GTGCATGAAA GTTCTTCTCT
3701 TCCAGATTGA AGAGTGTACA ATCTAAAGCA TTTTAAAACT TTAAATCCCT
3751 TATTAGCTTA AATATAATTT AAAATTTTAG TTTGCCGTAC CTATAATTTG
3801 TCTGTACACT AGGTTACTAA GGGTGATATG ATTACATATG TGGATACAAA
3851 ATAATTTTAA TGGAAAATGA AATTAGGGTA CTCAACAAAG ATAAAGGGTA
3901 ATGATCATGT ACACTAACCG TATTTGAGAT TAGTTTAAGC CTGGGGTAGC
3951 TATACTTATG TTTCACAGAC CTTGAGAAGA TAGGGAAAAA AAGCTTTTAT
4001 CAACATTGCT AAGGAACAGG TAAAAGCTAA CATTAGGTAA CTAAGAGGTG
4051 ACATAAAAAA GACTGAATAA AATATCATGG AGGTTTCATA ATAAGATTGG
4101 AAATTCCATA GACTAGGAGA GAAAAGATCC CAAAATATAC ATGCTCATTG
4151 GGAAAACAGC TAGTAAGAAC AAGGAGAGAT CTCTATTTAA TGATACAATA
4201 GTAGAGTTAT AATTTCCTGT ATATTGTAAA TTTCAAGCAT TTAAACATTT
4251 TCATTGAATT ATAAAATATT ATTTGTAAAA GAAAGAAAAA CAGCACAACT
4301 GCAGATTACA GATGACTAAG ATAGATGAAT CATGAAAAGG TGCTAGATTG
4351 TGAGCGGATA ACAATTTCAC ACAGGAAACA GCTATGACCA TGATTACGCC
4401 AAGCTCTCGA CGGGATCGCG GCCGCGATCC AGACATGATA AGATACATTG
4451 ATGAGTTTGG ACAAACCACA ACTAGAATGC AGTGAAAAAA ATGCTTTATT
4501 TGTGAAATTT GTGATGCTAT TGCTTTATTT GTAACCATTA TAAGCTGCAA
4551 TAAACAAGTT GGGGTGGGCG AAGAACTCCA GCATGAGATC CCCGCGCTGG
4601 AGGATCATCC AGCCGGCGTC CCGGAAAACG ATTCCGAAGC CCAACCTTTC
4651 ATAGAAGGCG GCGGTGGAAT CGAAATCTCG TGATGGCAGG TTGGGCGTCG
4701 CTTGGTCGGT CATTTCGAAC CCCAGAGTCC CGCTCAGAAG AACTCGTCAA
4751 GAAGGCGATA GAAGGCGATG CGCTGCGAAT CGGGAGCGGC GATACCGTAA
4801 AGCACGAGGA AGCGGTCAGC CCATTCGCCG CCAAGCTCTT CAGCAATATC
4851 ACGGGTAGCC AACACTATGT CCTGATAGCG GTCCGCCACA CCCAGCCGGC
4901 CACAGTCGAT GAATCCAGAA AAGCGGCCAT TTTCCACCAT GATATTCGGC
4951 AAGCAGGCAT CGCCATGGGT CACGACGAGA TCCTCGCCGT CGGGCATGCT
5001 CGCCTTGAGC CTGGCGAACA GTTCGGCTGG CGCGAGCCCC TGATGCTCTT
5051 CGTCCAGATC ATCCTGATCG ACAAGACCGG CTTCCATCCG AGTACGTGCT
5101 CGCTCGATGC GATGTTTCGC TTGGTGGTCG AATGGGCAGG TAGCCGGATC
5151 AAGCGTATGC AGCCGCCGCA TTGCATCAGC CATGATGGAT ACTTTCTCGG
5201 CAGGAGCAAG GTGAGATGAC AGGAGATCCT GCCCCGGCAC TTCGCCCAAT
5251 AGCAGCCAGT CCCTTCCCGC TTCAGTGACA ACGTCGAGCA CAGCTGCGCA
5301 AGGAACGCCC GTCGTGGCCA GCCACGATAG CCGCGCTGCC TCGTCTTGCA
5351 GTTCATTCAG GGCACCGGAC AGGTCGGTCT TGACAAAAAG AACCGGGCGC
5401 CCCTGCGCTG ACAGCCGGAA CACGGCGGCA TCAGAGCAGC CGATTGTCTG
5451 TTGTGCCCAG TCATAGCCGA ATAGCCTCTC CACCCAAGCG GCCGGAGAAC
5501 CTGCGTGCAA TCCATCTTGT TCAACCATGG TGGATCGATC CAAGCTCCCA
5551 ACACAACTAT GTCAGAAGCA AATGTGAGGA GCAACTGATC CTACCTCACC

5601 TTATATGCTC TGCCCTGGCT CCTGCCCTCT CTATCCTGTG TGAGCAGATT
5651 GGCCCTTACC AAGGTGTGGC TCTACGGAAT CAGGCTTCGG TGATGACAAG
5701 CATATTTCTC CCTAGAATGC TGTGCCACTC ACTGGCTTAG GAGTCTCAGC
5751 TCTGGGTACT CCCTCTGAAT AATGTTTGTC CTTATCTGTG CAGAGAACAC
5801 TGTCTCTAAA GCATCCTTTT TGGCAACGCA TTTGCTCAAT CAACTACTGA
5851 ATTGGTGTTA AAATTAATTT TCCTTTTTTT CTCATTATGC AAATAAGAAA
5901 TTGAGAAGCA AAGCTAGCAG AGATTTCTAT CACACCTATC AGGGATACAC
5951 AATTTCCAAG AATTTCAGAA GTGTTTGGTG TTCCTATTAA CATAAATCCG
6001 GAAATAACAC CTGAGTGAAC TGTCTTCTAA TTCTTCAACT GGATGGCTTT
6051 TTAGTGTAAA AGATGTTGAA TACTGATTGA CTTTTTAATA ATTTTATAGT
6101 ATATGTCAGA AATATTGCAC AGTCCCTATT TACATCATTC TACAGTGGTT
6151 TTTAAAATGT TTTAAGAATA AAAAACATGA AAACTTTATT TGATTTTTCT
6201 GAGGAAATAA CTTTTTGGAT TTAATTTCAA TGAAACCGTT GATAACATTT
6251 CCCTCCCCAA CAATCTCTGG CAACGATCCC TCAGATTTTA ATGATTATGT
6301 ATTATTACCT TTTAATACAA GTAGAATAAC ACTCAGGGAA TTTACAACAT
6351 TTGTTATTTT CAGTAAATAC ATTGGTTGAA GTTTAAAAGT CTATCCGTAG
6401 TAAACTTACA TCTTTCAGGA GCTTGGTCAA TGTGTTCTGG ACAAAGCAGG
6451 AAGATGTGAC TGAAATCCTG AAAGGAGCCG GCTCCTGCAG CACAAGGATA
6501 ATGATACATC TGGGTACATT TCTCTTCACA GCATTTGATA GTGGCTCCAA
6551 AGTGCTTACA AAATGCACAT TGCTGAAAGG GGTAAAGGAG AGAAATCTCT
6601 TTATAAAACC TTGAAAAGGA ATATTTAAAT ATAAGCTGGG AAGGTATAAA
6651 AAACTCTCTG TAACATCACA AGTAAACAAA TTGAACCTGC AAAATATTAA
6701 ACAAAGGATT CATTAAAAAT AATAAAATCT ACATTACTCA ATTTAGTGCT
6751 TTGTGTGCTA CCAACTCATC CTTCCATTCA AATTAGAAAG TTAGAATTTC
6801 ATTCCTTATA TTTTCAAAAA TAAATTGTGA AGCATTTTAG AAACAAAACC
6851 TAAAATTTTT TTTTAAAAGC AAATAGTAAT ATGGTTAAAG GGGCAGGTTT
6901 CTATATTGAG GATTATTATA AAGTTTTTAA ATCCTACCAA AACTAGTAAT
6951 AGGAACATAT ATTATTTATG AGACATATTA CTATTTTTTA CCCTGCCTAA
7001 AAATAAATAC AAATAAATTC ATCAATTATA AGTTAACAGG GACACAAATG
7051 GTTAAAGACT CACACACAAA AAAAACAAAA CTACATACTT CAATGTAGCA
7101 ATCAACTTCA AATTTCTTAA CAAAAGATGG AAATGTTGGG GAAAAAATTA
7151 GTCATCTGGT ATCTTTCCCA TTTCAACCTG CCTCCATTAT CTTGCAAGTG
7201 GTAAAATGCA CAGAAATAAG CCTCAAACAA GAGGGGCAGT CTAGGGCAAG
7251 TGAACACATA AGTCGGAAGA AATTATGTAA AATGTTGCAT TTACTTATTC
7301 AGTTTTCCCT TAGAATGATT CACAAACTCT TCCTCATTCT CCCAAGTCCA
7351 TTTTGAGTAT CATTTTCTTT GAAGAGAGTC TGATGGGCCC TGTACTATAC
7401 AGTATGAAAT CTCTCTGTGG GAAATGACTA TCTAACATAA ATTTTTGTTT
7451 ACACCGTTAC ATGGTACCTA CTTGCTTATG CCATTACATG ATCAGTTTAC
7501 CTTTTTCTCA ACCTAATCCA AGATCCTTCA ATTGAGGCAC TATACTATCT
7551 TTGTATCCAA AGCACCAAAA ATGCTGCTTC AAACAGGCCC TAATAGATAG
7601 GTGTTCCTAT ACATATACCA AAAAGACTTA ACTTTTGGTG ATCTTGTTTG
7651 TGAGTGTGGC TCATAAACAG CTTAGTTGAG ATAACTGGAG CCTCATGTAG
7701 CAGAGACAGT TGGACCCTGC TAACATTACT GTGGATATCT TCACATGTTA
7751 CTACATTGAC TTTATATTCT GCTAATTAAC CAGGGACTAC AGTAGTTAAA

7801 ATTATAATTG TTTTCAATGT TTTATGTGTA AATCTGTATC TCACATACTA
7851 TCAAACTCTT CCTCACTGTC ATCAGTCTAC TGCATTGAAT CCAACATAAC
7901 AAAGCTAAAT GACTCCTGAG GGCTGAATCA GAAAGAAGAA AAGAAAGAGA
7951 TACAAAACTT TAGTCGGCCC GGTGGCTCAC ACCTGTAATC CCAGCACTTT
8001 GGAAGGCCAA GGCGGGCGGA TCACGAGGTC AGGAGATCGA GACCATCCTG
8051 GCTGATACAG TGAAACTCCA TCTCTACTGA AAATACAAAA AATTAGCTGG
8101 ACGTGGTGGT GGGCACCTGT AGTCCCAGCT ACTCAGGAGG CTGAAGCAGG
8151 AGAAGCTTCT AAATAACTCA TAAACACTAA TTACTGTTGT GACACTTTAA
8201 TTTTATACAA TATTTATAAG TATACAGAAT AACATTTCAG TGCTATTTTG
8251 GCACTCAAGG GTATTAATGC ATAGCTTGAG TATTCTATAG TGTCACCTAA
8301 ATAGCTTGGC GTAATCATGG TCATAGCTGT TTCCTGTGTG AAATTGTTAT
8351 CCGCTCACAA TTCCACACAA CATACGAGCC GGAAGCATAA AGTGTAAAGC
8401 CTGGGGTGCC TAATGAGTGA GCTAACTCAC ATTAATTGCG TTGCGCTCAC
8451 TGCCCGCTTT CCAGTCGGGA AACCTGTCGT GCCAGCTGCA TTAATGAATC
8501 GGCCAACGCG CGGGGAGAGG CGGTTTGCGT ATTGGGCGCT CTTCCGCTTC
8551 CTCGCTCACT GACTCGCTGC GCTCGGTCGT TCGGCTGCGG CGAGCGGTAT
8601 CAGCTCACTC AAAGGCGGTA ATACGGTTAT CCACAGAATC AGGGGATAAC
8651 GCAGGAAAGA ACATGTGAGC AAAAGGCCAG CAAAAGGCCA GGAACCGTAA
8701 AAAGGCCGCG TTGCTGGCGT TTTTCGATAG GCTCCGCCCC CCTGACGAGC
8751 ATCACAAAAA TCGACGCTCA AGTCAGAGGT GGCGAAACCC GACAGGACTA
8801 TAAAGATACC AGGCGTTTCC CCCTGGAAGC TCCCTCGTGC GCTCTCCTGT
8851 TCCGACCCTG CCGCTTACCG GATACCTGTC CGCCTTTCTC CCTTCGGGAA
8901 GCGTGGCGCT TTCTCATAGC TCACGCTGTA GGTATCTCAG TTCGGTGTAG
8951 GTCGTTCGCT CCAAGCTGGG CTGTGTGCAC GAACCCCCCG TTCAGCCCGA
9001 CCGCTGCGCC TTATCCGGTA ACTATCGTCT TGAGTCCAAC CCGGTAAGAC
9051 ACGACTTATC GCCACTGGCA GCAGCCACTG GTAACAGGAT TAGCAGAGCG
9101 AGGTATGTAG GCGGTGCTAC AGAGTTCTTG AAGTGGTGGC CTAACTACGG
9151 CTACACTAGA AGGACAGTAT TTGGTATCTG CGCTCTGCTG AAGCCAGTTA
9201 CCTTCGGAAA AAGAGTTGGT AGCTCTTGAT CCGGCAAACA AACCACCGCT
9251 GGTAGCGGTG GTTTTTTTGT TTGCAAGCAG CAGATTACGC GCAGAAAAAA
9301 AGGATCTCAA GAAGATCCTT TGATCTTTTC TACGGGGTCT GACGCTCAGT
9351 GGAACGAAAA CTCACGTTAA GGGATTTTGG TCATGAGATT ATCAAAAAGG
9401 ATCTTCACCT AGATCCTTTT AAATTAAAAA TGAAGTTTTA AATCAATCTA
9451 AAGTATATAT GAGTAAACTT GGTCTGACAG TTACCAATGC TTAATCAGTG
9501 AGGCACCTAT CTCAGCGATC TGTCTATTTC GTTCATCCAT AGTTGCCTGA
9551 CTCCCCGTCG TGTAGATAAC TACGATACGG GAGGGCTTAC CATCTGGCCC
9601 CAGTGCTGCA ATGATACCGC GAGACCCACG CTCACCGGCT CCAGATTTAT
9651 CAGCAATAAA CCAGCCAGCC GGAAGGGCCG AGCGCAGAAG TGGTCCTGCA
9701 ACTTTATCCG CCTCCATCCA GTCTATTAAT TGTTGCCGGG AAGCTAGAGT
9751 AAGTAGTTCG CCAGTTAATA GTTTGCGCAA CGTTGTTGGC ATTGCTACAG
9801 GCATCGTGGT GTCACGCTCG TCGTTTGGTA TGGCTTCATT CAGCTCCGGT
9851 TCCCAACGAT CAAGGCGAGT TACATGATCC CCCATGTTGT GCAAAAAAGC
9901 GGTTAGCTCC TTCGGTCCTC CGATCGTTGT CAGAAGTAAG TTGGCCGCAG
9951 TGTTATCACT CATGGTTATG GCAGCACTGC ATAATTCTCT TACTGTCATG

10001 CCATCCGTAA GATGCTTTTC TGTGACTGGT GAGTACTCAA CCAAGTCATT
10051 CTGAGAATAC CGCGCCCGGC GACCGAGTTG CTCTTGCCCG GCGTCAATAC
10101 GGGATAATAG TGTATGACAT AGCAGAACTT TAAAAGTGCT CATCATTGGA
10151 AAACGTTCTT CGGGGCGAAA ACTCTCAAGG ATCTTACCGC TGTTGAGATC
10201 CAGTTCGATG TAACCCACTC GTGCACCCAA CTGATCTTCA GCATCTTTTA
10251 CTTTCACCAG CGTTTCTGGG TGAGCAAAAA CAGGAAGGCA AAATGCCGCA
10301 AAAAAGGGAA TAAGGGCGAC ACGGAAATGT TGAATACTCA TACTCTTCCT
10351 TTTTCAATAT TATTGAAGCA TTTATCAGGG TTATTGTCTC ATGAGCGGAT
10401 ACATATTTGA ATGTATTTAG AAAAATAAAC AAATAGGGGT TCCGCGCACA
10451 TTTCCCCGAA AAGTGCCACC TGTATGCGGT GTGAAATACC GCACAGATGC
10501 GTAAGGAGAA AATACCGCAT CAGGCGACGC GCCCTGTAGC GGCGCATTAA
10551 GCGCGGCGGG TGTGGTGGTT ACGCGCAGCG TGACCGCTAC ACTTGCCAGC
10601 GCCCTAGCGC CCGCTCCTTT CGCTTTCTTC CCTTCCTTTC TCGCCACGTT
10651 CGCCGGCTTT CCCCGTCAAG CTCTAAATCG GGGGCTCCCT TTAGGGTTCC
10701 GATTTAGAGC TTTACGGCAC CTCGACCGCA AAAAACTTGA TTTGGGTGAT
10751 GGTTCACGTA GTGGGCCATC GCCCTGATAG ACGGTTTTTC GCCCTTTGAC
10801 GTTGGAGTCC ACGTTCTTTA ATAGTGGACT CTTGTTCCAA ACTGGAACAA
10851 CACTCAACCC TATCTCGGTC TATTCTTTTG ATTTATAAGG GATTTTGCCG
10901 ATTTCGGCCT ATTGGTTAAA AAATGAGCTG ATTTAACAAA TATTTAACGC
10951 GAATTTTAAC AAAATATTAA CGTTTACAAT TTCCATTCGC CATTCAGGCT
11001 GCGCAACTGT TGGGAAGGGC GATCGGTGCG GGCCTCTTCG CTATTACGCC
11051 AGCTGGCGAA AGGGGGATGT GCTGCAAGGC GATTAAGTTG GGTAACGCCA
11101 GGGTTTTCCC AGTCACGACG TTGTAAAACG ACGGCCAGTG AATTGTAATA
11151 CGACTCACTA TA

## Claims

1. A vector having the sequence set forth in SEQ ID NO: 2.

2. An in vitro method of gene expression including the following steps:
(i) cloning a gene of interest into the vector of claim 1;
(ii) transferring the gene of interest into the target region of D, G, group chromosomes of human cells; and
(iii) expressing the gene of interest.

3. Use of the sequence between nucleotide position 75590 and 79448 of SEQ ID NO: 1 as gene leading sequence for the preparation of a vector for use in gene therapy.

4. A vector having a gene of interest cloned into the vector of claim 1 for use in gene therapy.

## Patentansprüche

1. Vektor mit der in der SEQ ID NO: 2 dargelegten Sequenz.

2. In-vitro-Verfahren zur Genexpression, das die folgenden Schritte einschließt:
(i) Klonieren eines Gens von Interesse in den Vektor nach Anspruch 1;
(ii) Übertragen des Gens von Interesse in die Zielregion von Chromosomen der D, G Gruppe aus menschlichen Zellen und
(iii) Exprimieren des Gens von Interesse.

3. Verwendung der Sequenz zwischen Nucleotidposition 75590 und 79448 der SEQ ID NO: 1 als Gen-Leader-Sequenz zur Herstellung eines Vektors zur Verwendung in der Gentherapie.

4. Vektor mit einem Gen von Interesse, kloniert in den Vektor nach Anspruch 1, zur Verwendung in der Gentherapie.

## Revendications

1. Vecteur ayant la séquence présentée dans la SEQ ID NO: 2.

2. Procédé *in vitro* d'expression génétique incluant les étapes suivantes :
(i) clonage d'un gène d'intérêt dans le vecteur de la revendication 1 ;
(ii) transfert du gène d'intérêt dans la région cible des chromosomes des groupes D, G de cellules humaines ; et
(iii) expression du gène d'intérêt.

3. Utilisation de la séquence entre la position des nucléotides 75590 et 79448 de la SEQ ID NO: 1 en tant que séquence génique de tête pour la préparation d'un vecteur pour une utilisation en thérapie génique.

4. Vecteur ayant un gène d'intérêt cloné dans le vecteur de la revendication 1, pour une utilisation en thérapie génique.
